(19) Europäisches Patentamt
European Patent Office
Office européen des brevets

(11) EP 2 492 988 B1

(12) **EUROPEAN PATENT SPECIFICATION**

(45) Date of publication and mention of the grant of the patent:
**26.12.2018 Bulletin 2018/52**

(21) Application number: **10825088.7**

(22) Date of filing: **21.10.2010**

(51) Int Cl.:
***H01L 51/50*** (2006.01) ***C07D 213/22*** (2006.01)
***C07D 251/16*** (2006.01) ***C07D 265/38*** (2006.01)
***C08K 5/3432*** (2006.01) ***C08L 65/00*** (2006.01)

(86) International application number:
**PCT/JP2010/069122**

(87) International publication number:
**WO 2011/049241 (28.04.2011 Gazette 2011/17)**

(54) **ORGANIC ELECTROLUMINESCENT ELEMENT**

ORGANISCHES ELEKTROLUMINESZENZELEMENT

ÉLÉMENT ÉLECTROLUMINESCENT ORGANIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**22.10.2009 JP 2009243349**
**22.10.2009 JP 2009243351**
**24.06.2010 JP 2010143554**
**24.06.2010 JP 2010143555**

(43) Date of publication of application:
**29.08.2012 Bulletin 2012/35**

(73) Proprietor: **Sumitomo Chemical Company, Limited**
**Tokyo 104-8260 (JP)**

(72) Inventors:
- **SOGA, Masayuki**
  **Nishi-tokyo-shi**
  **Tokyo 188-0012 (JP)**
- **ISHII, Yusuke**
  **Toda-shi**
  **Saitama 335-0023 (JP)**

(74) Representative: **J A Kemp**
**14 South Square**
**Gray's Inn**
**London WC1R 5JJ (GB)**

(56) References cited:
**EP-A1- 1 933 396** **WO-A1-2006/070619**
**WO-A1-2006/070619** **WO-A1-2007/086552**
**WO-A1-2009/123269** **WO-A2-2005/059951**
**JP-A- 2004 139 819** **JP-A- 2006 128 636**
**JP-A- 2006 128 636** **JP-A- 2008 010 805**
**JP-A- 2009 149 846**

- **"LIGHT-EMITTING METAL COMPLEXES", chemicalland21.com , XP002693568, Retrieved from the Internet: URL:http://chemicalland21.com/info/LED%20METAL%20COMPLEXES.htm [retrieved on 2013-03-11]**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 492 988 B1

Printed by Jouve, 75001 PARIS (FR)

**Description**

Technical Field

The present invention relates to an organic electroluminescent device.

Background Art

It is known that an organic electroluminescent device of high light emission efficiency having an anode and a cathode, a light emitting layer disposed between the electrodes, and a hole transporting layer disposed so as to be in contact with the light emitting layer is obtained, by using a composition prepared by doping a polymer compound-containing host material with a phosphorescent compound as a dopant for fabrication of the light emitting layer and by using a hole transporting polymer compound having lowest excitation triplet energy larger than that of the phosphorescent compound for fabrication of the hole transporting layer (Patent document 1).

[Patent Document 1]
JP-A No. 2008-179617

Recently, an organic electroluminescent device equipped with a hole transporting layer composed of a polymer compound has been developed. As this organic electroluminescent device, an organic electroluminescent device equipped with a hole transporting layer composed of a polymer compound having a substituted triphenylamine residue as a repeating unit and an organic electroluminescent device equipped with a hole transporting layer composed of a polymer compound having a fluorenediyl group as a repeating unit are known (Patent documents 2 and 3) .

[Patent Document 2]
JP-A No. 10-92582
[Patent Document 3]
International Publication WO 2005/49548 pamphlet

WO 2006/070619 A1 discloses an organic electroluminescent device comprising a single layer A which is arranged between an anode and a cathode and contains at least one common host material. This organic electroluminescent device is characterized in that the single layer A has a hole-transporting region containing at least one hole-transporting material and a light-emitting region containing at least one light-emitting dopant; the hole-transporting region and the light-emitting region do not overlap each other; not less than 0.1% by mass of the common host material is contained in all the regions of the single layer A; and the amount of the common host material contained in each region is generally constant.

JP 2006 128636 A discloses a light emitting element has an organic compound layer containing at least one layer of luminescent layer between a pair of electrodes. The luminescent layer contains at least one kind of luminescent dopant and plurality of host compounds.

JP 2004 139819 describes an OLED comprising a light emitting layer and two hole transporting layers, wherein the triplet energy $T_1$ of the first hole transporting material is lower than $T_1$ of the host compound of the light emitting layer and $T_1$ of the second hole transporting material is higher than $T_1$ of the host compound of the light emitting layer.

Disclosure of the Invention

The organic electroluminescent device disclosed in the above-described patent document 1, however, has an insufficient luminance life.

The organic electroluminescent devices disclosed in the above-described patent documents 2 and 3 show an increase in driving voltage at the half life of luminance when driven at a constant current value.

The present invention has an object of providing an organic electroluminescent device having a long luminance life.

The present invention provides the following organic electroluminescent devices.

[1] An organic electroluminescent device comprising
an anode,
a cathode,
a light emitting layer that is disposed between the anode and the cathode and contains a first light emitting layer material containing a phosphorescent compound and a second light emitting layer material containing a charge

transporting polymer compound, and
a hole transporting layer that is disposed between the anode and the light emitting layer so as to be in contact with the light emitting layer and is composed of a hole transporting polymer compound,
wherein the lowest excitation triplet energy $T1_e$ (eV) of the first light emitting layer material, the lowest excitation triplet energy $T1_h$ (eV) of the second light emitting layer material and the lowest excitation triplet energy $T1_t$ (eV) of the hole transporting polymer compound satisfy the following formulae (A) and (B):

$$T1_e \leq T1_h \qquad (A)$$

$$T1_t - T1_e \leq 0\ eV \qquad (B).$$

[2] The organic electroluminescent device according to [1], wherein, $T1_t$ and $T1_e$ further satisfy the following formula (B'):

$$T1_t - T1_e \geq -0.30\ eV \qquad (B').$$

[3] The organic electroluminescent device according to [1] or [2], wherein the minimum value $IP_{eh}$ (eV) of the ionization potential of the above-described first light emitting layer material and the ionization potential of the above-described second light emitting layer material, and the ionization potential $IP_t$ (eV) of the above-described hole transporting polymer compound satisfy the following formula (C):

$$IP_{eh} - IP_t \geq -0.20\ eV \qquad (C).$$

[4] The organic electroluminescent device according to any one of [1] to [3], wherein the above-described hole transporting polymer compound is a polymer compound containing a repeating unit represented by the following formula (4):

$$-\!\left(Ar^1\right)\!- \qquad (4)$$

in the formula (4), $Ar^1$ represents an arylene group, a divalent aromatic heterocyclic group, or a divalent group composed of two or more directly linked identical or different groups selected from the group consisting of the arylene group and the divalent aromatic heterocyclic group, wherein the group represented by $Ar^1$ may have an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group, a fluorine atom or a cyano group as a substituent;
and a repeating unit represented by the following formula (5):

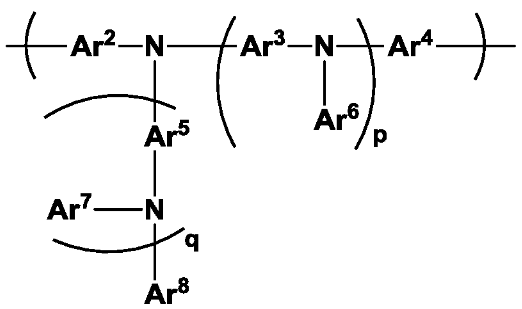

(5)

in the formula (5), $Ar^2$, $Ar^3$, $Ar^4$ and $Ar^5$ each independently represent an arylene group, a divalent aromatic heterocyclic group, or a divalent group composed of two or more directly linked identical or different groups selected from the group consisting of the arylene group and the divalent aromatic heterocyclic group; $Ar^6$, $Ar^7$ and $Ar^8$ each independently represent an aryl group or a monovalent aromatic heterocyclic group; p and q each independently represent 0 or 1, wherein the groups represented by $Ar^2$, $Ar^3$, $Ar^4$, $Ar^5$, $Ar^6$, $Ar^7$ and $Ar^8$ may have an alkyl group, an aryl group, a monovalent aromatic heterocyclic group, an alkoxy group, an aryloxy group, an aralkyl group, an

arylalkoxy group, a substituted amino group, a substituted carbonyl group, a substituted carboxyl group, a fluorine atom or a cyano group as a substituent, and the groups represented by $Ar^5$, $Ar^6$, $Ar^7$ and $Ar^8$ may each be linked directly or via -O-, -S-, -C(=O)-, -C(=O)-O-, -N($R^A$)-, -C(=O)-N($R^A$)- or -C($R^A$)$_2$- to the group represented by $Ar^2$, $Ar^3$, $Ar^4$, $Ar^5$, $Ar^6$, $Ar^7$ or $Ar^8$ linked to the nitrogen atom to which the groups are attached, thereby forming a 5 to 7-membered ring; $R^A$ represents an alkyl group, an aryl group, a monovalent aromatic heterocyclic group or an aralkyl group.

[5] The organic electroluminescent device according to any one of [1] to [4], wherein the above-described hole transporting polymer compound is a crosslinkable hole transporting polymer compound.

[6] The organic electroluminescent device according to any one of [1] to [5], wherein the above-described charge transporting polymer compound is a polymer compound containing at least one repeating unit selected from the group consisting of repeating units represented by the following formula (4):

$$\left[ Ar^1 \right] \qquad (4)$$

in the formula (4), $Ar^1$ represents an arylene group, a divalent aromatic heterocyclic group, or a divalent group composed of two or more directly linked identical or different groups selected from the group consisting of the arylene group and the divalent aromatic heterocyclic group, wherein the group represented by $Ar^1$ may have an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group, a fluorine atom or a cyano group as a substituent;

and repeating units represented by the following formula (5) :

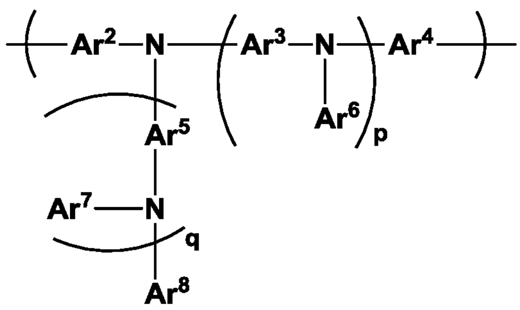

(5)

in the formula (5), $Ar^2$, $Ar^3$, $Ar^4$ and $Ar^5$ each independently represent an arylene group, a divalent aromatic heterocyclic group, or a divalent group composed of two or more directly linked identical or different groups selected from the group consisting of the arylene group and the divalent aromatic heterocyclic group; $Ar^6$, $Ar^7$ and $Ar^8$ each independently represent an aryl group or a monovalent aromatic heterocyclic group; p and q each independently represent 0 or 1, wherein the groups represented by $Ar^2$, $Ar^3$, $Ar^4$, $Ar^5$, $Ar^6$, $Ar^7$ and $Ar^8$ may have an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group, a fluorine atom or a cyano group as a substituent, and the groups represented by $Ar^5$, $Ar^6$, $Ar^7$ and $Ar^8$ may each be linked directly or via -O-, -S-, -C(=O)-, -C(=O)-O-, -N($R^A$)-, -C(=O)-N($R^A$)- or -C($R^A$)$_2$- to the group represented by $Ar^2$, $Ar^3$, $Ar^4$, $Ar^5$, $Ar^6$, $Ar^7$ or $Ar^8$ linked to the nitrogen atom to which the groups are attached, thereby forming a 5 to 7-membered ring; $R^A$ represents an alkyl group, an aryl group, a monovalent aromatic heterocyclic group or an aralkyl group.

[7] The organic electroluminescent device according to any one of [4] to [6], containing a repeating unit represented by the following formula (6) and/or a repeating unit represented by the following formula (7), as the repeating unit represented by the above-described formula (4) :

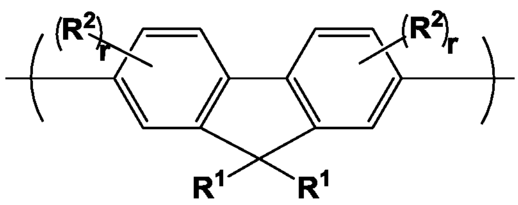

(6)

in the formula (6), each $R^1$ represents an alkyl group, an aryl group, a monovalent aromatic heterocyclic group or

an aralkyl group; each $R^2$ represents an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group, a fluorine atom or a cyano group; each r represents an integer of 0 to 3, wherein two $R^1$ moieties may be the same or different, and two $R^1$ moieties may be linked to form a ring; when a plurality of $R^2$ moieties are present, these may be the same or different; two characters of r may be the same or different.

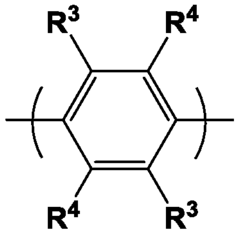

(7)

in the formula (7), each $R^3$ represents an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group or a cyano group; each $R^4$ represents a hydrogen atom, an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group, a fluorine atom or a cyano group, wherein two $R^3$ moieties may be the same or different, and two $R^4$ moieties may be the same or different.

[8] The organic electroluminescent device according to [7], wherein the repeating unit represented by the above-described formula (4) is a repeating unit represented by the above-described formula (6).

[9] The organic electroluminescent device according to [7], wherein the repeating unit represented by the above-described formula (4) is a repeating unit represented by the above-described formula (7).

[10] The organic electroluminescent device according to any one of [4] to [9], wherein at least one of p and q is 1 in the above-described formula (5).

[11] The organic electroluminescent device according to any one of [1] to [10], wherein the above-described phosphorescent compound is an iridium complex.

[12] The organic electroluminescent device according to any one of [1] to [11], having a hole injection layer between the above-described anode and the above-described hole transporting layer.

Modes for Carrying Out the Invention

The present invention will be illustrated below. In the present specification, Me represents a methyl group and Et represents an ethyl group.

In the present specification, "the hole transporting layer composed of a hole transporting polymer compound" includes a hole transporting layer containing a hole transporting polymer compound itself, a hole transporting layer containing a hole transporting polymer compound in cross-linked condition in its molecule and/or between molecules, and the like.

For the organic electroluminescent device of the present invention, the above-described formula (A) is $T1_e \leq T1_h$, and if $T1_e$ is larger than $T1_h$, there is a tendency of lowering of the light emission efficiency.

In the organic electroluminescent device of the present invention, the above-described formula (B) is $T1_t$-$T1_e \leq 0$ eV. If $T1_t$-$T1_e$ is larger than 0.10 eV, there is a tendency of shortening of the luminance life. $T1_t$-$T1_e$ is therefore 0 eV or less, from the viewpoint of the luminance life.

Further, it is preferable that $T1_t$-$T1_e$ satisfies the following formula (B'):

$$T1_t - T1_e \geq -0.30 \qquad (B'),$$

and it is more preferably -0.20 eV or more, particularly preferably -0.10 eV or more, from the viewpoint of the light emission efficiency.

In the present invention, the lowest excitation triplet energy is determined by a scientific calculation method. In the scientific calculation method, a repeating unit is optimized in its structure, by a density functional approach at B3LYP level, using a quantum chemistry calculation program Gaussian 03, with a base function of 3-21G*. Thereafter, the lowest excitation triplet energy is calculated, by a time-dependent density functional approach at B3LYP level, with a

base function of 3-21G*. In the case of the presence of an atom to which 3-21G* cannot be applied as the base function, LANL2DZ is used as the base function for this atom.

In the present invention, when the above-described hole transporting polymer compound and the above-described charge transporting polymer compound are composed of one repeating unit, the lowest excitation triplet energy is calculated for a dimer of this repeating unit and the calculated value is used as the lowest excitation triplet energy of the polymer compound. When the above-described hole transporting polymer compound and the above-described charge transporting polymer compound are composed of two or more repeating units, the lowest excitation triplet energies are calculated for all dimers which can be generated in polymerization from repeating units contained in a molar ratio of 1% or more, and the minimum value among them is used as the lowest excitation triplet energy of the polymer compound.

In the organic electroluminescent device of the present invention, when the hole transporting layer is formed by using two or more of the above-described hole transporting polymer compound and when the light emitting layer is formed by using two or more of the above-described charge transporting polymer compound, the lowest excitation triplet energies are calculated for all the hole transporting polymer compounds and the charge transporting polymer compounds used in formation of the layers, and the minimum value among them is used as the lowest excitation triplet energy of the polymer compound.

In the organic electroluminescent device of the present invention, the minimum value $IP_{eh}$ (eV) of the ionization potential of the above-described first light emitting layer material and the ionization potential of the above-described second light emitting layer material, and the ionization potential $IP_t$ (eV) of the above-described hole transporting polymer compound preferably satisfy the following formula (C):

$$IP_{eh}-IP_t \geq -0.20\ \text{eV} \qquad (C)$$

and $IP_{eh}$-$IP_t$ is more preferably -0.10 eV or more, further preferably -0.05 eV or more, particularly preferably 0 eV or more, from the viewpoint of the hole injectability.

In the present invention, the ionization potential of the above-described first light emitting layer material, the above-described second light emitting layer material and the above-described hole transporting polymer compound can be directly measured by a photoelectron spectroscopic method, and specifically, can be measured by a low energy electron spectrometer.

In the organic electroluminescent device of the present invention, when light emitting layer contains two or more of the above-described first light emitting layer material and two or more of the above-described second light emitting layer material, the ionization potentials are measured for all the light emitting layer materials contained in a weight ratio of 5% or more in the layer, and the minimum value of them is used as the ionization potential of the material.

In the organic electroluminescent device of the present invention, when the hole transporting layer is formed by using two or more of the above-described hole transporting polymer compound, the ionization potentials are measured for all the compounds contained in a weight ratio of 5% or more, and the minimum value of them is used as the ionization potential of the hole transporting polymer compound.

<Light emitting layer>

•First light emitting layer material

The first light emitting layer material is usually composed only of a phosphorescent compound (that is, only a phosphorescent compound as an essential component), however, additionally, a fluorescent compound such as an anthracene derivative, a perylene derivative, a coumarin derivative, a rubrene derivative, a quinacridone derivative, a squarylium derivative, a porphyrin derivative, a styryl dye, a tetracene derivative, a pyrazolone derivative, decacyclene, phenoxazone and the like may also be contained. The components constituting the first light emitting layer material may each be composed of a single compound or two or more compounds. The first light emitting layer material is, in general, called a guest material in some cases.

The above-described phosphorescent compound includes phosphorescent metal complexes. This phosphorescent metal complex has a central metal and a ligand. The central metal is usually an atom having an atomic number of 50 or more and is a metal manifesting spin-orbit interaction in the compound and capable of causing intersystem crossing between the singlet state and the triplet state. This central metal includes preferably gold, platinum, iridium, osmium, rhenium, tungsten, europium, terbium, thulium, dysprosium, samarium, praseodymium, gadolinium and ytterbium, more preferably gold, platinum, iridium, osmium, rhenium and tungsten, further preferably gold, platinum, iridium, osmium and rhenium, particularly preferably platinum and iridium, and especially preferably iridium.

The ligand in the above-described phosphorescent metal complex is preferably an aromatic ring (single ring

or condensed ring) containing a coordinating atom for the central metal, and more preferably an aromatic ring in which a part or all of hydrogen atoms in the aromatic ring are substituted by a monovalent group having no coordinating atom. This monovalent group is preferably an alkyl group, an aryl group or an aromatic heterocyclic group, more preferably an aryl group or an aromatic heterocyclic group, since the luminance life of the light emitting device becomes excellent.

Preferable as the above-described phosphorescent metal complex are iridium complexes such as $Ir(ppy)_3$ (described, for example, in Appl. Phys. Lett., (1999), 75(1), 4 and Jpn. J. Appl. Phys., 34, 1883(1995)), $Btp_2Ir(acac)$ (described, for example, in Appl. Phys. Lett., (2001), 78(11), 1622), ADS066GE commercially marketed from American Dye Source, Inc. (trade name) and the like containing iridium as the central metal, platinum complexes such as PtOEP and the like containing platinum as the central metal (described, for example, in Nature, (1998), 395, 151), and europium complexes such as $Eu(TTA)_3phen$ and the like containing europium as the central metal, and more preferable are iridium complexes.

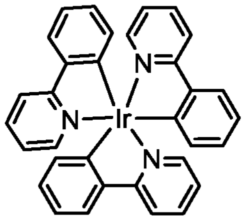

$Ir(ppy)_3$

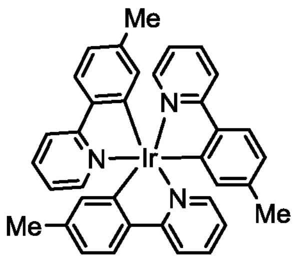

ADS066GE

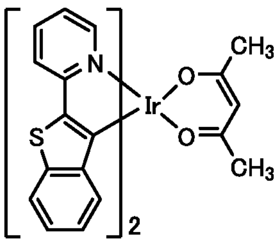

$Btp_2Ir(acac)$

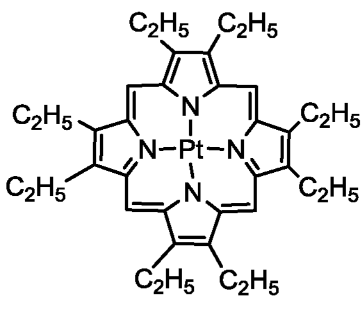

PtOEP

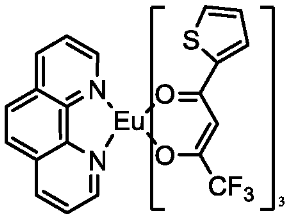

Eu(TTA)$_3$phen

As the above-described phosphorescent metal complex, complexes such as FIrpic, light emitting materials A to S and the like described in Proc. SPIE-Int. Soc. Opt. Eng. (2001), 4105 (Organic Light-Emitting Materials and Devices IV), 119, J. Am. Chem. Soc., (2001), 123, 4304, Appl. Phys. Lett., (1997), 71(18), 2596, Syn. Met., (1998), 97(2), 113, Syn. Met., (1999), 99(2), 127, Adv. Mater., (1999), 11(10), 852, Inorg. Chem., (2003), 42, 8609, Inorg. Chem., (2004), 43, 6513, Inorg. Chem., 2007, 46, 11082, Journal of the SID 11/1, 161 (2003), WO2002/066552, WO2004/020504, WO2004/020448 and the like can also be used, in addition to the above-described complexes.

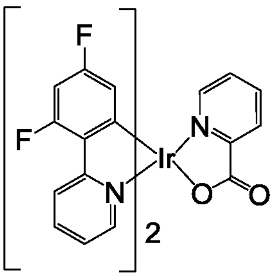

FIrpic

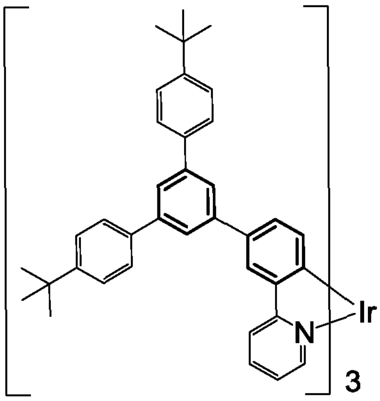

light emitting material A

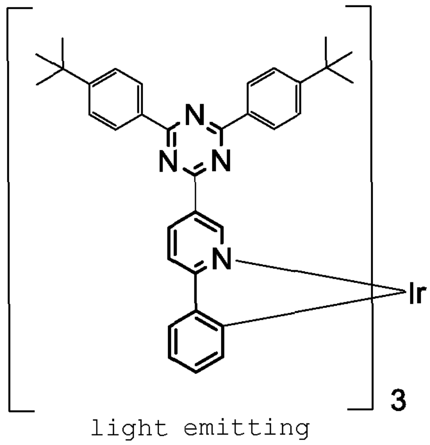

light emitting material B

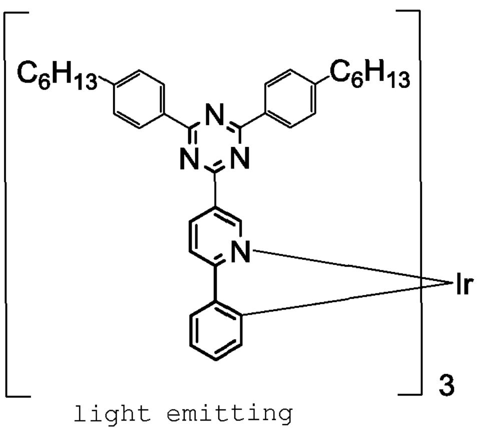

light emitting material C

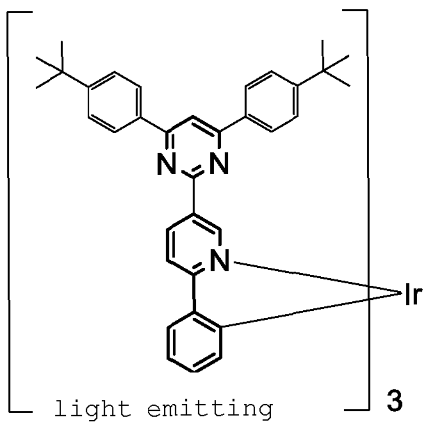

light emitting material D

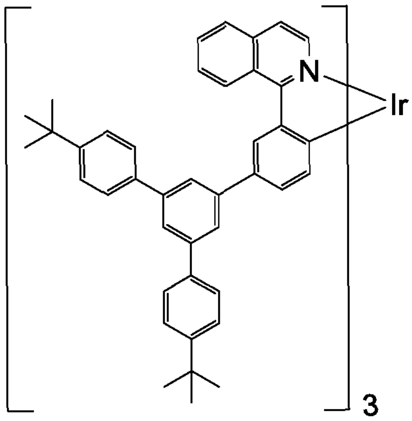

light emitting material E

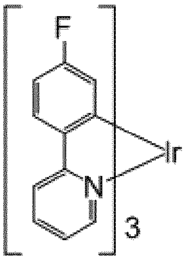

light emitting material F

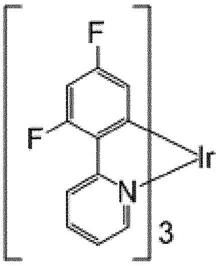

light emitting material G

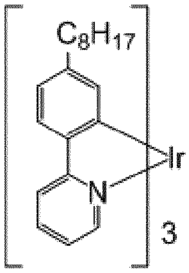

light emitting material H

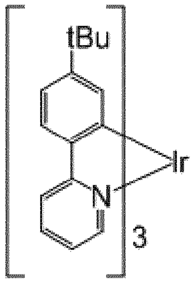

light emitting material I

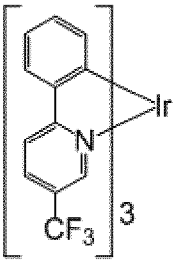

light emitting material J

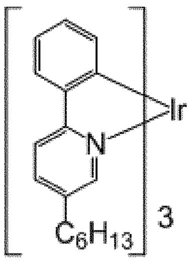

light emitting material K

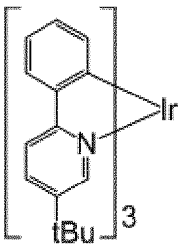

light emitting material L

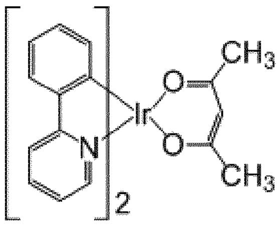

light emitting material M

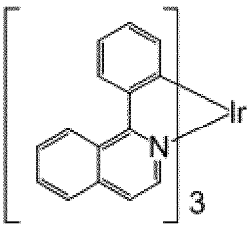

light emitting material N

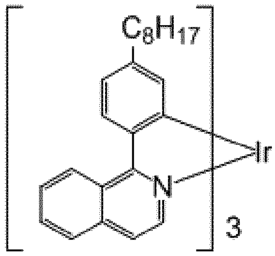

light emitting material O

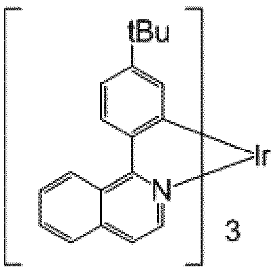

light emitting material P

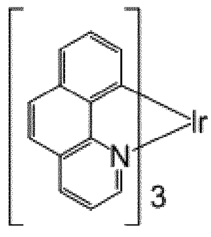

light emitting material Q

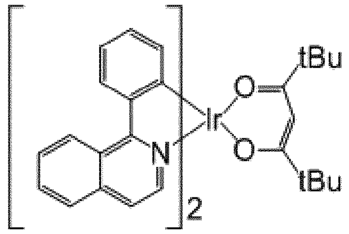

light emitting material R

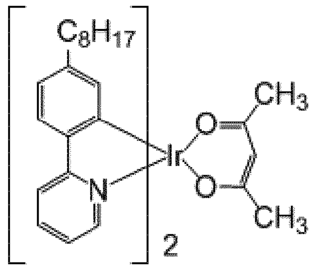

light emitting material S

The weight proportion of the first light emitting layer material with respect to the weight of the second light emitting layer material described later is usually 0.01 to 1.0, and from the viewpoint of goodness of the luminance life of the light emitting device, it is preferably 0.02 to 0.8, more preferably 0.05 to 0.65.

•Second light emitting layer material

The second light emitting layer material is usually composed only of a charge transporting polymer compound (that is, only a charge transporting polymer compound as an essential component), however, additionally, a charge transporting low molecular weight compound such as an aromatic amine, a carbazole derivative, a polyparaphenylene derivative, an oxadiazole derivative, anthraquinodimethane and its derivatives, benzoquinone and its derivatives, naphthoquinone and its derivatives, anthraquinone and its derivatives, tetracyanoanthraquinodimethane and its derivatives, diphenoquinone and its derivatives, triazine and its derivatives, a metal complex of 8-hydroxyquinoline and its derivatives, and the like may also be contained. The components constituting the second light emitting layer material may each be composed of a single compound or two or more compounds. The second light emitting layer material is, in general, called a host material in some cases.

The above-described charge transporting polymer compound is preferably a polymer compound containing at least one repeating unit selected from the group consisting of repeating units represented by the following formula (4) :

$$\{Ar^1\} \qquad (4)$$

in the formula (4), $Ar^1$ represents an arylene group, a divalent aromatic heterocyclic group, or a divalent group composed of two or more directly linked identical or different groups selected from the group consisting of the arylene group and the divalent aromatic heterocyclic group, wherein the group represented by $Ar^1$ may have an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group, a fluorine atom or a cyano group as a substituent.]
and repeating units represented by the following formula (5) :

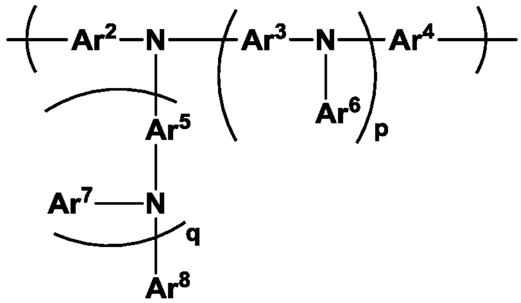

(5)

in the formula (5), $Ar^2$, $Ar^3$, $Ar^4$ and $Ar^5$ each independently represent an arylene group, a divalent aromatic heterocyclic group, or a divalent group composed of two or more directly linked identical or different groups selected from the group consisting of the arylene group and the divalent aromatic heterocyclic group; $Ar^6$, $Ar^7$ and $Ar^8$ each independently represent an aryl group or a monovalent aromatic heterocyclic group; p and q each independently represent 0 or 1, wherein the groups represented by $Ar^2$, $Ar^3$, $Ar^4$, $Ar^5$, $Ar^6$, $Ar^7$ and $Ar^8$ may have an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group, a fluorine atom or a cyano group

as a substituent, and the groups represented by $Ar^5$, $Ar^6$, $Ar^7$ and $Ar^8$ may each be linked directly or via -O-, -S-, -C(=O)-, -C(=O)-O-, -N($R^A$)-, -C(=O)-N($R^A$)- or -C($R^A$)$_2$- to the group represented by $Ar^2$, $Ar^3$, $Ar^4$, $Ar^5$, $Ar^6$, $Ar^7$ or $Ar^8$ linked to the nitrogen atom to which the groups are attached, thereby forming a 5 to 7-membered ring; $R^A$ represents an alkyl group, an aryl group, a monovalent aromatic heterocyclic group or an aralkyl group,
from the viewpoint of the charge injectability and the charge transportability. Of them, the above-described polymer compound includes more preferably polymer compounds in which the molar ratio of a repeating unit represented by the above-described formula (4) is 80% or more and the molar ratio of a repeating unit represented by the above-described formula (5) is less than 20%, and particularly preferably polymer compounds in which the molar ratio of a repeating unit represented by the above-described formula (4) is 90% or more and the molar ratio of a repeating unit represented by the above-described formula (5) is less than 10%.

The repeating unit represented by the above-described formula (4) is more preferably a repeating unit represented by the following formula (6):

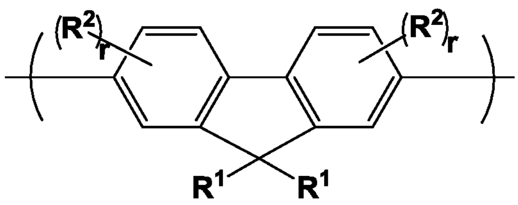

**(6)**

in the formula (6), each $R^1$ represents an alkyl group, an aryl group, a monovalent aromatic heterocyclic group or an aralkyl group; each $R^2$ represents an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group, a fluorine atom or a cyano group; each r represents an integer of 0 to 3, wherein two R’ moieties may be the same or different, and two $R^1$ moieties may be linked to form a ring; when a plurality of $R^2$ moieties are present, these may be the same or different; two characters of r may be the same or different,
or a repeating unit represented by the following formula (7) :

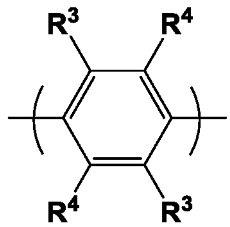

**(7)**

in the formula (7), each $R^3$ represents an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group or a cyano group; each $R^4$ represents a hydrogen atom, an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group, a fluorine atom or a cyano group, wherein two $R^3$ moieties may be the same or different, and two $R^4$ moieties may be the same or different,
from the viewpoint of the charge injectability and the charge transportability.

It is more preferable from the viewpoint of the driving voltage that a repeating unit represented by the following formula (6) and/or a repeating unit represented by the following formula (7) is contained as the repeating unit represented by the above-described formula (4).

The arylene group represented by $Ar^1$ in the above-described formula (4) and the arylene groups represented by $Ar^2$ to $Ar^5$ in the above-described formula (5) are an atomic group obtained by removing two hydrogen atoms from an aromatic hydrocarbon and include groups having a condensed ring, and groups having two or more independent benzene rings or condensed rings or both of them linked directly or via a conjugated connecting group such as a vinylene group and the like. The arylene group may have a substituent. The carbon atom number of a portion of the arylene group excluding the substituent is usually 6 to 60, and the total carbon atom number including the substituent is usually 6 to 100.

The substituent which the above-described arylene group may have includes preferably an alkyl group, an alkenyl group, an alkynyl group, an alkoxy group, an aryl group, an aryloxy group, a halogen atom and a cyano group from the viewpoint of the polymerizability and easiness of synthesis of a monomer, preferably an alkenyl group and an alkynyl group from the viewpoint of easiness of fabrication of an organic electroluminescent device, and preferably an

alkyl group, an alkenyl group, an alkynyl group and an aryl group from the viewpoint of the light emission property when made into a device.

The above-described arylene group includes phenylene groups (the formulae Ar1 to Ar3), naphthalenediyl groups (the formulae Ar4 to Ar13), anthracenediyl groups (the formulae Ar14 to Ar19), biphenyldiyl groups (the formulae Ar20 to Ar25), terphenyldiyl groups (the formulae Ar26 to Ar28), condensed ring compound groups (the formulae Ar29 to Ar35), fluorenediyl groups (the formulae Ar36 to Ar68) and benzofluorenediyl groups (the formulae Ar69 to Ar88). Phenylene groups, biphenyldiyl groups, terphenyldiyl groups and fluorenediyl groups are preferable, phenylene groups and fluorenediyl groups are more preferable and fluorenediyl groups are particularly preferable, from the viewpoint of the light emission property when made into a device. These groups may have a substituent.

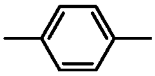
**Ar1**

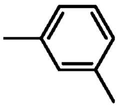
**Ar2**

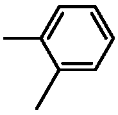
**Ar3**

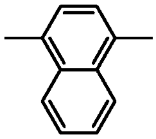
**Ar4**

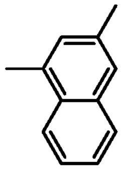
**Ar5**

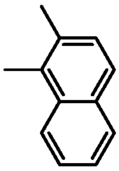
**Ar6**

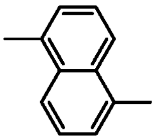
**Ar7**

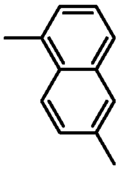
**Ar8**

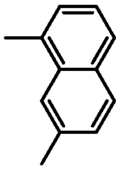
**Ar9**

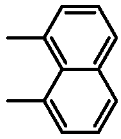
**Ar10**

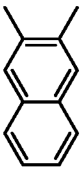
**Ar11**

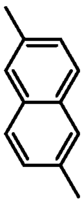
**Ar12**

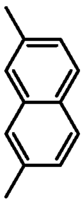
**Ar13**

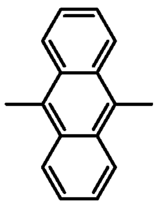
**Ar14**

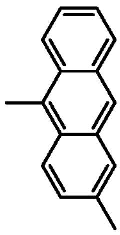
**Ar15**

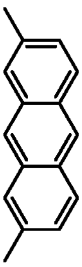
**Ar16**

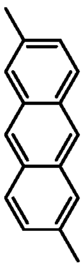
**Ar17**

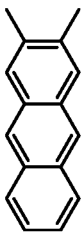
**Ar18**

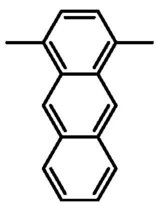
**Ar19**

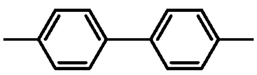
**Ar20**

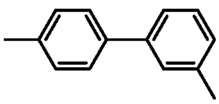
**Ar21**

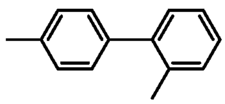
**Ar22**

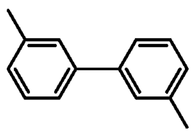
**Ar23**

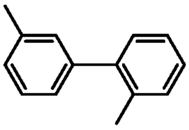
Ar24

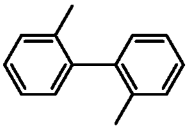
Ar25

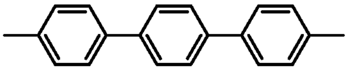
Ar26

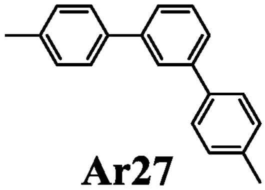
Ar27

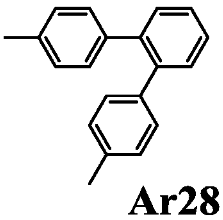
Ar28

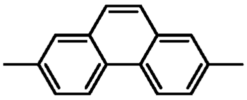
Ar29

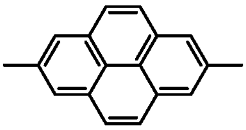
Ar30

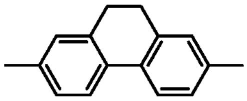
Ar31

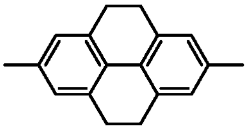
Ar32

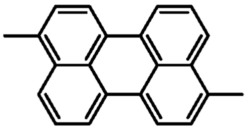
Ar33

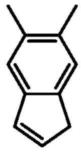
Ar34

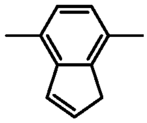
Ar35

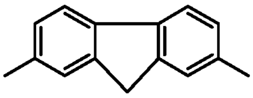
Ar36

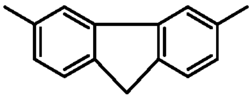
Ar37

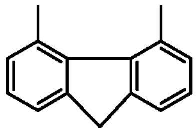
Ar38

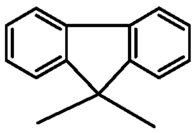
Ar40

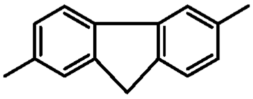
Ar41

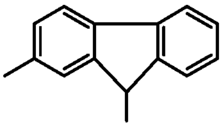
Ar42

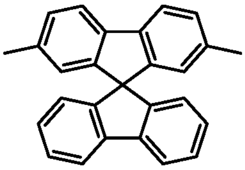
Ar43

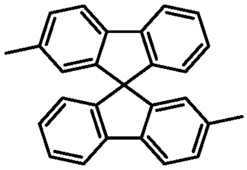
Ar44

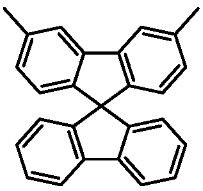
Ar45

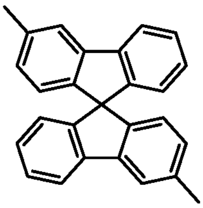
Ar46

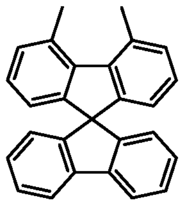
Ar47

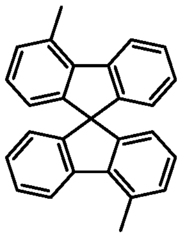
Ar48

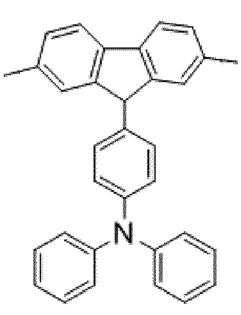

Ar49

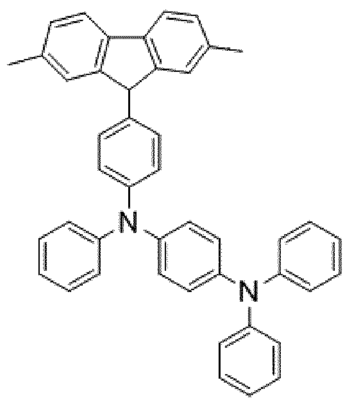

Ar50

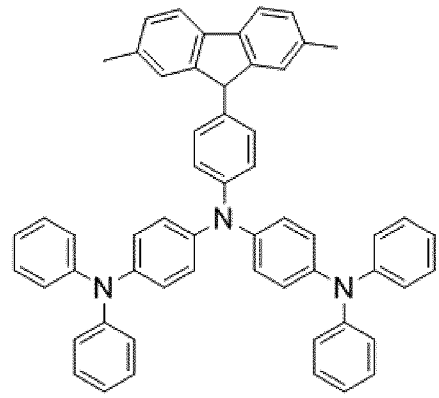

Ar51

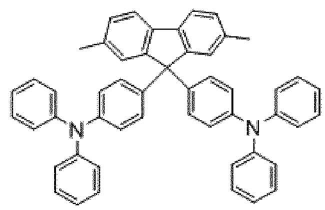

Ar52

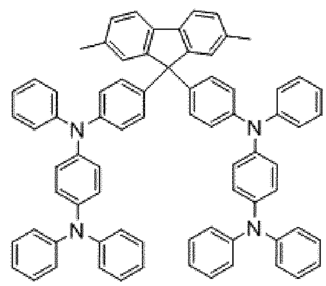

Ar53

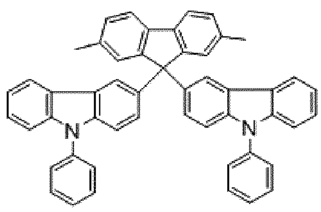

Ar54

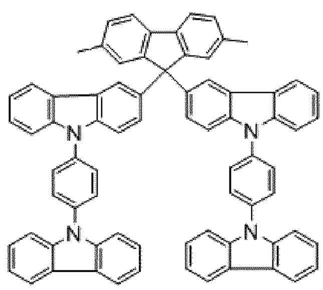

Ar55

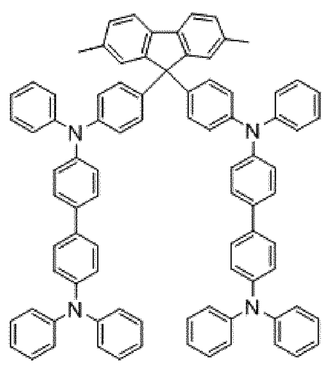

Ar56

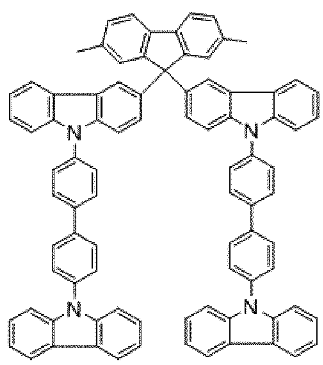

Ar57

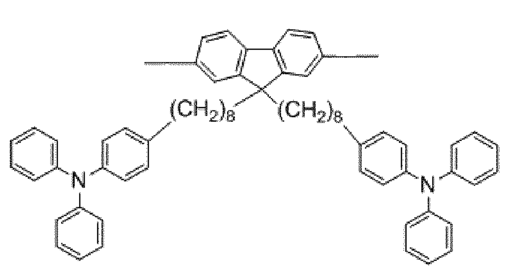

Ar58

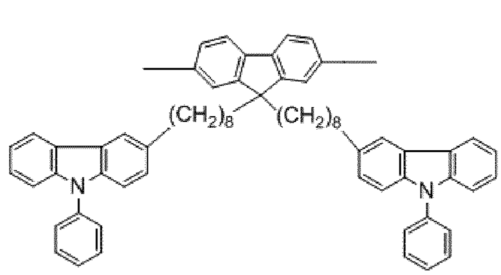

Ar59

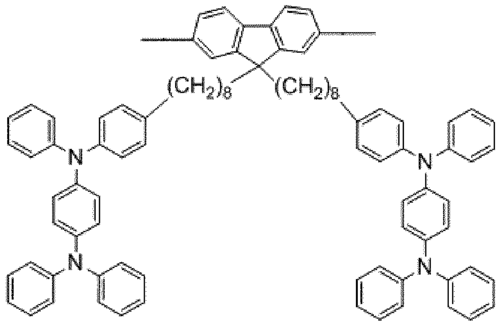

Ar60

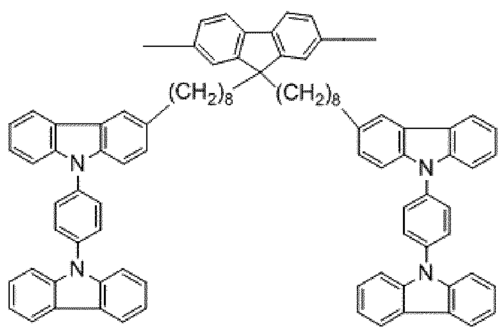

Ar61

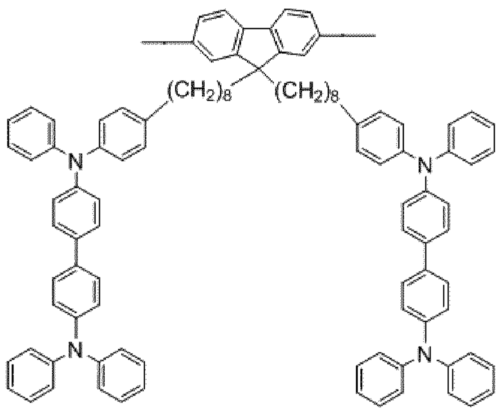

Ar62

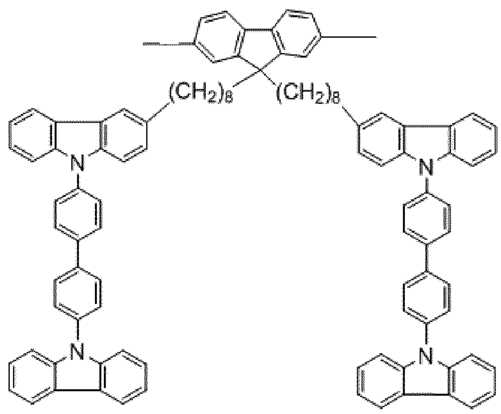

Ar63

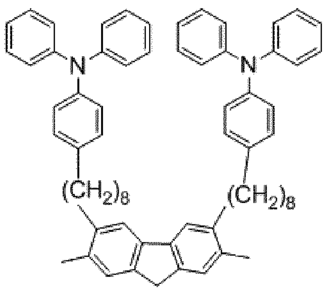

Ar64

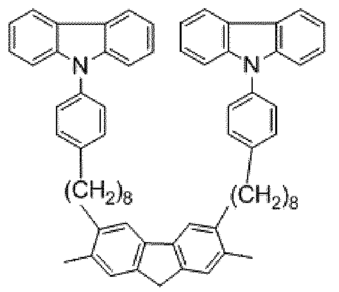

Ar65

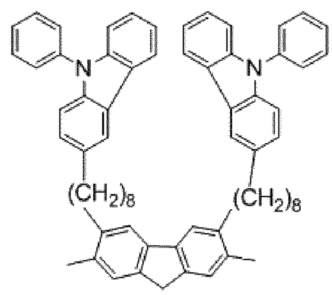

Ar66

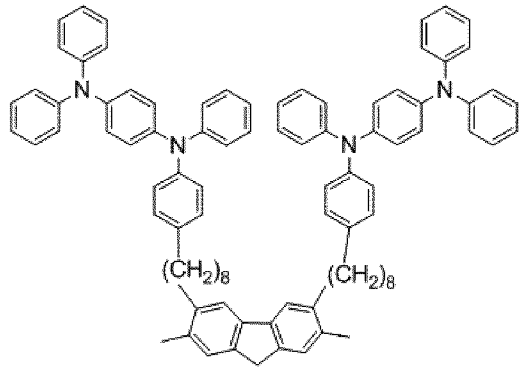

Ar67

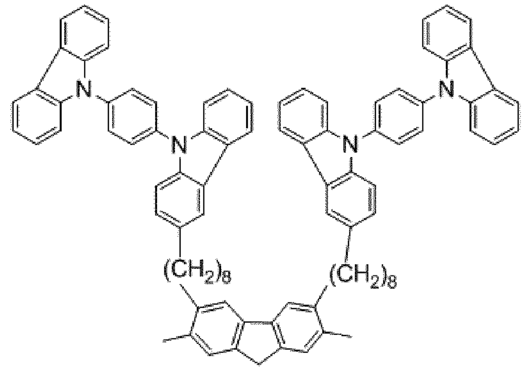

Ar68

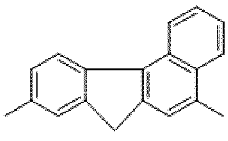
Ar69

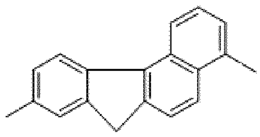
Ar70

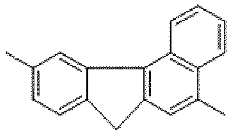
Ar71

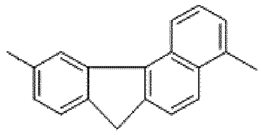
Ar72

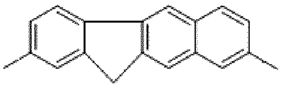
Ar73

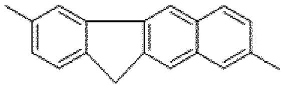
Ar74

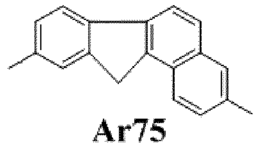
Ar75

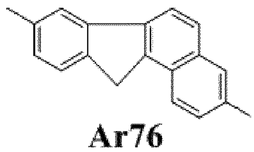
Ar76

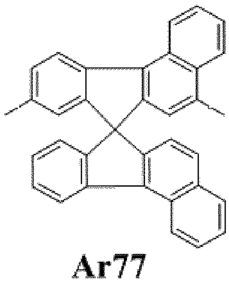
Ar77

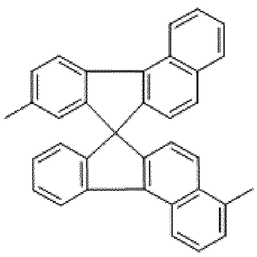
Ar78

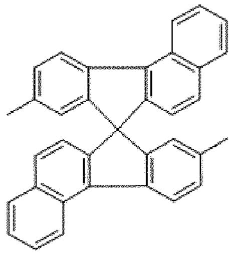
Ar79

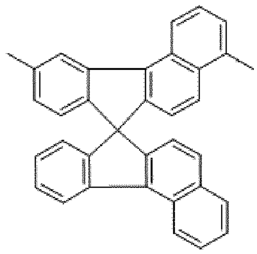
Ar80

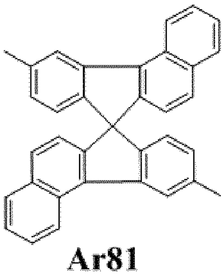
Ar81

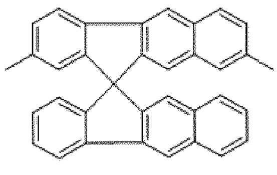
Ar82

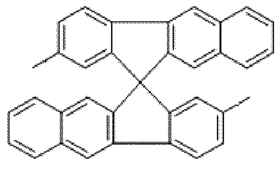
Ar83

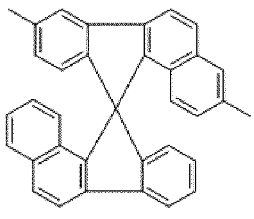
Ar84

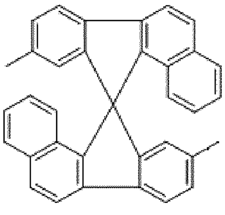
Ar85

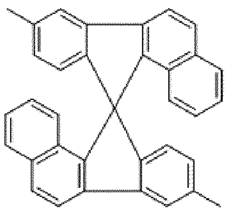
Ar86

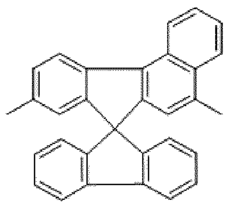
Ar87

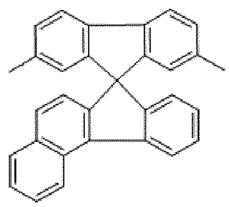
Ar88

In the above-described formula (4), the divalent aromatic heterocyclic group represented by $Ar^1$ means an atomic group remaining after removal of two hydrogen atoms from an aromatic heterocyclic compound. The aromatic heterocyclic compound includes heterocyclic compounds containing a hetero atom wherein the hetero ring itself shows an aromatic property, such as oxadiazole, thiadiazole, thiazole, oxazole, thiophene, pyrrole, phosphole, furan, pyridine, pyrazine, pyrimidine, triazine, pyridazine, quinoline, isoquinoline, carbazole and dibenzophosphole, and compounds wherein even if the hetero ring itself containing a hetero atom shows no aromatic property, an aromatic ring is condensed to the hetero ring, such as phenoxazine, phenothiazine, dibenzoborole, dibenzosilole and benzopyran. Examples of the above-described divalent aromatic heterocyclic group include pyridinediyl groups (the formulae B1 to B3); diazaphenylene groups (the formulae B4 to B8); triazinediyl groups (the formula B9); quinoline-diyl groups (the formulae B10 to B12); quinoxaline-diyl groups (the formulae B13 to B15); acridinediyl groups (the formulae B16 and B17); phenanthrolinediyl groups (the formulae B18 and B19); groups having a structure in which a benzo ring is condensed to a cyclic structure containing a hetero atom (the formulae B20 to B26); phenoxazinediyl groups (the formulae B27 and B28);

phenothiazinediyl groups (the formulae B29 and B30); nitrogen bond-containing polycyclic diyl groups (the formulae B31 to B35); 5-membered ring groups containing an oxygen atom, a sulfur atom, a nitrogen atom, a silicon atom and the like as a hetero atom (the formulae B36 to B39); and 5-membered ring condensed groups containing an oxygen atom, a sulfur atom, a nitrogen atom, a silicon atom and the like as a hetero atom (the formulae B40 to B47). A hydrogen atom in these divalent aromatic heterocyclic groups may be substituted by an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group, a fluorine atom or a cyano group.

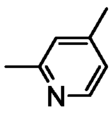
B1
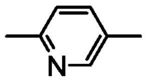
B2
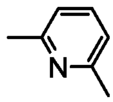
B3
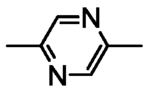
B4
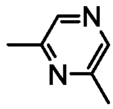
B5
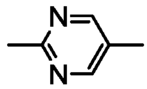
B6
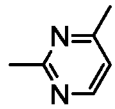
B7

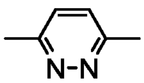
B8
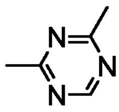
B9
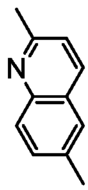
B10
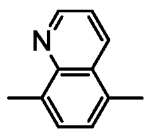
B11
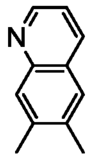
B12
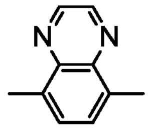
B13
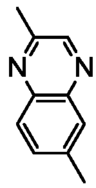
B14

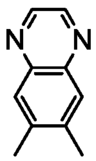
B15
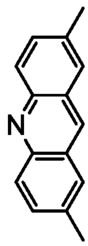
B16
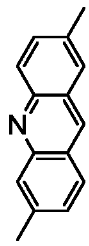
B17
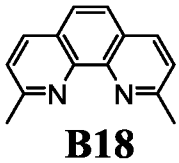
B18
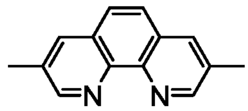
B19

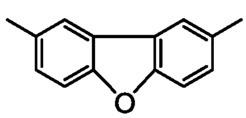
B20
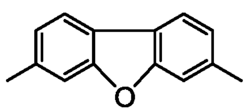
B21
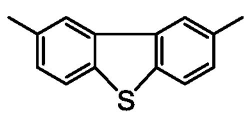
B22
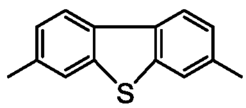
B23

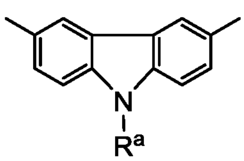
B24
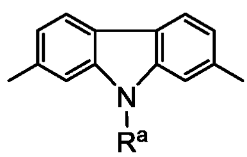
B25
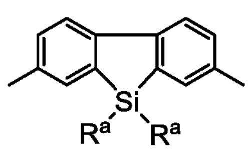
B26
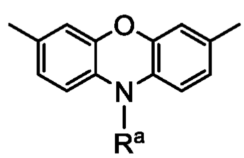
B27

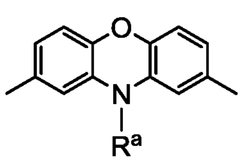
B28
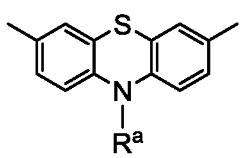
B29
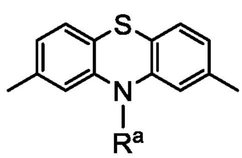
B30
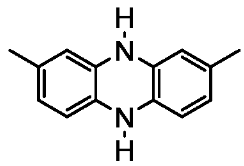
B31

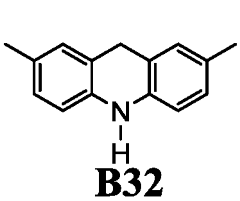
B32

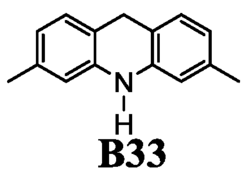
B33

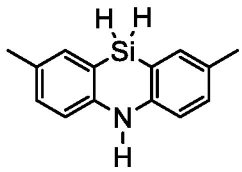
B34

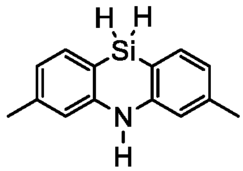
B35

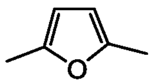
B36

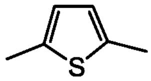
B37

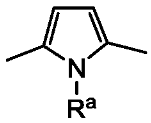
B38

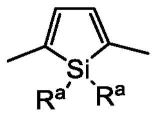
B39

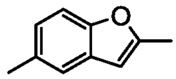
B40

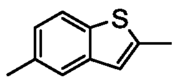
B41

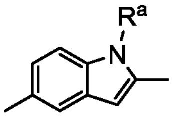
B42

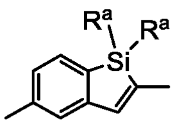
B43

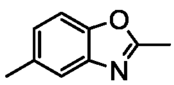
B44

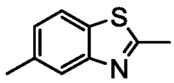
B45

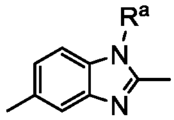
B46

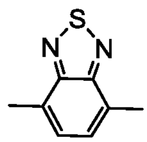
B47

[wherein $R^a$ represents a hydrogen atom, a hydroxyl group, an alkyl group, an aryl group, a monovalent aromatic heterocyclic group, an alkoxy group, an aryloxy group, an aralkyl group or an arylalkoxy group.].

The repeating unit represented by the above-described formula (4) includes repeating units represented by the following formulae Ka-1 to Ka-52.

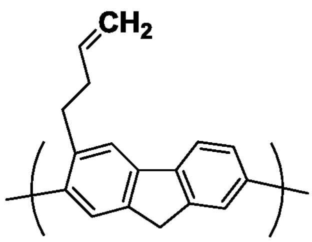
Ka-1

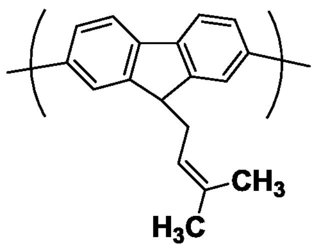
Ka-2

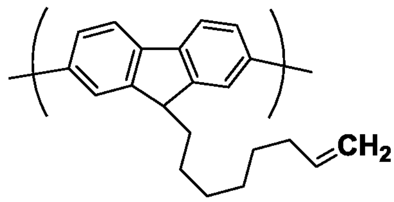
Ka-3

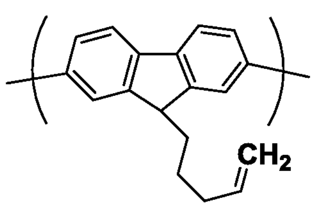
Ka-4

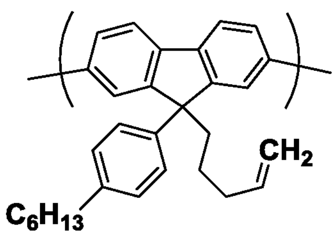
Ka-5

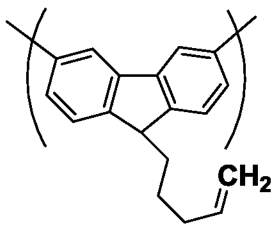
Ka-6

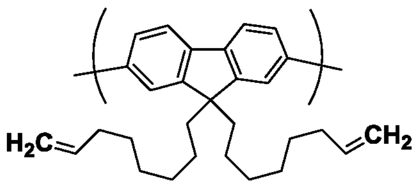

Ka-7

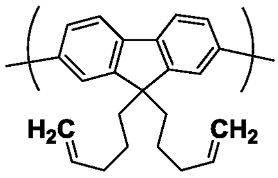

Ka-8

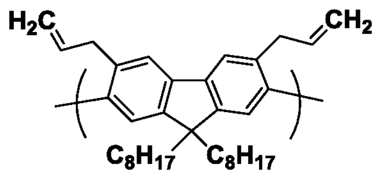

Ka-9

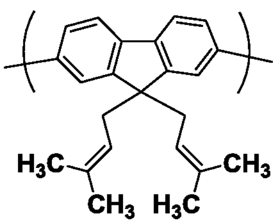

Ka-10

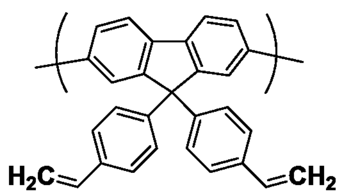

Ka-11

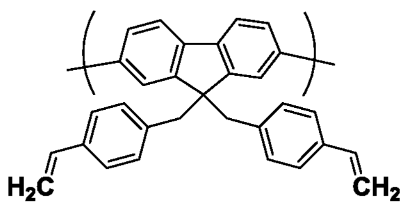

Ka-12

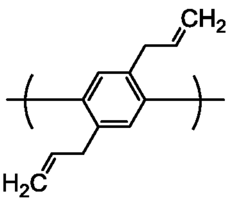

Ka-13

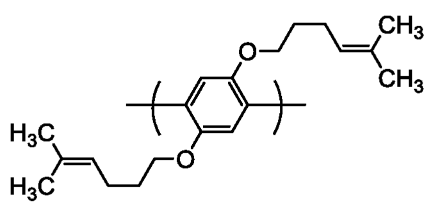

Ka-14

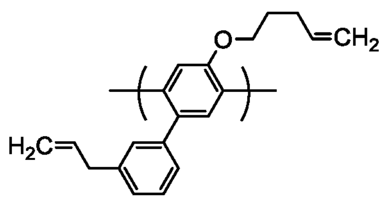

Ka-15

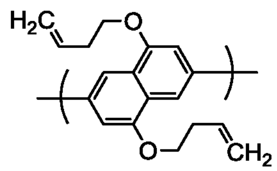

Ka-16

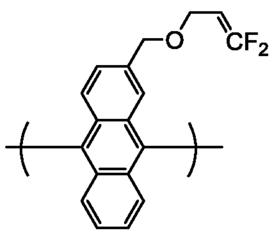

Ka-17

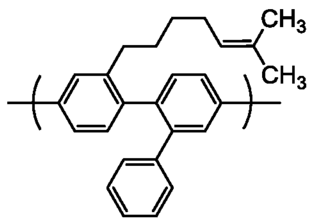

Ka-18

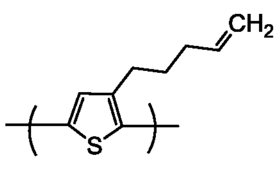

Ka-19

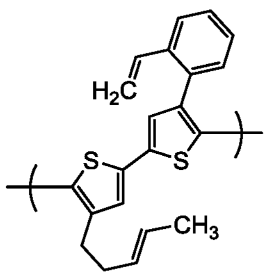

Ka-20

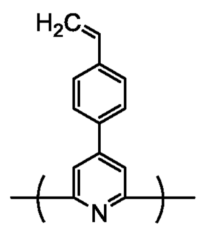

Ka-21

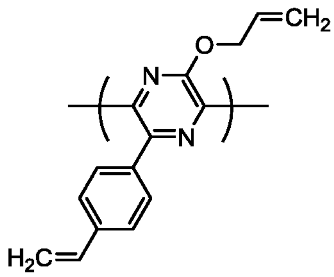

Ka-22

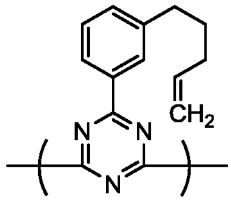

Ka-23

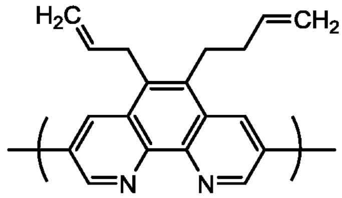

Ka-24

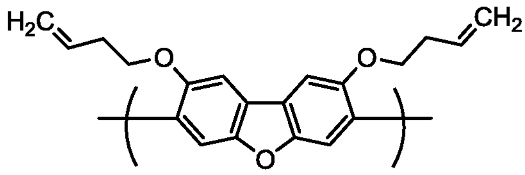

Ka-25

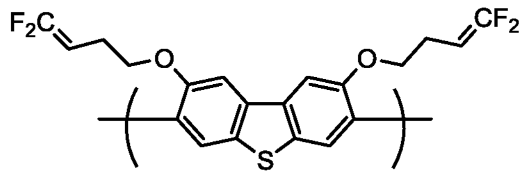

Ka-26

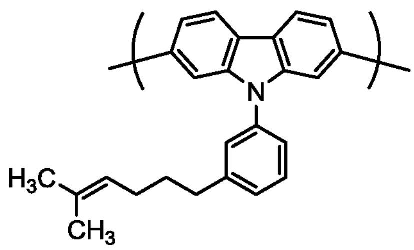

Ka-27

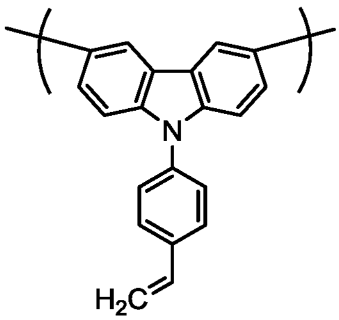

Ka-28

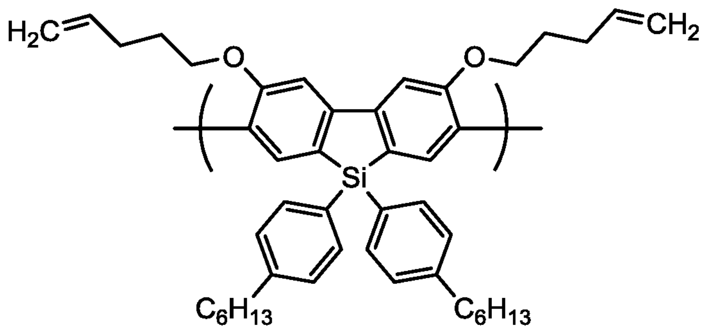

Ka-29

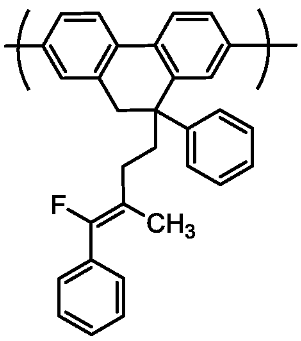

Ka-30

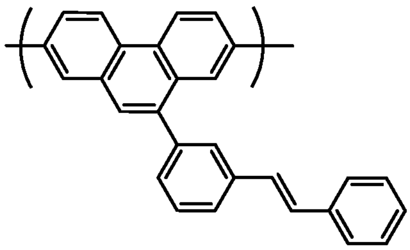

Ka-31

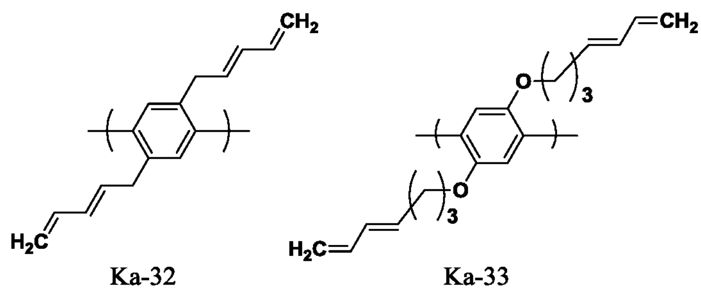

Ka-32 Ka-33

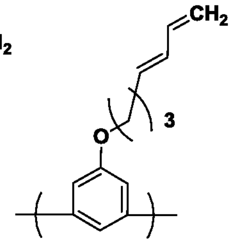

Ka-34

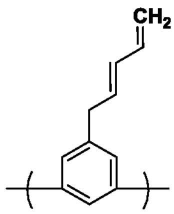

Ka-35

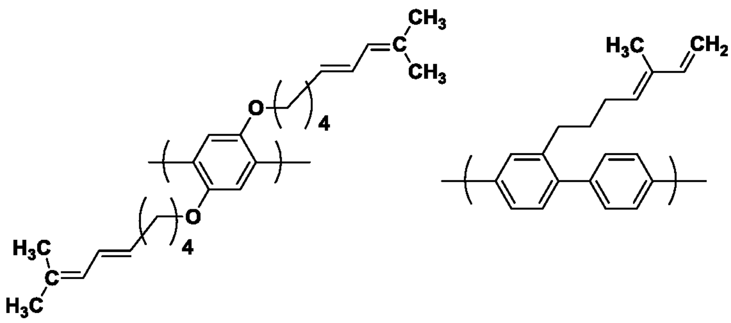

Ka-36 Ka-37

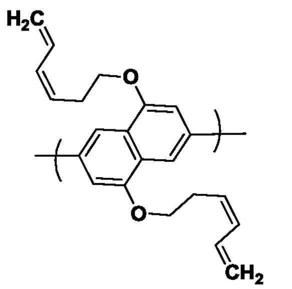

Ka-38

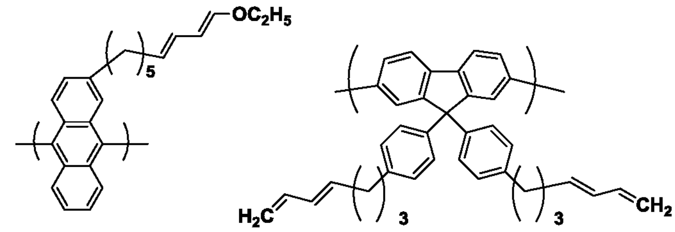

Ka-39 Ka-40

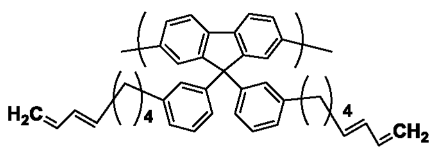

Ka-41

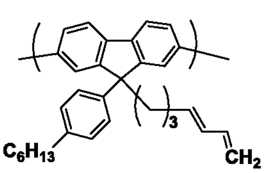

Ka-42

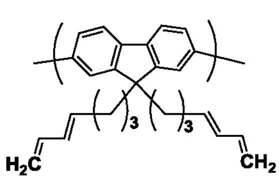

Ka-43

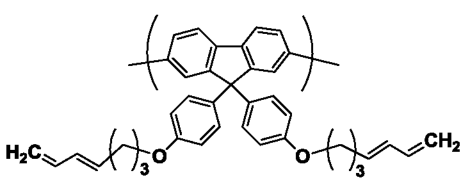

Ka-44

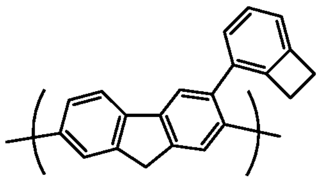

Ka-45

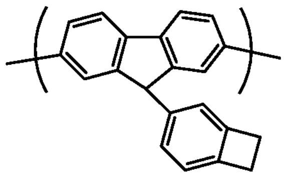

Ka-46

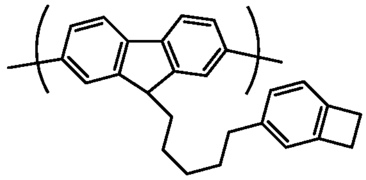

Ka-47

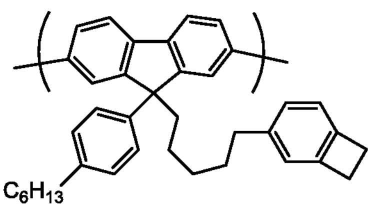

Ka-48

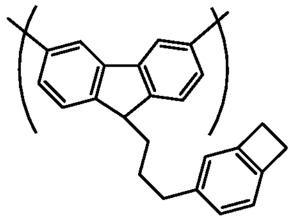

Ka-49

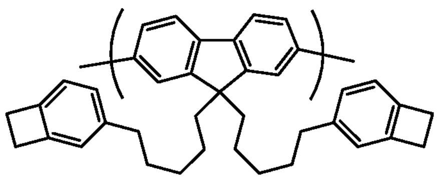

Ka-50

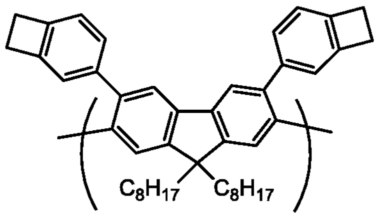

Ka-51

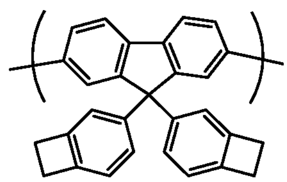

Ka-52

The aryl groups represented by $Ar^6$, $Ar^7$ and $Ar^8$ in the above-described formula (5) are an atomic group obtained by removing one hydrogen atom from an aromatic hydrocarbon, and include groups having a condensed ring. The above-described aryl group has a carbon atom number of usually 6 to 60, preferably 6 to 48, more preferably 6 to 20. This carbon atom number does not include the carbon atom number of the substituent. Examples of the above-described aryl group are a phenyl group, a 1-naphthyl group, a 2-naphthyl group, a 1-anthryl group, a 2-anthryl group, a 9-anthryl group, a 1-pyrenyl group, a 2-pyrenyl group, a 4-pyrenyl group, a 1-phenanthryl group, a 2-phenanthryl group, a 3-phenanthryl group, a 4-phenanthryl group, a 9-phenanthryl group, a 2-fluorenyl group, a 3-fluorenyl group, a 9-fluorenyl group, a 2-perylenyl group, a 3-perylenyl group and a 4-biphenylyl group. The above-described aryl group may have a substituent.

As the above-described aryl group, a substituted or unsubstituted phenyl group and a substituted or unsubstituted 4-biphenylyl group are preferable. The substituent on the phenyl group and the 4-biphenylyl group includes preferably an alkyl group, a monovalent aromatic heterocyclic group, an alkoxy group and an aryloxy group, more preferably an alkyl group.

The monovalent aromatic heterocyclic groups represented by $Ar^6$, $Ar^7$ and $Ar^8$ in the above-described formula (5) are an atomic group obtained by removing one hydrogen atom from an aromatic heterocyclic compound, and include groups having a condensed ring. The above-described monovalent aromatic heterocyclic group has a carbon atom number of usually 3 to 60, preferably 3 to 20. This carbon atom number does not include the carbon atom number of the substituent. The above-described monovalent aromatic heterocyclic group includes a 2-oxadiazole group, a 2-thiadiazole group, a 2-thiazole group, a 2-oxazole group, a 2-thienyl group, a 2-pyrrolyl group, a 2-furyl group, a 2-pyridyl group, a 3-pyridyl group, a 4-pyridyl group, a 2-pyrazyl group, a 2-pyrimidyl group, a 2-triazyl group, a 3-pyridazyl group, a 3-carbazolyl group, a 2-phenoxazinyl group, a 3-phenoxazinyl group, a 2-phenothiazinyl group, a 3-phenothiazinyl group and the like, preferably a 2-pyridyl group, a 3-pyridyl group, a 4-pyridyl group, a 2-pyrazyl group, a 2-pyrimidyl group, a 2-triazyl group and a 3-pyridazyl group. The above-described monovalent aromatic heterocyclic group may have a substituent. This substituent includes preferably an alkyl group, an aryl group and a monovalent aromatic heterocyclic group.

The divalent aromatic heterocyclic groups represented by $Ar^2$ to $Ar^5$ in the above-described formula (5) have the same meaning as the divalent aromatic heterocyclic group represented by $Ar^1$ in the above-described formula (4).

It is preferable that at least one of p and q is 1 in the above-described formula (5).

The repeating unit represented by the above-described formula (5) includes repeating units represented by the

following formulae Am1 to Am6 and Kb-1 to Kb-7, and from the viewpoint of the light emission property and the hole transportability when made into a device, preferably includes repeating units represented by the formulae Am2 to Am5. These repeating units may have a substituent.

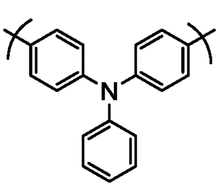

Am1

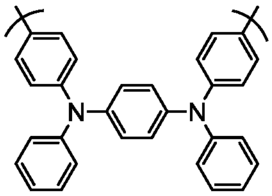

Am2

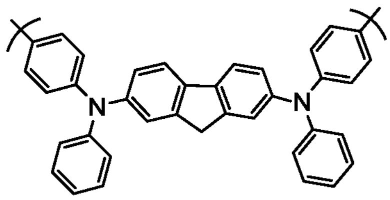

Am3

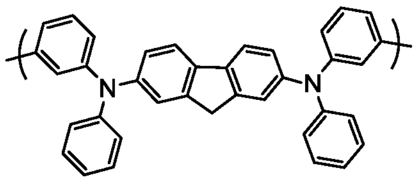

Am4

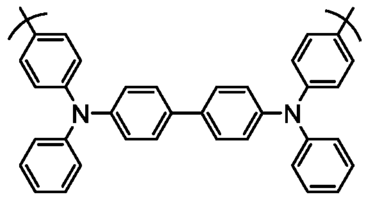

Am5

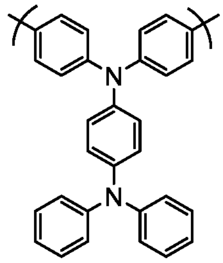

Am6

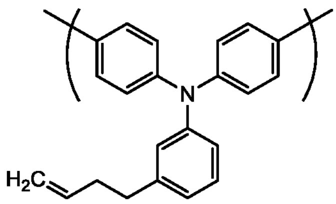

Kb-1

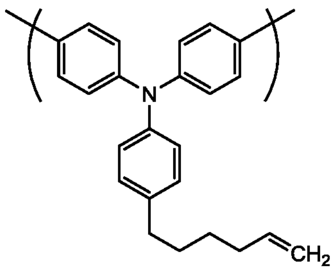

Kb-2

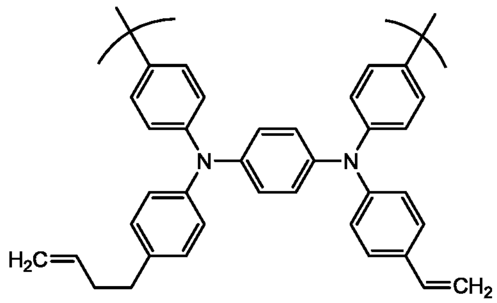

Kb-3

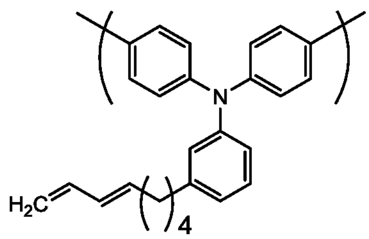

Kb-4

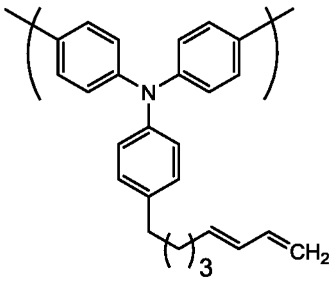

Kb-5

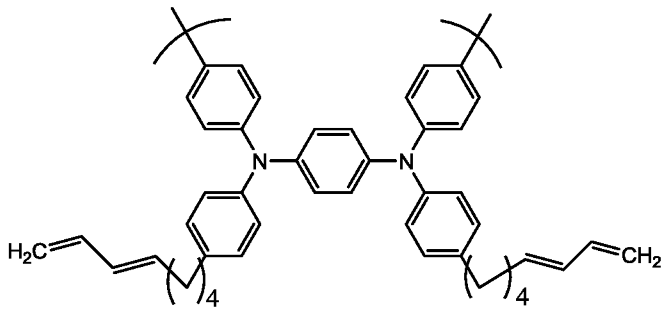

Kb-6

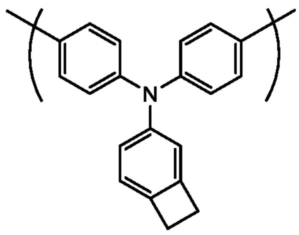

Kb-7

The above-described charge transporting polymer compound may also be a compound obtained by cross-linkage of the charge transporting polymer compound as described above.

The above-described charge transporting polymer compound has a polystyrene-equivalent weight-average molecular weight of usually $1\times10^3$ to $1\times10^8$, preferably $5\times10^4$ to $5\times10^6$. The above-described charge transporting polymer compound has a polystyrene-equivalent number-average molecular weight of usually $1\times10^3$ to $1\times10^8$, preferably $1\times10^4$ to $1\times10^6$.

The above-described charge transporting polymer compound includes the following compounds EP-1 to EP-4.

Table 1

| compounds | repeating units and molar ratio thereof | | | | |
|---|---|---|---|---|---|
| | formulae Ar1 to Ar35 | formulae Ar36 to Ar67 | formulae B1 to B42 | formulae Am1 to Am6 | others |
| | v | w | x | y | z |
| EP-1 | 0.001 to 0.999 | 0.001 to 0.999 | 0 | 0 | 0 to 0.3 |
| EP-2 | 0.001 to 0.998 | 0.001 to 0.998 | 0.001 to 0.998 | 0 | 0 to 0.3 |
| EP-3 | 0.001 to 0.998 | 0.001 to 0.998 | 0 | 0.001 to 0.198 | 0 to 0.3 |
| EP-4 | 0.001 to 0.997 | 0.001 to 0.997 | 0.001 to 0.997 | 0.001 to 0.197 | 0 to 0.3 |

(in the table, v, w, x, y and z are numbers showing the molar ratios. Of them, the molar ratios of repeating units represented by the above-described formula (4) are represented by v, w and x, the molar ratio of a repeating unit represented by the above-described formula (5) is represented by y, and the molar ratio of other repeating units is represented by z. v, w, x, y and z satisfy conditions: $v + w + x + y + z = 1$ and $1 \geq v + w + x + y \geq 0.7$.) .

Here, the above-described formulae Ar1 to Ar35, formulae Ar36 to Ar67, formulae B1 to B42 and formulae Am1 to Am6 have the same meaning as described above. "Others" mean repeating units other than the above-described formulae Ar1 to Ar35, formulae Ar36 to Ar67, formulae B1 to B42 and formulae Am1 to Am6.

As the above-described charge transporting polymer compound, a single compound may be contained or two or more compounds may be contained. When two or more charge transporting polymer compounds are contained, the molar ratios of repeating units represented by the above-described formulae (4) and (5) indicate an arithmetic average value, namely, the sum of products obtained by multiplying the molar ratios of respective charge transporting polymer compounds by the composition ratios by weight of respective charge transporting polymer compounds.

•Other materials

In the organic electroluminescent device of the present invention, the above-described light emitting layer may contain the first light emitting layer material and the second light emitting layer material, and other components.

<Hole transporting layer>

•Hole transporting polymer compound

The above-described hole transporting polymer compound is a polymer compound containing a repeating unit represented by the above-described formula (4) and a repeating unit represented by the above-described formula (5), preferably a polymer compound containing a repeating unit represented by the above-described formula (5) in a molar ratio of 20% or more, more preferably a polymer compound containing a repeating unit represented by the above-described formula (5) in a molar ratio of 30% or more, from the viewpoint of the hole injectability and the hole transportability.

The repeating unit represented by the above-described formula (4) is preferably a repeating unit represented by the above-described formula (6) or a repeating unit represented by the above-described formula (7), and from the viewpoint of the hole transportability, a repeating unit represented by the above-described formula (6) is more preferable.

The above-described hole transporting polymer compound has a polystyrene-equivalent weight-average molecular weight of usually $1\times10^3$ to $1\times10^8$, preferably $5\times10^4$ to $5\times10^6$. The above-described hole transporting polymer compound has a polystyrene-equivalent number-average molecular weight of usually $1\times10^3$ to $1\times10^8$, preferably $1\times10^4$ to $1\times10^6$.

The above-described hole transporting polymer compound includes the following compounds EP-5 to EP-10.

Table 2

| | repeating units and molar ratio thereof | | | | |
|---|---|---|---|---|---|
| | formulae Ar1 to Ar35 | formulae Ar36 to Ar67 | formulae B1 to B42 | formulae Am1 to Am6 | others |
| | v' | w' | x' | y' | z' |
| EP-5 | 0.001 to 0.8 | 0 | 0 | 0.2 to 0.999 | 0 to 0.3 |
| EP-6 | 0 | 0.001 to 0.8 | 0 | 0.2 to 0.999 | 0 to 0.3 |
| EP-7 | 0.001 to 0.799 | 0.001 to 0.799 | 0 | 0.2 to 0.998 | 0 to 0.3 |
| EP-8 | 0.001 to 0.799 | 0 | 0.001 to 0.799 | 0.2 to 0.998 | 0 to 0.3 |
| EP-9 | 0 | 0.001 to 0.799 | 0.001 to 0.799 | 0.2 to 0.998 | 0 to 0.3 |
| EP-10 | 0.001 to 0.798 | 0.001 to 0.798 | 0.001 to 0.798 | 0.2 to 0.997 | 0 to 0.3 |

(in the table, v', w', x', y' and z' are numbers showing the molar ratios. Of them, the molar ratios of repeating units represented by the above-described formula (4) are represented by v', w' and x', the molar ratio of a repeating unit represented by the above-described formula (5) is represented by y', and the molar ratio of other repeating units is represented by z'. v', w', x', y' and z' satisfy conditions: $v' + w' + x' + y' + z' = 1$ and $1 \geq v' + w' + x' + y' \geq 0.7$.).

Here, the above-described formulae Ar1 to Ar35, formulae Ar36 to Ar67, formulae B1 to B42 and formulae Am1 to Am6 have the same meaning as described above. "Others" mean repeating units other than the above-described formulae Ar1 to Ar35, formulae Ar36 to Ar67, formulae B1 to B42 and formulae Am1 to Am6.

As the above-described hole transporting polymer compound, a single compound may be contained or two or more compounds may be contained. When two or more hole transporting polymer compounds are contained, the molar ratios of repeating units represented by the above-described formulae (4) and (5) indicate an arithmetic average value, namely, the sum of products obtained by multiplying the molar ratios of respective hole transporting polymer compounds by the composition ratios by weight of respective hole transporting polymer compounds.

In the organic electroluminescent device of the present invention, a crosslinkable hole transporting polymer compound may be used as the above-described hole transporting polymer compound, and cross-linked in its molecule or between molecules thereof in a process of device production, to be contained under cross-linked condition in the hole transporting layer, from the viewpoint of insolubilization into a solvent in fabrication of the device.

•Other material

In the organic electroluminescent device of the present invention, the above-described hole transporting layer may be formed by using the above-described hole transporting polymer compound, and other components.

<Device constitution>

The layer structure of the organic electroluminescent device of the present invention includes the following structures a) to b).

a) anode/hole transporting layer/light emitting layer/cathode
b) anode/hole transporting layer/light emitting layer/electron transporting layer/cathode

(wherein "/" indicates contact lamination of layers. The same shall apply hereinafter.)

Of the hole transporting layer and the electron transporting layer disposed so as to be in contact with an electrode, one having a function of improving the efficiency of injection of charges (holes, electrons) from the electrode and manifesting an effect of lowering the driving voltage of a device is called a charge injection layer.

In the organic electroluminescent device of the present invention, it is preferable that a hole injection layer is present between the above-described anode and the above-described hole transporting layer. In the organic electroluminescent device of the present invention, an insulation layer may be disposed so as to be in contact with an electrode. For improvement of close adherence of an interface, prevention of mixing and the like, a thin buffer layer may be inserted between the above-described anode and the above-described hole transporting layer and a thin buffer layer may be inserted between the above-described light emitting layer and the above-described cathode. The order and the number of layers to be laminated and the thickness of each layer may advantageously be regulated in view of the light emission efficiency and the luminance life.

The layer structure of the organic electroluminescent device having a charge injection layer includes the following structures c) to h).

c) anode/hole injection layer/hole transporting layer/light emitting layer/cathode
d) anode/hole transporting layer/light emitting layer/electron injection layer/cathode
e) anode/hole injection layer/hole transporting layer/light emitting layer/electron injection layer/cathode
f) anode/hole injection layer/hole transporting layer/light emitting layer/electron transporting layer/cathode
g) anode/hole transporting layer/light emitting layer/electron transporting layer/electron injection layer/cathode
h) anode/hole injection layer/hole transporting layer/light emitting layer/electron transporting layer/electron injection layer/cathode

The anode is usually transparent or semitransparent and constituted of a film made of a metal oxide, a metal sulfide or a metal having high electric conductivity, and of them, materials of high transmission are preferably used for its constitution. As the material of the above-described anode, use is made of films (NESA and the like) fabricated using an electric conductive inorganic compound composed of indium oxide, zinc oxide, tin oxide, and composite thereof: indium•tin•oxide (ITO), indium•zinc•oxide and the like, and gold, platinum, silver, copper and the like, and preferable are ITO, indium•zinc•oxide and tin oxide. For fabrication of the above-described anode, methods such as a vacuum vapor-deposition method, a sputtering method, an ion plating method, a plating method and the like can be used. As the above-described anode, organic transparent electric conductive films made of polyaniline and derivatives thereof, polythiophene and derivatives thereof, and the like may be used.

The thickness of the anode may advantageously be selected in view of the light transmission and the electric conductivity, and it is usually 10 nm to 10 $\mu$m, preferably 20 nm to 1 $\mu$m, more preferably 50 nm to 500 nm.

The material used in the hole injection layer includes phenylamine compounds, starburst type amine compounds, phthalocyanine compounds, oxides such as vanadium oxide, molybdenum oxide, ruthenium oxide, aluminum oxide and the like, and electric conductive polymer compounds such as amorphous carbon, polyaniline and derivatives thereof, polythiophene and derivatives thereof, and the like.

When the material used in the hole injection layer is an electric conductive polymer compound, an anion such as a polystyrene sulfonate ion, an alkylbenzene sulfonate ion, a camphor sulfonate ion and the like may be doped for improving the electric conductivity of the electric conductive polymer compound.

As the method for forming a hole transporting layer, film formation from a solution containing the above-described hole transporting polymer compound is used. The solvent used for film formation from a solution may advantageously be a solvent which dissolves the above-described hole transporting polymer compound. This solvent includes chlorine-based solvents such as chloroform, methylene chloride, dichloroethane and the like, ether solvents such as tetrahydrofuran and the like, aromatic hydrocarbon solvents such as toluene, xylene and the like, ketone solvents such as acetone, methyl ethyl ketone and the like, and ester solvents such as ethyl acetate, butyl acetate, ethyl cellosolve acetate and the like.

For formation of the hole transporting layer, coating methods such as a spin coat method, a casting method, a micro gravure coat method, a gravure coat method, a bar coat method, a roll coat method, a wire bar coat method, a dip coat method, a spray coat method, a screen printing method, a flexo printing method, an offset printing method, an inkjet print method and the like can be used.

The thickness of the hole transporting layer may advantageously be selected in view of the driving voltage and the light emission efficiency, and a thickness causing no generation of pin holes is necessary, and when it is too thick, the driving voltage of an organic electroluminescent device may increase in some cases. Therefore, the thickness of the hole transporting layer is usually 1 nm to 1 μm, preferably 2 nm to 500 nm, more preferably 5 nm to 200 nm.

The method for forming a light emitting layer includes a method for coating a solution containing the first light emitting layer material and the second light emitting layer material on or above the hole transporting layer, and the like. The solvent to be used in the above-described solution may advantageously be a solvent which dissolves the first light emitting layer material and the second light emitting layer material. This solvent includes chlorine-based solvents such as chloroform, methylene chloride, dichloroethane and the like, ether solvents such as tetrahydrofuran and the like, aromatic hydrocarbon solvents such as toluene, xylene and the like, ketone solvents such as acetone, methyl ethyl ketone and the like, and ester solvents such as ethyl acetate, butyl acetate, ethyl cellosolve acetate and the like. Here, the above-described solvent is preferably selected in view of dissolvability for a lower layer in addition to the viscosity of the solution and the film formability.

For formation of the light emitting layer, coating methods such as a spin coat method, a dip coat method, an inkjet print method, a flexo printing method, a gravure printing method, a slit coat method and the like can be used.

The thickness of the light emitting layer may advantageously be selected in view of the driving voltage and the light emission efficiency, and it is usually 2 to 200 nm.

In the case of formation of the light emitting layer subsequent to a hole transporting layer, particularly when both the layers are formed by a coating method, a layer formed previously is dissolved in a solvent contained in a coating solution to be used in subsequent formation of a layer, leading to impossibility of fabrication of a laminated structure in some cases. In this case, a method for insolubilizing the hole transporting layer in a solvent can be used. The method for insolubilization in a solvent includes (1) a method in which a hole transporting layer is formed by using a crosslinkable hole transporting polymer compound as the above-described hole transporting polymer compound, and polymer chains are cross-linked in a process of device production, (2) a method in which a low molecular weight compound having an aromatic ring and having a cross-linkage group typified by an aromatic bisazide is mixed as a cross-linking agent with the hole transporting polymer compound and a hole transporting layer is formed, and polymer chains are cross-linked via the low molecular weight compound in a process of device production, (3) a method in which a low molecular weight compound having no aromatic ring and having a cross-linkage group typified by an acrylate group is mixed as a cross-linking agent with the hole transporting polymer compound and a hole transporting layer is formed, and polymer chains are cross-linked via the low molecular weight compound in a process of device production and (4) a method in which a hole transporting layer as a lower layer is formed, then, heated to be insolubilized in an organic solvent to be used for formation of a light emitting layer as an upper layer, and the above-described method (1) is preferable. The heating temperature in heating a hole transporting layer in performing cross-linkage is usually 150 to 300°C, and the heating time is usually 1 minute to 1 hour. As other methods than cross-linkage for laminating a hole transporting layer without dissolution, there is a method for using a solution of difference polarity as a solution for forming a contact layer, and examples thereof include a method in which a hole transporting layer as a lower layer is formed by using a polymer compound which is not dissolved in a polar solvent, to cause no dissolution of the hole transporting layer even if a coating solution containing a light emitting layer material and a polar solvent is coated in formation of a light emitting layer as an upper layer; and other methods.

The material used in the electron transporting layer includes polymer compounds containing an electron transporting group (oxadiazole group, oxathiadiazole group, pyridyl group, pyrimidyl group, pyridazyl group, triazyl group and the like) as a repeating unit and/or a substituent, and examples thereof include polyquinoline and derivatives thereof, polyquinoxaline and derivatives thereof, polyfluorene and derivatives thereof, and the like.

For formation of the electron transporting layer, methods of forming a film from a solution or melted condition are used. For film formation from a solution or melted condition, a polymer binder may be used together. The film formation method from a solution is the same as the above-described method for forming a hole transporting layer by film formation from a solution.

The thickness of the electron transporting layer may advantageously be regulated in view of the driving voltage and the light emission efficiency, and a thickness causing no formation of pin holes is necessary, and when the thickness is too large, the driving voltage of a device increases in some cases. Therefore, the thickness of the electron transporting layer is usually 1 nm to 1 μm, preferably 2 nm to 500 nm, further preferably 5 nm to 200 nm.

The electron injection layer includes, depending on the kind of a light emitting layer, an electron injection layer having a single layer structure composed of a Ca layer, or an electron injection layer having a lamination structure composed of a Ca layer and a layer formed of one or two or more materials selected from the group consisting of metals belonging to group IA and group IIA of the periodic table of elements and having a work function of 1.5 to 3.0 eV excluding Ca, and oxides, halides and carbonates of the metals. As the metals belonging to group IA of the periodic table of elements and having a work function of 1.5 to 3.0 eV and oxides, halides and carbonates thereof, listed are lithium, lithium fluoride, sodium oxide, lithium oxide, lithium carbonate and the like. As the metals belonging to group IIA of the periodic table of elements and having a work function of 1.5 to 3.0 eV excluding Ca, and oxides, halides and carbonates thereof, listed are strontium, magnesium oxide, magnesium fluoride, strontium fluoride, barium fluoride, strontium oxide, magnesium carbonate and the like.

For formation of the electron injection layer, a vapor-deposition method, a sputtering method, a printing method and the like are used. The thickness of the electron injection layer is preferably 1 nm to 1 $\mu$m.

As the material of a cathode, materials which have small work function and easily perform injection of electrons into a light emitting layer are preferable, and these materials include metals such as lithium, sodium, potassium, rubidium, cesium, beryllium, magnesium, calcium, strontium, barium, aluminum, scandium, vanadium, zinc, yttrium, indium, cerium, samarium, europium, terbium, ytterbium and the like; alloys composed of two or more of these metals; alloys composed of at least one of these metals and at least one of gold, silver, platinum, copper, manganese, titanium, cobalt, nickel, tungsten and tin; graphite, graphite intercalation compounds and the like.

The above-described alloy includes a magnesium-silver alloy, a magnesium-indium alloy, a magnesium-aluminum alloy, an indium-silver alloy, a lithium-aluminum alloy, a lithium-magnesium alloy, a lithium-indium alloy, a calcium-aluminum alloy and the like.

When the cathode has a lamination structure composed of two or more layers, it is preferable to combine a layer containing the above-described metal, metal oxide, metal fluoride or alloy thereof and a layer containing a metal such as aluminum, silver, chromium and the like.

The thickness of the cathode may advantageously be selected in view of the electric conductivity and the durability, and it is usually 10 nm to 10 $\mu$m, preferably 20 nm to 1 $\mu$m, more preferably 50 nm to 500 nm.

For fabrication of the cathode, a vacuum vapor-deposition method, a sputtering method, a laminate method for thermally compression-bonding a metal film, and the like are used. After cathode fabrication, it is preferable to install a protective layer and/or a protective cover for protecting an organic electroluminescent device.

As the protective layer, high molecular weight compounds, metal oxides, metal fluorides, metal borides and the like can be used. As the protective cover, a metal plate, a glass plate, and a plastic plate having a surface which has been subjected to a low water permeation treatment, and the like can be used. As the protective method, a method in which the protective cover is pasted to a device substrate with a thermosetting resin or a photocuring resin to attain encapsulation is used. When a space is kept using a spacer, blemishing of a device can be prevented easily. If an inert gas such as nitrogen, argon and the like is filled in this space, oxidation of a cathode can be prevented, further, by placing a drying agent such as barium oxide and the like in this space, it becomes easy to suppress moisture adsorbed in a production process or a small amount of water invaded through a hardened resin from imparting a damage to the device. It is preferable to adopt at least one strategy among these methods.

The organic electroluminescent device of the present invention can be used as a planar light source, a display (segment display, dot matrix display), back light of a liquid crystal display, or the like. For obtaining light emission in the form of plane using the above-described organic electroluminescent device, a planar anode and a planar cathode may advantageously be placed so as to overlap. For obtaining light emission in the form of pattern, there are a method in which a mask having a window in the form of pattern is placed on the surface of the above-described planar organic electroluminescent device, a method in which an organic layer in non-light emitting parts is formed with extremely large thickness to give substantially no light emission, a method in which either an anode or a cathode, or both electrodes are formed in the form of pattern. By forming a pattern by any of these methods and placing several electrodes so that ON/OFF thereof is independently possible, a display of segment type is obtained which can display digits, letters, simple marks and the like. Further, for providing a dot matrix device, it may be advantageous that both an anode and a cathode are formed in the form of stripe, and placed so as to cross. By adopting a method in which several polymer compounds showing different emission colors are painted separately or a method in which a color filter or a fluorescence conversion filter is used, partial color display and multi-color display are made possible. In the case of a dot matrix device, passive driving is possible, and active driving may also be carried out in combination with TFT and the like. These display devices can be used as a display of a computer, a television, a portable terminal, a cellular telephone, a car navigation, a view finder of a video camera, and the like. Further, the above-described planar organic electroluminescent device is of self emitting and thin type, and can be suitably used as a planar light source for back light of a liquid crystal display, or as a planar light source for illumination, and the like. If a flexible substrate is used, it can also be used as a curved light source or display.

EXAMPLES

Examples will be shown below for illustrating the present invention further in detail, but the present invention is not limited to these examples.

For the number-average molecular weight and the weight-average molecular weight, the polystyrene-equivalent number-average molecular weight and weight-average molecular weight were measured by size exclusion chromatography (SEC). SEC using an organic solvent as the mobile phase is called gel permeation chromatography (GPC). A measurement sample was dissolved in tetrahydrofuran at a concentration of about 0.05 wt%, and 30 μL of the solution was injected into GPC (manufactured by Shimadzu Corp., trade name: LC-10Avp). Tetrahydrofuran was used as the mobile phase of GPC, and flowed at a flow rate of 0.6 mL/min. As the column, two columns of TSKgel SuperHM-H (manufactured by Tosoh Corp.) and one column of TSKgel SuperH2000 (manufactured by Tosoh Corp.) were serially connected. As the detector, a differential refractive index detector (manufactured by Shimadzu Corp., trade name: RID-10A) was used.

Measurement of LC-MS was carried out according to the following method. A measurement sample was dissolved in chloroform or tetrahydrofuran at a concentration of about 2 mg/mL, and 1 μL of the solution was injected into LC-MS (manufactured by Agilent Technologies, trade name: 1100LCMSD). Ion exchanged water, acetonitrile, tetrahydrofuran and a mixed solution thereof were used as the mobile phase of LC-MS, and acetic acid was added if necessary. As the column, L-column 2 ODS (3 μm) (manufactured by Chemicals Evaluation and Research Institute, Japan, internal diameter: 2.1 mm, length: 100 mm, particle size: 3 μm) was used.

Measurement of TLC-MS was carried out according to the following method. A measurement sample was dissolved in chloroform, toluene or tetrahydrofuran, and the resultant solution was coated in small amount on the surface of a previously cut TLC glass plate (manufactured by Merck, trade name: Silica gel 60 $F_{254}$). This was measured by TLC-MS (manufactured by JEOL Ltd., trade name: JMS-T100TD) using a helium gas heated at 240 to 350°C.

A measurement sample (5 to 20 mg) was dissolved in about 0.5 mL of deuterated chloroform and subjected to measurement of NMR using an NMR instrument (manufactured by Varian, Inc., trade name: MERCURY 300).

In examples, the lowest excitation triplet energy of a compound was determined by a scientific calculation method.

In examples, the ionization potential of a compound was measured according to the following method. First, a compound was dissolved in toluene, and the resultant solution was coated on the surface of a quartz substrate by a spin coat method, to form a film. Using this film on a quartz substrate, the ionization potential of the compound was measured by Photoelectron Spectrometer in Air "AC-2" (trade name) manufactured by RIKEN KEIKI Co., Ltd.

<Synthesis Example 1> (Synthesis of compound M-1)

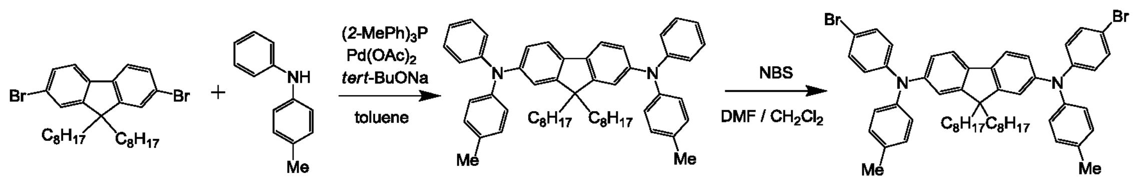


M−1

Into a nitrogen-purged reactor were charged 0.90 g of palladium(II) acetate, 2.435 g of tris(2-methylphenyl)phosphine and 125 mL of toluene, and the mixture was stirred at room temperature for 15 minutes. To this were added 27.4 g of 2,7-dibromo-9,9-dioctylfluorene, 22.91 g of (4-methylphenyl)phenylamine and 19.75 g of sodium-tert-butoxide, and the mixture was refluxed with heating overnight, then, cooled down to room temperature, 300 mL of water was added and washing thereof was performed. The organic layer was taken out and the solvent was distilled off under reduced pressure. The residue was dissolved in 100 mL of toluene, the resultant solution was passed through an alumina column. The eluate was concentrated under reduced pressure, to this was added methanol, to cause generation of a precipitate. The precipitate was filtrated, and recrystallized from p-xylene. This crystal was dissolved again in 100 mL of toluene, and the resultant solution was passed through an alumina column. The solution was concentrated to 50 to 100 mL, then, poured into 250 mL of methanol under stirring, to find generation of a precipitate. The precipitate was collected, and dried at room temperature under reduced pressure for 18 hours, to obtain white 2,7-bis[N-(4-methylphenyl)-N-phenyl]amino-9,9-dioctylfluorene (25.0 g).

Into a nitrogen-purged reactor were added 12.5 g of 2,7-bis[N-(4-methylphenyl)-N-phenyl]amino-9,9-dioctylfluorene and 95 mL of dichloromethane, and the reaction solution was cooled down to -10°C while stirring. A solution of

5.91 g of N-bromosuccinimide (NBS) dissolved in 20 mL of dimethylformamide (DMF) was slowly dropped into this. The mixture was stirred for 3.5 hours, then, mixed with 450 mL of cold methanol, the generated precipitate was filtrated, and recrystallized from p-xylene. The resultant crystal was recrystallized again using toluene and methanol, to obtain 12.1 g of a compound M-1 as a white solid.
$^1$H-NMR (300 MHz, $CDCl_3$): δ 0.61-0.71 (m, 4H), 0.86 (t, J = 6.8 Hz, 6H), 0.98-1.32 (m, 20H), 1.72-1.77 (m, 4H), 2.32 (br, 6H), 6.98-7.08 (m, 16H), 7.29 (d, J = 8.3 Hz, 4H), 7.44 (br, 2H)

<Synthesis Example 2> (Synthesis of compound M-2)

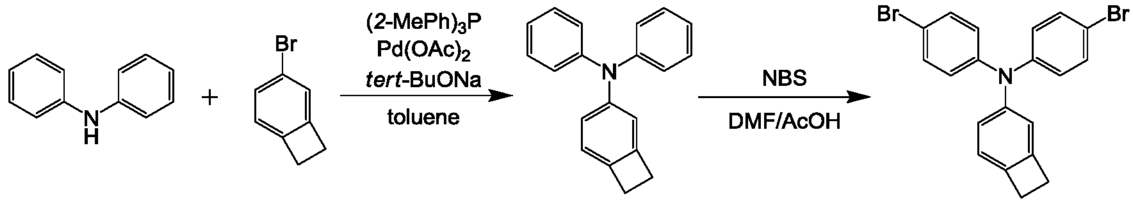


M-2

Into a nitrogen-purged 500mL three-necked round bottom flask were charged 196 mg of palladium(II) acetate, 731 mg of tris(2-methylphenyl)phosphine and 100 mL of toluene, and the mixture was stirred at room temperature. To the reaction solution were added 20.0 g of diphenylamine, 23.8 g of 3-bromobicyclo[4.2.0]octa-1,3,5-triene and 400 mL of toluene, subsequently, 22.8 g of sodium-tert-butoxide, and the mixture was refluxed with heating for 22 hours. To this was added 30 mL of 1M hydrochloric acid, to stop the reaction. The resultant reaction mixture was washed with 100 mL of a 2M sodium carbonate aqueous solution, the organic layer was passed through alumina, the eluate was collected, and the solvent was distilled off from this under reduced pressure. To the resultant oily yellow residue was added isopropyl alcohol, then, the mixture was stirred, and the generated precipitate was filtrated. This precipitate was recrystallized from isopropyl alcohol, to obtain 3-N,N-diphenylaminobicyclo[4.2.0]octa-1,3,5-triene.

Into a 250mL round bottom flask were charged 3-N,N-diphenylaminobicyclo[4.2.0]octa-1,3,5-triene (8.00 g) and 100 mL of dimethylformamide containing five drops of glacial acetic acid, and the mixture was stirred. To this was added N-bromosuccinimide (10.5 g), and the mixture was stirred for 5 hours. The resultant reaction mixture was poured into 600 mL of methanol/water (volume ratio 1/1), to stop the reaction, generating a precipitate. This precipitate was filtrated, and recrystallized from isopropyl alcohol, to obtain a compound M-2.
$^1$H NMR (300 MHz, $CDCl_3$): δ 3.11-3.15 (m, 4H), 6.80 (br, 1H), 6.87-6.92 (m, 5H), 6.96 (d, 1H), 7.27-7.33 (m, 4H)

<Synthesis Example 3> (Synthesis of compound M-3)

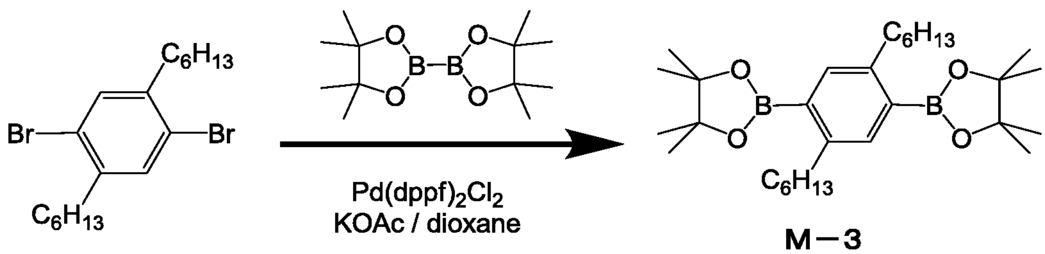


M-3

Into a 300 ml four-necked flask were charged 8.08 g of 1,4-dihexyl-2,5-dibromobenzene, 12.19 g of bis(pinacolate)diboron and 11.78 g of potassium acetate, and an atmosphere in the flask was purged with argon. Into this was charged 100 ml of dehydrated 1,4-dioxane, and the mixture was deaerated with argon. Into this was charged 0.98 g of [1,1'-bis(diphenylphosphino)ferrocene]dichloropalladium(II) ($Pd(dppf)_2Cl_2$), and the mixture was further deaerated with argon. The resultant mixed liquid was refluxed with heating for 6 hours. To the reaction solution was added toluene, and the mixture was washed with ion exchanged water. To the washed organic layer were added anhydrous sodium sulfate and activated carbon, and the mixture was filtrated through a funnel pre-coated with celite. The resultant filtrate was concentrated, to obtain 11.94 g of a dark brown crystal. This crystal was recrystallized from n-hexane, and the crystal was washed with methanol. The resultant crystal was dried under reduced pressure, to obtain 4.23 g of a white needle

crystal of a compound M-3. The yield was 42%.
$^1$H-NMR (300 MHz, $CDCl_3$): δ 0.88 (t, 6H), 1.23-1.40 (m, 36H), 1.47-1.56 (m, 4H), 2.81 (t, 4H), 7.52 (s, 2H) LC-MS (ESI, positive) m/z$^+$ = 573 $[M+K]^+$

<Synthesis Example 4> (Synthesis of compound M-4)

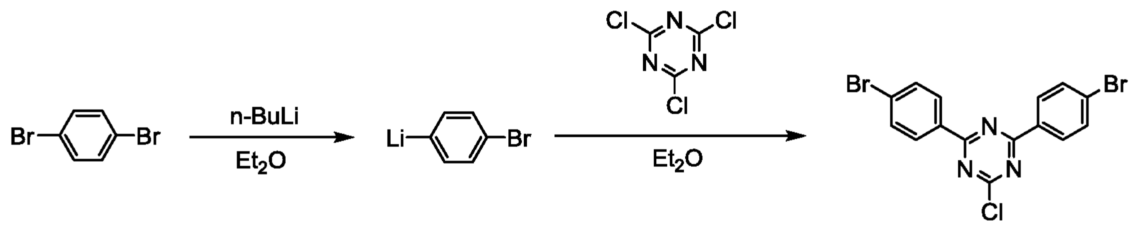


Under a nitrogen atmosphere, a solution of 27.1 g of 1,4-dibromobenzene in 217 ml of dehydrated diethyl ether was cooled by using a dry ice/methanol mixed bath. Into the resultant suspension, 37.2 ml of a 2.77 M solution of n-butyllithium in hexane was dropped slowly, then, the mixture was stirred for 1 hour, to prepare a lithium reagent.

Under a nitrogen atmosphere, a suspension of 10.0 g of cyanuric chloride in 68 ml of dehydrated diethyl ether was cooled by using a dry ice/methanol mixed bath, the above-described lithium reagent was added slowly, then, the mixture was warmed up to room temperature and reacted at room temperature. The resultant product was filtrated, and dried under reduced pressure. The resultant solid (16.5 g) was purified, to obtain 13.2 g of a needle crystal of 2,4-bis(4-bromophenyl)-6-chloro-1,3,5-triazine.

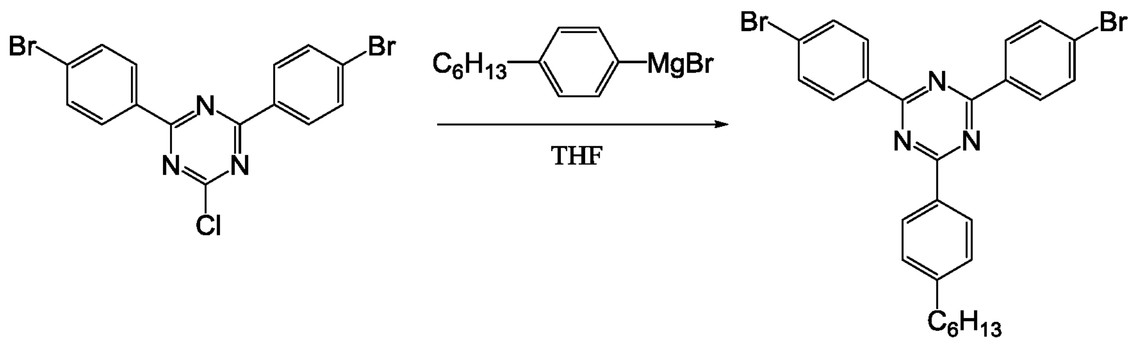


**M−4**

Under a nitrogen atmosphere, to a suspension obtained by adding 65 ml of dehydrated tetrahydrofuran to 1.37 g of magnesium was added portion-wise a solution of 14.2 g of 4-hexylbromobenzene in 15 ml of dehydrated tetrahydrofuran, and the mixture was heated, and stirred under reflux. To the resultant reaction liquid, after standing to cool, was added 0.39 g of magnesium additionally, and the mixture was heated again, and reacted under reflux, to prepare a Grignard reagent.

Under a nitrogen atmosphere, to a suspension of 12.0 g of the above-described needle crystal of 2,4-bis(4-bromophenyl)-6-chloro-1,3,5-triazine in 100 ml of dehydrated tetrahydrofuran was added the above-described Grignard reagent while stirring, and the mixture was refluxed with heating. The resultant reaction liquid was, after standing to cool, washed with a dilute hydrochloric acid aqueous solution. It was separated into an organic layer and an aqueous layer, and the aqueous layer was extracted with diethyl ether. The resultant organic layers were combined, washed with water again, the organic layer was dehydrated over anhydrous magnesium sulfate, and filtrated and concentrated. The resultant white solid was purified by a silica gel column, and further recrystallized, to obtain 6.5 g of a compound M-4 as a white solid.
$^1$H-NMR (300 MHz, $CDCl_3$): δ 0.90 (t, J = 6.2 Hz, 3H), 1.25-1.42 (m, 6H), 1.63-1.73 (m, 2H), 2.71 (t, J = 7.6 Hz, 2H), 7.34 (d, J = 7.9Hz, 2H), 7.65 (d, J = 7.9Hz, 4H), 8.53-8.58 (m, 6H)

<Synthesis Example 5> (Synthesis of phosphorescent compound A)

A phosphorescent compound A was synthesized according to a synthesis method described in International

Publication WO 2002/066552 pamphlet. Specifically, under a nitrogen atmosphere, 2-bromopyridine and 1.2 equivalent of 3-bromophenylboric acid were subjected to the Suzuki coupling (catalyst: tetrakis(triphenylphosphine)palladium(0), base: 2M sodium carbonate aqueous solution, solvent: ethanol, toluene), to obtain 2-(3'-bromophenyl)pyridine represented by the following formula:

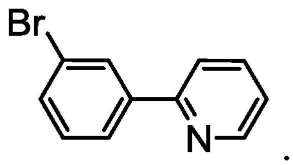

Next, under a nitrogen atmosphere, tribromobenzene and 2.2 equivalent of 4-tert-butylphenylboric acid were subjected to the Suzuki coupling (catalyst: tetrakis(triphenylphosphine)palladium(0), base: 2M sodium carbonate aqueous solution, solvent: ethanol, toluene), to obtain a bromo compound represented by the following formula:

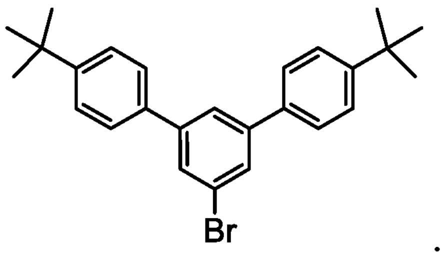

Under a nitrogen atmosphere, this bromo compound was dissolved in anhydrous THF, then, cooled down to -78°C, and a slight excess amount of tert-butyllithium was dropped. Under cooling, further, $B(OC_4H_9)_3$ was dropped, and reacted at room temperature. The resultant reaction liquid was post-treated with 3M hydrochloric acid water, to obtain a boric acid compound represented by the following formula:

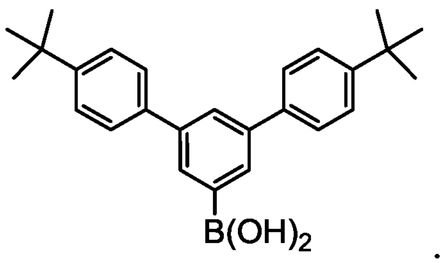

2-(3'-bromophenyl)pyridine and 1.2 equivalent of the above-described boric acid compound were subjected to the Suzuki coupling (catalyst: tetrakis(triphenylphosphine)palladium(0), base: 2M sodium carbonate aqueous solution, solvent: ethanol, toluene), to obtain a ligand (that is, a compound acting as a ligand) represented by the following formula:

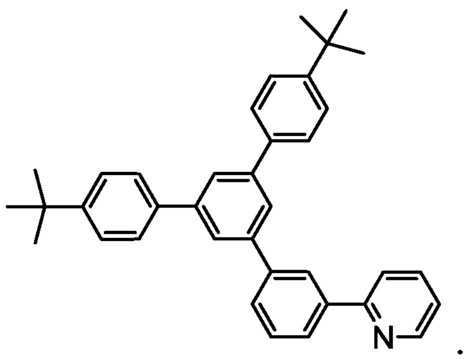

Under an argon atmosphere, $IrCl_3 \cdot 3H_2O$ and, 2.2 equivalent of the above-described ligand, 2-ethoxyethanol and ion exchanged water were charged, and refluxed. The deposited solid was filtrated under suction. The resultant solid was washed with ethanol and ion exchanged water in this order, then, dried, to obtain a compound represented by the following formula as a yellow powder:

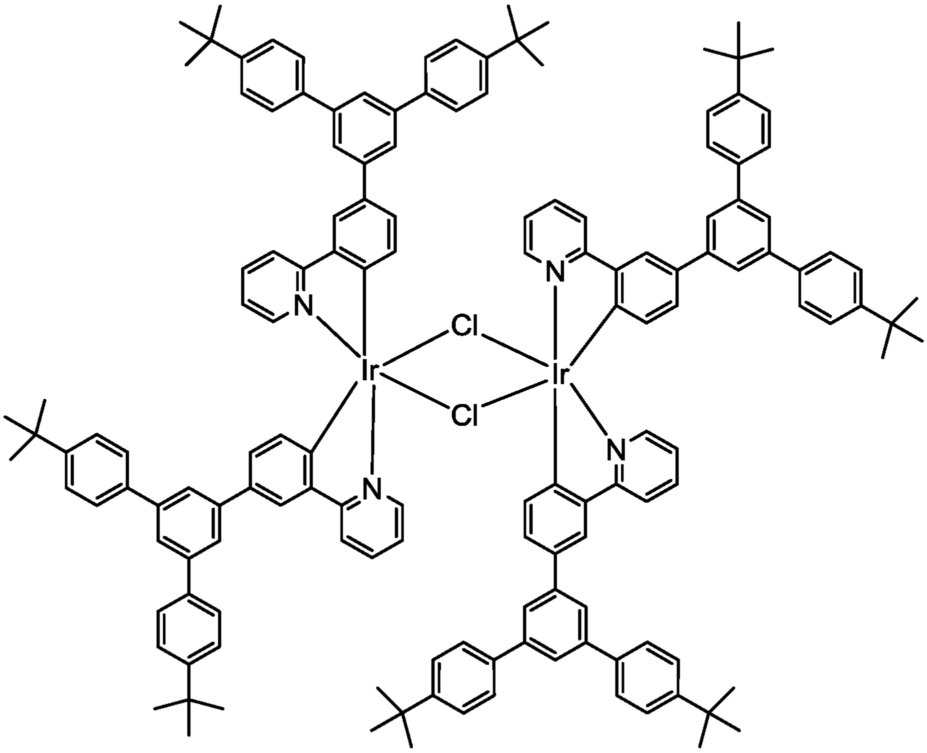

Under an argon atmosphere, to the above-described yellow powder were added 2 equivalent of the above-described ligand and 2 equivalent of silver trifluoromethanesulfonate, and the mixture was heated in diethylene glycol dimethyl ether, to obtain a phosphorescent compound A represented by the following formula:

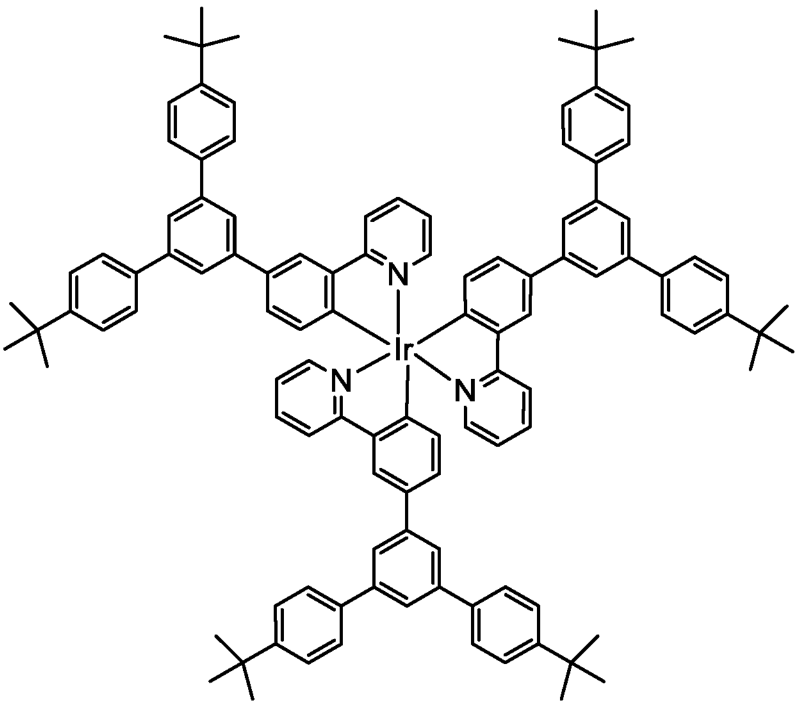

$^1$H-NMR (300 MHz, $CDCl_3$): δ 1.38 (s, 54H), 6.93 (dd, J = 6.3 Hz and 6.6 Hz, 3H), 7.04 (br, 3H), 7.30 (d, J = 7.9 Hz, 3H), 7.48 (d, J = 7.3 Hz, 12H), 7.61-7.70 (m, 21H), 7.82 (s, 6H), 8.01 (s, 3H), 8.03 (d, J = 7.9 Hz, 3H) LC-MS (APCI, positive): $m/z^+$ = 1677 $[M+H]^+$

The phosphorescent compound A had a lowest excitation triplet energy of 2.60 eV and an ionization potential of 5.24 eV.

<Synthesis Example 6> (Synthesis of polymer compound P-1)

Under an inert atmosphere, 5.20 g of 2,7-bis(1,3,2-dioxaborolan-2-yl)-9,9-dioctylfluorene, 5.42 g of bis(4-bromophenyl)-(4-sec-butylphenyl)-amine, 2.2 mg of palladium acetate, 15.1 mg of tris(2-methylphenyl)phosphine, 0.91 g of trioctylmethylammonium chloride (trade name: Aliquat336, manufactured by Aldrich) and 70 ml of toluene were mixed, and heated at 105°C. Into the reaction solution, 19 ml of a 2M sodium carbonate aqueous solution was dropped, and the mixture was refluxed for 4 hours. After the reaction, 121 mg of phenylboronic acid was added, and the mixture was further refluxed for 3 hours. Then, an aqueous solution of sodium N,N-diethyldithiocarbamate trihydrate was added, and the mixture was stirred at 80°C for 2 hours. After cooling, the reaction solution was washed with water, 3 wt% acetic acid aqueous solution and water in this order, and the resultant toluene solution was purified by passing through an alumina column and a silica gel column. The resultant toluene solution was dropped into a large amount of methanol, stirred, then, the resultant precipitate was filtrated and dried, to obtain a polymer compound P-1. The polymer compound P-1 had a polystyrene-equivalent number-average molecular weight Mn of $1.2 \times 10^5$ and a polystyrene-equivalent weight-average molecular weight Mw of $2.6 \times 10^5$.

The polymer compound P-1 is a copolymer composed of a repeating unit represented by the following formula:

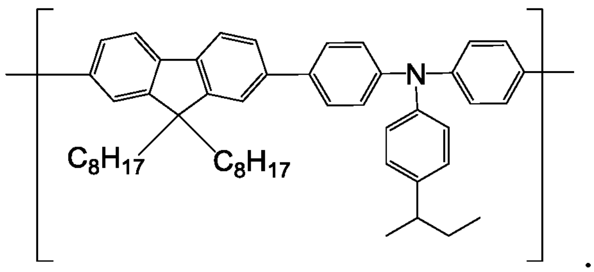

The polymer compound P-1 had a lowest excitation triplet energy of 2.76 eV and an ionization potential of 5.46 eV.

<Synthesis Example 7> (Synthesis of polymer compound P-2)

Into an inert gas-purged reaction vessel, 17.57 g (33.13 mmol) of 2,7-bis(1,3,2-dioxaborolan-2-yl)-9,9-dioctylfluorene, 12.88 g (28.05 mmol) of bis(4-bromophenyl)-(4-sec-butylphenyl)-amine, 2.15 g (5.01 mmol) of the compound M-2, 3 g of methyltrioctylammonium chloride (trade name: Aliquat 336, manufactured by Aldrich) and 200 g of toluene were measured and charged. The reaction vessel was heated at 100°C, and 7.4 mg of palladium(II) acetate, 70 mg of tris(2-methylphenyl)phosphine and 64 g of an about 18 wt% sodium carbonate aqueous solution were added, and stirring thereof was continued with heating for 3 hours or more. Thereafter, 400 mg of phenylboronic acid was added, and stirring thereof was further continued with heating for 5 hours. The reaction liquid was diluted with toluene, washed with a 3 wt% acetic acid aqueous solution and ion exchanged water in this order, then, the organic layer was taken out and to this was added 1.5 g of sodium diethyldithiocarbamate trihydrate, and the mixture was stirred for 4 hours. The resultant solution was purified by column chromatography using an equal mixture of alumina and silica gel as the stationary phase. The resultant toluene solution was dropped into methanol, stirred, then, the resultant precipitate was filtrated and dried, to obtain a polymer compound P-2. The polymer compound P-2 had a polystyrene-equivalent number-average molecular weight Mn of $8.9 \times 10^4$ and a polystyrene-equivalent weight-average molecular weight Mw of $4.2 \times 10^5$.

The polymer compound P-2 is a copolymer containing a repeating unit represented by the following formula (hereinafter, referred to as "MN1"):

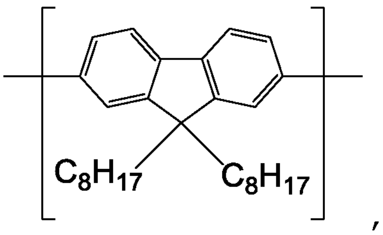

a repeating unit represented by the following formula (hereinafter, referred to as "MN2"):

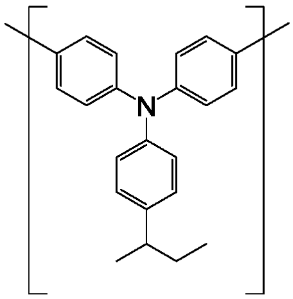

and a repeating unit represented by the following formula (hereinafter, referred to as "MN3"):

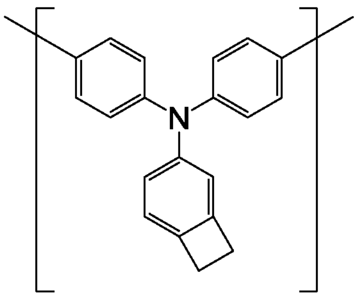

at a molar ratio of MN1:MN2:MN3 = 50:42:8, according to the theoretical value calculated from the charged raw materials.
The polymer compound P-2 had a lowest excitation triplet energy of 2.75 eV and an ionization potential of 5.45 eV.

<Synthesis Example 8> (Synthesis of polymer compound P-3)

A polymer compound P-3 was obtained in the same manner as in Synthesis Example 7, excepting that bis(4-bromophenyl)-(4-sec-butylphenyl)-amine was replaced by the compound M-1, and 2,7-bis(1,3,2-dioxaborolan-2-yl)-9,9-dioctylfluorene, the compound M-1 and the compound M-2 were used at a molar ratio of 50:42:8 in Synthesis Example 7.
The polymer compound P-3 had a polystyrene-equivalent number-average molecular weight of $6.0 \times 10^4$ and a polystyrene-equivalent weight-average molecular weight of $4.0 \times 10^5$.
The polymer compound P-3 is a copolymer containing the repeating unit (MN1), a repeating unit represented by the following formula (hereinafter, referred to as "MN4"):

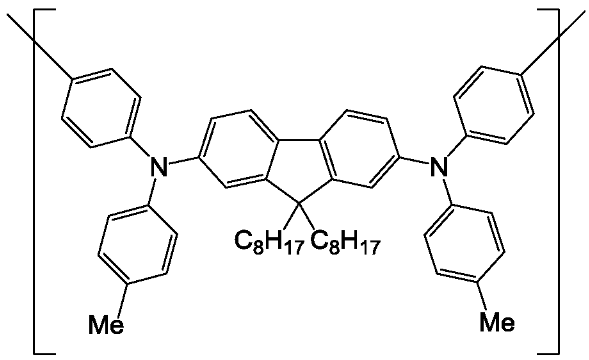

and the repeating unit (MN3) at a molar ratio of MN1:MN4:MN3 = 50:42:8, according to the theoretical value calculated from the charged raw materials.

The polymer compound P-3 had a lowest excitation triplet energy of 2.55 eV and an ionization potential of 5.29 eV.

<Synthesis Example 9> (Synthesis of polymer compound P-4)

Under an inert gas atmosphere, the compound M-3 (3.13 g), the compound M-4 (0.70 g), 2,7-dibromo-9,9-dioctylfluorene (2.86 g), palladium(II) acetate (2.1 mg), tris(2-methoxyphenyl)phosphine (13.4 mg) and toluene (80 mL) were mixed, and the mixture was heated at 100°C. A 20 wt% tetraethylammonium hydroxide aqueous solution (21.5 ml) was dropped into the reaction solution, and the mixture was refluxed for 5 hours. After the reaction, phenylboric acid (78 mg), palladium(II) acetate (2.1 mg), tris(2-methoxyphenyl)phosphine (13.3 mg), toluene (6 mL) and a 20 wt% tetraethylammonium hydroxide aqueous solution (21.5 ml) were added, and the mixture was further refluxed for 17.5 hours. Then, to this was added a 0.2 M sodium diethyldithiocarbamate aqueous solution (70 ml), and the mixture was stirred at 85°C for 2 hours. The solution was cooled down to room temperature, and washed with water, a 3 wt% acetic acid aqueous solution and water in this order. The organic layer was dropped into a large amount of methanol, the resultant precipitate was filtrated, then, dried, to obtain a solid. This solid was dissolved in toluene, and purified by passing through an alumina column and a silica gel column. The resultant toluene solution was dropped into methanol (1500 ml), and the resultant precipitate was filtrated and dried, to obtain 3.43 g of a polymer compound P-4.

The polymer compound P-4 had a polystyrene-equivalent number-average molecular weight Mn of $1.9\times10^5$ and a polystyrene-equivalent weight-average molecular weight Mw of $5.7\times10^5$.

The polymer compound P-4 is a copolymer containing a repeating unit represented by the following formula (hereinafter, referred to as "MN5"):

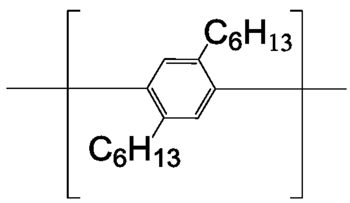

,

the repeating unit (MN1) and a repeating unit represented by the following formula (hereinafter, referred to as "MN6"):

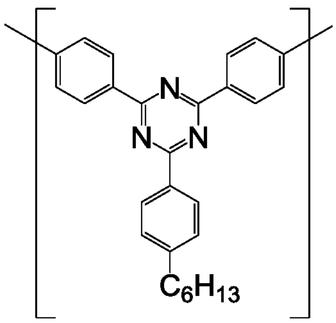

at a molar ratio of MN5:MN1:MN6 = 50:40:10, according to the theoretical value calculated from the charged raw materials.

The polymer compound P-4 had a lowest excitation triplet energy of 2.98 eV and an ionization potential of 6.10 eV.

<Example 1> (Fabrication of organic electroluminescent device 1)

On a glass substrate carrying thereon an ITO film having a thickness of 150 nm formed by a sputtering method, a suspension of poly(3,4)ethylenedioxythiophene/polystyrenesulfonic acid (Manufactured by H. C. Starck, trade name: CLEVIOS P AI4083) (hereinafter, referred to as "CLEVIOS P") was placed, and spin-coated to form a film having a thickness of about 65 nm, and dried on a hot plate at 200°C for 10 minutes. Next, the polymer compound P-3 was dissolved at a concentration of 0.7 wt% in xylene (manufactured by Kanto Chemical Co., Inc.: for Electronics (EL grade)), the resultant xylene solution was placed on the film of CLEVIOS P, and spin-coated to form a film having a thickness of about 20 nm, and under a nitrogen atmosphere having an oxygen concentration and a moisture concentration of each 10 ppm or less (based on weight), the film was dried at 180°C for 60 minutes to obtain a thermally-treated film. Next, the polymer compound P-4 and the phosphorescent compound A were dissolved at a concentration of 1.5 wt% (weight ratio: polymer compound P-4/phosphorescent compound A = 70/30) in xylene (manufactured by Kanto Chemical Co., Inc.: for Electronics (EL grade)). The resultant xylene solution was placed on the thermally-treated film of the polymer compound P-3, and spin-coated to form a light emitting layer 1 having a thickness of about 80 nm. Then, under a nitrogen atmosphere having an oxygen concentration and a moisture concentration of each 10 ppm or less (based on weight), the film was dried at 130°C for 10 minutes. After pressure reduction to $1.0 \times 10^{-4}$ Pa or lower, barium was vapor-deposited with a thickness of about 5 nm on the film of the light emitting layer 1, then, aluminum was vapor-deposited with a thickness of about 60 nm on the barium layer, as a cathode. After vapor deposition, encapsulation was performed using a glass substrate, to fabricate an organic electroluminescent device 1.

Voltage was applied on the organic electroluminescent device 1, to observe electroluminescence (EL) of green light emission. The light emission efficiency at a luminance of 1000 cd/$m^2$ was 25.9 cd/A, and the voltage under this condition was 5.8 V. The current density at a voltage of 6.0 V was 4.7 mA/$cm^2$. The luminance half life under an initial luminance of 4000 cd/$m^2$ was 52.8 hours.

<Comparative Example 1> (Fabrication of organic electroluminescent device C1)

On a glass substrate carrying thereon an ITO film having a thickness of 150 nm formed by a sputtering method, a suspension of CLEVIOS P was placed, and spin-coated to form a film having a thickness of about 65 nm, and dried on a hot plate at 200°C for 10 minutes. Next, the polymer compound P-1 was dissolved at a concentration of 0.7 wt% in xylene (manufactured by Kanto Chemical Co., Inc.: for Electronics (EL grade)), the resultant xylene solution was placed on the film of CLEVIOS P, and spin-coated to form a film having a thickness of about 20 nm, and under a nitrogen atmosphere having an oxygen concentration and a moisture concentration of each 10 ppm or less (based on weight), the film was dried at 180°C for 60 minutes to obtain a thermally-treated film. Next, the polymer compound P-4 and the phosphorescent compound A were dissolved at a concentration of 1.5 wt% (weight ratio: polymer compound P-4/phosphorescent compound A = 70/30) in xylene (manufactured by Kanto Chemical Co., Inc.: for Electronics (EL grade)). The resultant xylene solution was placed on the thermally-treated film of the polymer compound P-1, and spin-coated to form a light emitting layer C1 having a thickness of about 80 nm. Then, under a nitrogen atmosphere having an oxygen concentration and a moisture concentration of each 10 ppm or less (based on weight), the film was dried at 130°C for 10 minutes. After pressure reduction to $1.0 \times 10^{-4}$ Pa or lower, barium was vapor-deposited with a thickness of about 5 nm on the film of the light emitting layer C1, then, aluminum was vapor-deposited with a thickness of about 60 nm on the barium layer, as a cathode. After vapor deposition, encapsulation was performed using a glass substrate, to fabricate an organic electroluminescent device C1.

Voltage was applied on the organic electroluminescent device C1, to observe electroluminescence (EL) of green light emission. The light emission efficiency at a luminance of 1000 cd/$m^2$ was 27.5 cd/A, and the voltage under this condition was 6.6 V. The current density at a voltage of 6.0 V was 1.7 mA/$cm^2$. The luminance half life under an initial luminance of 4000 cd/$m^2$ was 27.8 hours.

<Comparative Example 2> (Fabrication of organic electroluminescent device C2)

On a glass substrate carrying thereon an ITO film having a thickness of 150 nm formed by a sputtering method, a suspension of CLEVIOS P was placed, and spin-coated to form a film having a thickness of about 65 nm, and dried on a hot plate at 200°C for 10 minutes. Next, the polymer compound P-2 was dissolved at a concentration of 0.7 wt% in xylene (manufactured by Kanto Chemical Co., Inc.: for Electronics (EL grade)), the resultant xylene solution was placed on the film of CLEVIOS P, and spin-coated to form a film having a thickness of about 20 nm, and under a nitrogen atmosphere having an oxygen concentration and a moisture concentration of each 10 ppm or less (based on weight),

the film was dried at 180°C for 60 minutes to obtain a thermally-treated film. Next, the polymer compound P-4 and the phosphorescent compound A were dissolved at a concentration of 1.5 wt% (weight ratio: polymer compound P-4/phosphorescent compound A = 70/30) in xylene (manufactured by Kanto Chemical Co., Inc.: for Electronics (EL grade)). The resultant xylene solution was placed on the thermally-treated film of the polymer compound P-2, and spin-coated to form a light emitting layer C2 having a thickness of about 80 nm. Then, under a nitrogen atmosphere having an oxygen concentration and a moisture concentration of each 10 ppm or less (based on weight), the film was dried at 130°C for 10 minutes. After pressure reduction to $1.0 \times 10^{-4}$ Pa or lower, barium was vapor-deposited with a thickness of about 5 nm on the film of the light emitting layer C2, then, aluminum was vapor-deposited with a thickness of about 60 nm on the barium layer, as a cathode. After vapor deposition, encapsulation was performed using a glass substrate, to fabricate an organic electroluminescent device C2.

Voltage was applied on the organic electroluminescent device C2, to observe electroluminescence (EL) of green light emission. The light emission efficiency at a luminance of 1000 cd/m$^2$ was 30.8 cd/A, and the voltage under this condition was 6.4 V. The current density at a voltage of 6.0 V was 2.2 mA/cm$^2$. The luminance half life under an initial luminance of 4000 cd/m$^2$ was 39.6 hours.

<Synthesis Example 10> (Synthesis of compound M-5)

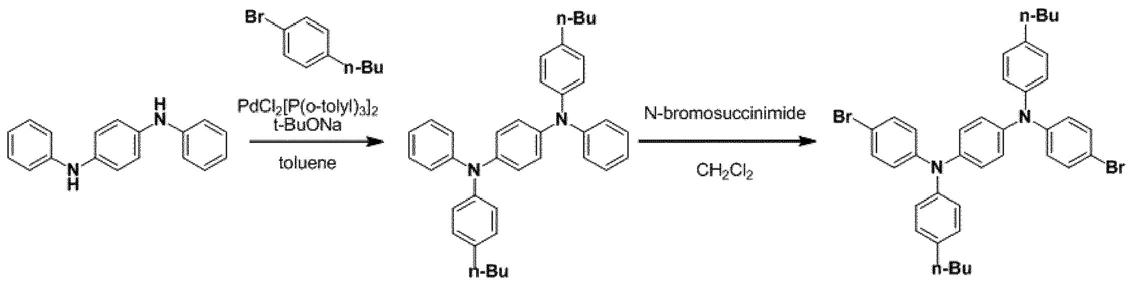


compound A1

M-5

Under a nitrogen gas atmosphere, N,N'-diphenyl-1,4-phenylenediamine (61.17 g), 4-n-butylbromobenzene (100.12 g), sodium-tert-butoxide (63.2 g) and toluene (3180 ml) were mixed, to this was added bis(tri-o-tolylphosphine)palladium(II) dichloride (7.39 g), then, the mixture was stirred for about 5 hours under reflux with heating. After cooling down to room temperature, the solid was removed by filtration through celite, washing was performed with saturated brine (about 1.2 L), then, the resultant organic layer was concentrated under reduced pressure, to obtain a brown viscous oil. This was recrystallized from acetone, filtrated, washed with an acetone/methanol mixed solvent, and dried under reduced pressure, to obtain a compound A1 (106.4 g) as a white crystal. The yield was 89%. The area percentage value in HPLC analysis was about 98%.

Under a nitrogen gas atmosphere, the compound A1 (100.0 g) synthesized by the same procedure as described above, N,N-dimethylformamide (500 ml) and hexane (1000 ml) were mixed, and heated at 40°C to obtain a uniform solution. This was cooled down to room temperature, then, a solution prepared by dissolving N-bromosuccinimide (72 g) in N,N-dimethylformamide (800 ml) was dropped over a period of 1 hour, and after completion of dropping, the mixture was stirred at room temperature for 1 hour, then, a 6 wt% sodium sulfite aqueous solution (200 ml) was added and the mixture was stirred thoroughly, liquid separation was carried out and the aqueous layer was removed. The resultant organic layer was concentrated under reduced pressure thereby distilling off hexane, to find deposition of a solid, this solid was filtrated, washed with a 6 wt% sodium sulfite aqueous solution (200 ml) and water (200 ml), and dried under reduced pressure, to obtain a white solid (88 g). The yield was 69%. The area percentage value in HPLC analysis was about 99.2%. An aliquot thereof (25.0 g) was dissolved in chloroform, activated carbon was added and the mixture was stirred and filtrated, then, recrystallized from toluene/hexane three times, to obtain the targeted compound M-5 (15.7 g) as a white solid. The area percentage value in HPLC analysis was about 99.9%. The yield after purification was 63%. The total yield was 43%.

<Synthesis Example 11> (Synthesis of compound M-6)

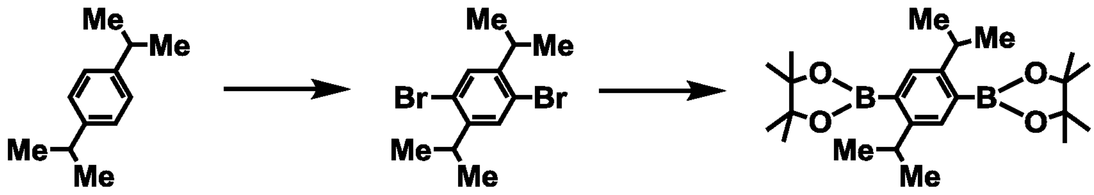

M-6

In a light-shielded 300ml round bottom flask under an argon gas atmosphere, 1,4-diisopropylbenzene (24.34 g, 150 mmol), an iron powder (0.838 g, 15 mmol), dehydrated chloroform (40 ml) and trifluoroacetic acid (1.71 g, 15 mmol) were mixed and stirred, and cooled by an ice bath, and a dilute solution of bromine (55.1 g, 345 mmol) in dehydrated chloroform (92 ml) was dropped into the cooled solution over a period of 30 minutes, and the mixture was further stirred and reacted for 5 hours while cooling by an ice bath. After completion of the reaction, a 10 wt% sodium hydroxide aqueous solution was cooled by an ice bath and to this was added slowly the above-described reaction solution, and the mixture was further stirred for 15 minutes. It was separated into an organic layer and an aqueous layer, extraction with chloroform (100 ml) from the aqueous solution was carried out, the resultant organic layers were combined, then, a 10 wt% sodium sulfite aqueous solution (200 ml) was added, and the mixture was stirred at room temperature for 30 minutes (in this operation, the color of the organic layer changed from pale yellow to approximately colorless transparent). The aqueous layer was separated and removed, the resultant organic layer was washed with 15 wt% brine (200 ml), then, dried over anhydrous magnesium sulfate (30 g), the solvent was distilled off by concentration under reduced pressure, to obtain about 47 g of a pale yellow oil. Ethanol (15 g) was added, the mixture was shaken to uniform, then, allowed to stand still for 3 hours in a -10°C freezer to cause deposition of a crystal which was then filtrated and washed with a small amount of methanol, and dried under reduced pressure overnight at room temperature, to obtain 1,4-dibromo-2,5-diisopropylbenzene (30.8 g, yield 64%) as a white crystal.
$^1$H-NMR (300 MHz, $CDCl_3$), $\delta$ = 1.24 (d, 12H), 3.30 (m, 2H), 7.50 (s, 2H)

In a 1000 ml flask under an argon gas atmosphere, to magnesium small pieces (9.724 g, 400 mmol) were added a small amount of dehydrated tetrahydrofuran and 1,2-dibromoethane (0.75 g, 4 mmol) sequentially. Activation of magnesium was confirmed by heat generation and foaming, then, a solution prepared by dissolving 1,4-dibromo-2,5-diisopropylbenzene (32.0 g, 100 mmol) synthesized by the same manner as described above in dehydrated tetrahydrofuran (100 ml) was dropped over a period of about 1 hour. After completion of dropping, the mixture was heated by a 80°C oil bath, and the mixture was stirred for 1 hour under reflux. The oil bath was removed, the solution was diluted with dehydrated tetrahydrofuran (200 ml), further cooled by an ice bath, then, 2-isopropoxy-4,4,5,5-tetramethyl-1,3,2-dioxaborolane (74.4 g, 400 mmol) was added. The ice bath was removed, the mixture was heated by a 80°C oil bath, and stirred under reflux for 1.5 hours. The oil bath was removed, further cooling by an ice bath was carried out, then, a saturated ammonium chloride aqueous solution (25 ml) was added, and the mixture was stirred for 30 minutes. The ice bath was removed, hexane (2000 ml) was added, and the mixture was stirred vigorously for 30 minutes. Stirring was stopped, the mixture was allowed to stand still for 15 minutes without any procedure, then, filtrated through a glass filter paved with silica gel, the silica gel was washed with hexane (1000 ml), the combined filtrates were concentrated under reduced pressure, to obtain a coarse product (59.0 g). Further, the same operation was carried out again at a scale of 80% of the above-described operation, to obtain a coarse product (44.8 g).

The same synthesis was further carried out, and the coarse products were combined. To the whole coarse product (103.8 g) was added methanol (520 ml), and the mixture was stirred under reflux with heating for 1 hour using a 75°C oil bath. The oil bath was removed, the mixture was cooled down to room temperature while stirring, then, the solid was filtrated, washed with methanol (100 ml), and dried under reduced pressure, to obtain a white crystal (48.8 g, HPLC area percentage (UV 254 nm): 93.3%). The dried crystal was dissolved with heating in isopropanol (690 ml), then, the solution was cooled slowly down to room temperature while allowing to stand still, to cause deposition of a crystal which was then filtrated and washed with methanol (50 ml), and dried under reduced pressure overnight at 50°C, to obtain the targeted compound M-6 as a white crystal (44.6 g, HPLC area percentage (UV 254 nm): 99.8%, yield 60%).
$^1$H-NMR (300 MHz, $CDCl_3$), $\delta$ = 1.23 (d, 12H), 1.34 (s, 24H), 3.58 (m, 2H), 7.61 (s, 2H)

<Synthesis Example 12> (Synthesis of compound M-7)

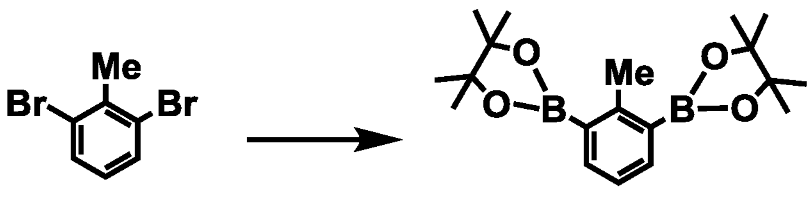

M-7

In a 1000 ml flask under an argon gas atmosphere, to magnesium small pieces (19.45 g, 800 mmol) were added a small amount of dehydrated tetrahydrofuran and 1,2-dibromoethane (1.50 g, 8 mmol) sequentially. Activation of magnesium was confirmed by heat generation and foaming, then, a solution prepared by dissolving 2,6-dibromotoluene (49.99 g, 200 mmol) in dehydrated tetrahydrofuran (200 ml) was dropped over a period of about 2 hours. After completion of dropping, heating by a 80°C oil bath was carried out, and the mixture was stirred for 1 hour under reflux. The oil bath was removed, the mixture was diluted with dehydrated tetrahydrofuran (400 ml), further cooled by an ice bath, then, 2-isopropyloxy-4,4,5,5-tetramethyl-1,3,2-dioxaborolane (148.85 g, 800 mmol) was added. The ice bath was removed, and the mixture was stirred for 1.5 hours under reflux by heating by a 80°C oil bath. The oil bath was removed, the mixture was further cooled by an ice bath, then, a saturated ammonium chloride aqueous solution (50 ml) was added, and the mixture was stirred for 30 minutes. The ice bath was removed, hexane (1500 ml) was added, and the mixture was stirred vigorously for 30 minutes. Stirring was stopped, the mixture was allowed to stand still for 15 minutes without any procedure, then, filtrated through a glass filter paved with silica gel, the silica gel was washed with hexane (1000 ml), and the combined filtrates were concentrated under reduced pressure, to obtain a coarse product (72.0 g). Further, the same operation was carried out again, to obtain a coarse product (75.4 g).

Next, methanol (740 ml) was added to the whole coarse product, and the mixture was stirred under reflux with heating for 1 hour using a 85°C oil bath. The oil bath was removed, the mixture was cooled down to room temperature while stirring, then, the solid was filtrated, washed with methanol (100 ml), and dried under reduced pressure to obtain a white crystal (59.7 g). The dried crystal was dissolved with heating in isopropanol (780 ml), then, the solution was cooled slowly down to room temperature while allowing to stand still, to cause deposition of a crystal which was filtrated and washed with methanol (100 ml), and dried under reduced pressure overnight at 50°C, to obtain the targeted compound M-7 (50.8 g, HPLC area percentage (ultraviolet wavelength 254 nm): 99.8%, yield 37%) as a white crystal.
$^1$H-NMR (300 MHz, $CDCl_3$) δ (ppm) = 1.34 (s, 24H), 2.74 (s, 3H), 7.14 (t, 1H), 7.79 (d, 2H)

<Synthesis Example 13> (Synthesis of electron transporting material ET-A)

According to the following reaction scheme, an electron transporting material ET-A was synthesized.

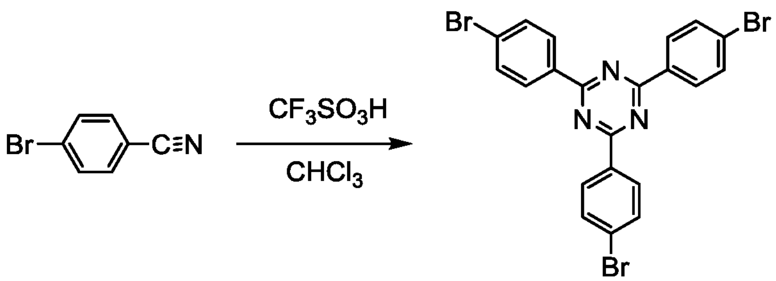

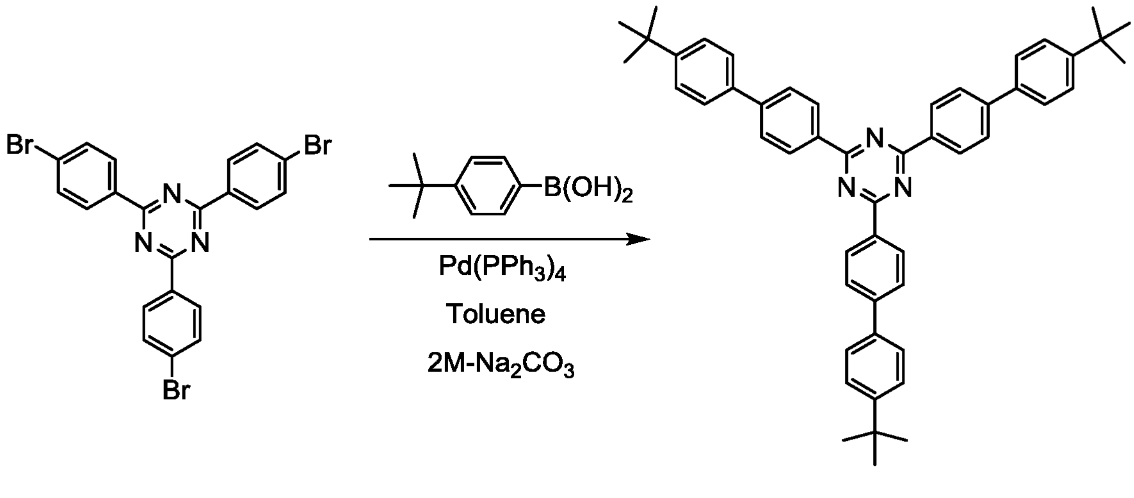


ET-A

Specifically, under a nitrogen atmosphere, 100 g (0.653 mol) of trifluoromethanesulfonic acid was charged in a flask, and stirred at room temperature. To this was added dropwise a solution prepared by dissolving 61.93 g (0.327 mol) of 4-bromobenzonitrile in 851 ml of dehydrated chloroform. The resultant solution was heated up to 95°C, stirred while heating, then, cooled down to room temperature, to this was added a dilute ammonia aqueous solution under an ice bath, to find generation of a solid. This solid was separated by filtration, washed with water, then, washed with diethyl ether, and dried while reducing pressure, to obtain 47.8 g of a white crystal.

Next, under a nitrogen atmosphere, 8.06 g (14.65 mol) of this white crystal, 9.15 g (49.84 mol) of 4-t-butylphenylboronic acid, 1.54 g (1.32 mol) of $Pd(PPh_3)_4$, 500 ml of toluene through which nitrogen had been bubbled previously and 47.3 ml of ethanol through which nitrogen had been bubbled previously were mixed, stirred, and heated to reflux. Into the reaction solution, 47.3 ml of a 2M sodium carbonate aqueous solution through which nitrogen had been bubbled previously was dropped, and further heated to reflux. The reaction solution was left to cool, then, separated, the aqueous layer was removed, and the organic layer was washed with dilute hydrochloric acid and water in this order, and separated. The organic layer was dried over anhydrous magnesium sulfate, filtrated and concentrated. The resultant coarse product was passed through a silica gel column, and to the resultant filtrate was added acetonitrile, to obtain a crystal. This crystal was dried while reducing pressure, to obtain 8.23 g of an electron transporting material ET-A as a white crystal. The result of the $^1$H-NMR analysis of the electron transporting material ET-A is shown below.

$^1$H-NMR (270 MHz/$CDCl_3$): $\delta$ 1.39 (s, 27H), 7.52 (d, 6H), 7.65 (d, 6H), 7.79 (d, 6H), 8.82 (d, 6H).

The electron transporting material ET-A had a lowest excitation triplet energy of 2.79 eV and an ionization potential of 6.13 eV.

<Synthesis Example 14> (Synthesis of polymer compound P-5)

To an inert gas-purged reaction vessel were added 2,7-bis(4,4,5,5-tetramethyl-1,3,2-dioxaborolan-2-yl)-9,9-dioctylfluorene (1.62 g, 2.50 mmol), the compound M-1 (2.30 g, 2.50 mmol), palladium(II) acetate (0.56 mg), tris(2-methoxyphenyl)phosphine (3.53 mg) and toluene (67 mL), and the mixture was heated at 100°C while stirring. Into the resultant solution, a 20 wt% tetraethylammonium hydroxide aqueous solution (8.5 mL) was dropped, and the mixture was refluxed for 7 hours. To this were added phenylboric acid (31 mg), palladium(II) acetate (0.56 mg), tris(2-methoxyphenyl)phosphine (3.53 mg) and a 20 wt% tetraethylammonium hydroxide aqueous solution (8.5 mL), and the mixture was further refluxed for 14 hours. Then, to this was added a solution prepared by dissolving sodium N,N-diethyldithiocarbamate trihydrate (1.39 g) in ion exchanged water (28 mL), and the mixture was stirred at 85°C for 4 hours. The organic layer was separated from the aqueous layer, then, the organic layer was washed with ion exchanged water (33 mL) three times, with a 3 wt% acetic acid aqueous solution (33 mL) three times and with ion exchanged water (33 mL) three times. The organic layer was dropped into methanol (520 mL), and the resultant precipitate was filtrated, then, dried to obtain a solid. This solid was dissolved in toluene, and purified by passing through a silica gel/alumina column through which toluene had been passed previously. The resultant eluate was dropped into methanol (600 mL), the resultant precipitate was filtrated, then, dried to obtain 2.48 g of a polymer compound P-5. The polymer compound P-5 had a polystyrene-equivalent number-average molecular weight Mn of $2.3\times10^4$ and a polystyrene-equivalent weight-average molecular weight Mw of $1.1\times10^5$.

The polymer compound P-5 is a copolymer composed of a repeating unit represented by the following formula:

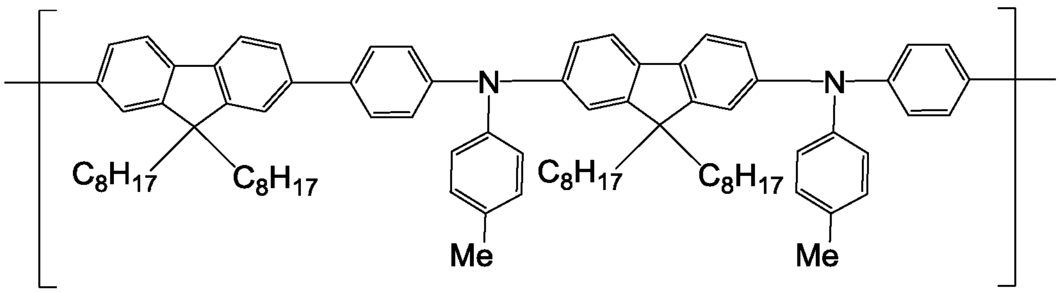

.

The polymer compound P-5 had lowest excitation triplet energy of 2.55 eV and an ionization potential of 5.28 eV.

<Synthesis Example 15> (Synthesis of polymer compound P-6)

To an inert gas-purged reaction vessel were added 2,7-bis(4,4,5,5-tetramethyl-1,3,2-dioxaborolan-2-yl)-9,9-dioctylfluorene (2.65 g, 4.12 mmol), the compound M-5 (2.40 g, 3.52 mmol), the compound M-2 (0.267 g, 0.622 mmol), dichlorobis(triphenylphosphine)palladium(II) (2.9 mg) and toluene (81 mL), and the mixture was heated at 100°C while stirring. Into the resultant solution, a 20 wt% tetraethylammonium hydroxide aqueous solution (14 mL) was dropped, and the mixture was refluxed for 4 hours. To this were added phenylboric acid (51 mg), dichlorobis(triphenylphosphine)palladium(II) (2.9 mg) and a 20 wt% tetraethylammonium hydroxide aqueous solution (14 mL), and the mixture was further refluxed for 19 hours. Then, to this was added a solution prepared by dissolving sodium N,N-diethyldithiocarbamate trihydrate (2.29 g) in ion exchanged water (46 mL), and the mixture was stirred at 85°C for 6 hours. The organic layer was separated from the aqueous layer, then, the organic layer was washed with ion exchanged water (72 mL) twice, a 3 wt% acetic acid aqueous solution (72 mL) twice and ion exchanged water (72 mL) twice. The organic layer was dropped into methanol (660 mL), and the resultant precipitate was filtrated, then, dried to obtain a solid. This solid was dissolved in toluene, and purified by passing through a silica gel/alumina column through which toluene had been passed previously. The resultant eluate was dropped into methanol (1400 mL), the resultant precipitate was filtrated, then, dried to obtain 2.82 g of a polymer compound P-6. The polymer compound P-6 had a polystyrene-equivalent number-average molecular weight Mn of $3.0\times10^4$ and a polystyrene-equivalent weight-average molecular weight Mw of $2.0\times10^5$.

The polymer compound P-6 is a copolymer containing the repeating unit (MN1), a repeating unit represented by the following formula (hereinafter, referred to as "MN7"):

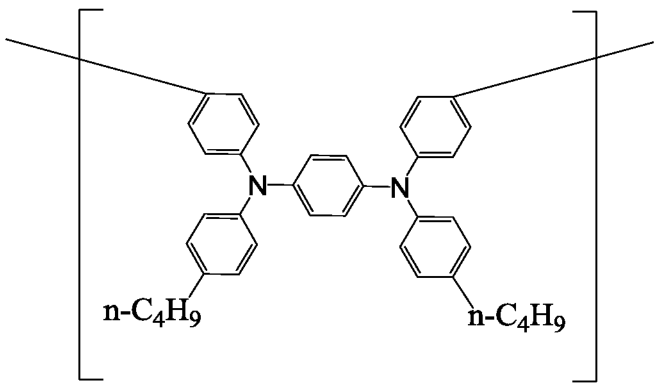

and the repeating unit (MN3) at a molar ratio of MN1:MN7:MN3 = 50:43:8, according to the theoretical value calculated from the charged raw materials.

The polymer compound P-6 had a lowest excitation triplet energy of 2.70 eV and an ionization potential of 5.29 eV.

<Synthesis Example 16> (Synthesis of polymer compound P-7)

Into an inert gas-purged reaction vessel were added the compound M-6 (1.29 g, 3.11 mmol), the compound M-7 (0.261 g, 0.759 mmol), 2,7-dibromo-9,9-dioctylfluorene (2.01 g, 3.66 mmol), bis(4-bromophenyl)(4-sec-butylphenyl)amine (0.104 g, 0.226 mmol), palladium(II) acetate (0.85 mg), tris(2-methoxyphenyl)phosphine (5.3 mg) and toluene (38 ml), and the mixture was heated at 100°C while stirring. Into the resultant solution, a 20 wt% tetraethylammonium

hydroxide aqueous solution (13 ml) was dropped, and the mixture was refluxed for about 21 hours. To this were added phenylboric acid (47 mg), palladium(II) acetate (0.85 mg), tris(2-methoxyphenyl)phosphine (5.4 mg) and toluene (6 mL), and the mixture was further refluxed for 15 hours. Then, to this was added a solution prepared by dissolving sodium N,N-diethyldithiocarbamate trihydrate (2.10 g) in ion exchanged water (46 mL), and the mixture was stirred at 85°C for 2 hours. The organic layer was separated from the aqueous layer, then, the organic layer was washed with ion exchanged water (50 mL) three times, with a 3 wt% acetic acid aqueous solution (50 mL) three times and with ion exchanged water (50 mL) three times. The organic layer was dropped into methanol (600 mL), and the resultant precipitate was filtrated, then, dried to obtain a solid. This solid was dissolved in toluene, and purified by passing through a silica gel/alumina column through which toluene had been passed previously. The resultant eluate was dropped into methanol (700 mL), the resultant precipitate was filtrated, then, dried to obtain 1.73 g of a polymer compound P-7. The polymer compound P-7 had a polystyrene-equivalent number-average molecular weight Mn of $5.1\times10^4$ and a polystyrene-equivalent weight-average molecular weight Mw of $1.2\times10^5$.

The polymer compound P-7 is copolymer containing a repeating unit represented by the following formula (hereinafter, referred to as "MN8"):

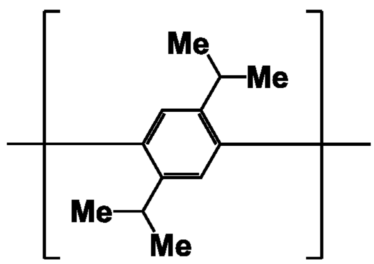

a repeating unit represented by the following formula (hereinafter, referred to as "MN9"):

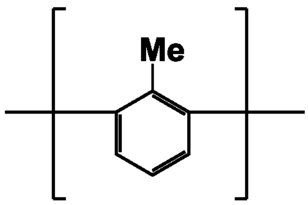

,

the repeating unit (MN1) and the repeating unit (MN2) at a molar ratio of MN8:MN9:MN1:MN2 = 40:10:47:3, according to the theoretical value calculated from the charged raw materials.

The polymer compound P-7 had a lowest excitation triplet energy of 3.08 V and an ionization potential of 5.83 eV.

<Example 2> (Fabrication of organic electroluminescent device 2)

On a glass substrate carrying thereon an ITO film having a thickness of 150 nm formed by a sputtering method, a suspension of poly(3,4)ethylenedioxythiophene/polystyrenesulfonic acid (CLEVIOS P) was placed, and spin-coated to form a film having a thickness of about 65 nm, and dried on a hot plate at 200°C for 10 minutes. Next, the polymer compound P-5 was dissolved at a concentration of 0.7 wt% in xylene (manufactured by Kanto Chemical Co., Inc.: for Electronics (EL grade)), the resultant xylene solution was placed on the film of CLEVIOS P, and spin-coated to form a film having a thickness of about 20 nm, and under a nitrogen atmosphere having an oxygen concentration and a moisture concentration of each 10 ppm or less (based on weight), the film was dried at 180°C for 60 minutes to obtain a thermally-treated film. Next, the polymer compound P-4 and the phosphorescent compound A were dissolved at a concentration of 1.5 wt% (weight ratio: polymer compound P-4/phosphorescent compound A = 70/30) in xylene (manufactured by Kanto Chemical Co., Inc.: for Electronics (EL grade)). The resultant xylene solution was placed on the thermally-treated film of the polymer compound P-5, and spin-coated to form a light emitting layer 2 having a thickness of about 80 nm. Then, under a nitrogen atmosphere having an oxygen concentration and a moisture concentration of each 10 ppm or less (based on weight), the film was dried at 130°C for 10 minutes. After pressure reduction to $1.0\times10^{-4}$ Pa or lower, barium was vapor-deposited with a thickness of about 5 nm on the film of the light emitting layer 2, then, aluminum was vapor-deposited with a thickness of about 60 nm on the barium layer, as a cathode. After vapor deposition, encapsulation was performed using a glass substrate, to fabricate an organic electroluminescent device 2.

Voltage was applied on the organic electroluminescent device 2, to observe electroluminescence (EL) of green

light emission. The light emission efficiency at a luminance of 1000 cd/$m^2$ was 24.3 cd/A, and the voltage under this condition was 5.9 V. The current density at a voltage of 6.0 V was 4.3 mA/$cm^2$. The luminance half life under an initial luminance of 4000 cd/$m^2$ was 54.0 hours.

<Example 3> (Fabrication of organic electroluminescent device 3)

On a glass substrate carrying thereon an ITO film having a thickness of 150 nm formed by a sputtering method, a suspension of poly(3,4)ethylenedioxythiophene/polystyrenesulfonic acid (CLEVIOS P) was placed, and spin-coated to form a film having a thickness of about 65 nm, and dried on a hot plate at 200°C for 10 minutes. Next, the polymer compound P-5 was dissolved at a concentration of 0.7 wt% in xylene (manufactured by Kanto Chemical Co., Inc.: for Electronics (EL grade)), the resultant xylene solution was placed on the film of CLEVIOS P, and spin-coated to form a film having a thickness of about 20 nm, and under a nitrogen atmosphere having an oxygen concentration and a moisture concentration of each 10 ppm or less (based on weight), the film was dried at 180°C for 60 minutes to obtain a thermally-treated film. Next, the polymer compound P-7, the electron transporting material ET-A and the phosphorescent compound A were dissolved at a concentration of 2.1 wt% (weight ratio: polymer compound P-7/electron transporting material ET-A/phosphorescent compound A = 42/28/30) in xylene (manufactured by Kanto Chemical Co., Inc.: for Electronics (EL grade)). The resultant xylene solution was placed on the thermally-treated film of the polymer compound P-5, and spin-coated to form a light emitting layer 3 having a thickness of about 80 nm. Then, under a nitrogen atmosphere having an oxygen concentration and a moisture concentration of each 10 ppm or less (based on weight), the film was dried at 130°C for 10 minutes. After pressure reduction to $1.0 \times 10^{-4}$ Pa or lower, barium was vapor-deposited with a thickness of about 5 nm on the film of the light emitting layer 3, then, aluminum was vapor-deposited with a thickness of about 60 nm on the barium layer, as a cathode. After vapor deposition, encapsulation was performed using a glass substrate, to fabricate an organic electroluminescent device 3.

Voltage was applied on the organic electroluminescent device 3, to observe electroluminescence (EL) of green light emission. The light emission efficiency at a luminance of 1000 cd/$m^2$ was 27.3 cd/A, and the voltage under this condition was 5.9 V. The current density at a voltage of 6.0 V was 4.0 mA/$cm^2$. The luminance half life under an initial luminance of 4000 cd/$m^2$ was 150.0 hours.

<Example 4> (Fabrication of organic electroluminescent device 4)

On a glass substrate carrying thereon an ITO film having a thickness of 150 nm formed by a sputtering method, a suspension of poly(3,4)ethylenedioxythiophene/polystyrenesulfonic acid (CLEVIOS P) was placed, and spin-coated to form a film having a thickness of about 65 nm, and dried on a hot plate at 200°C for 10 minutes. Next, the polymer compound P-3 was dissolved at a concentration of 0.7 wt% in xylene (manufactured by Kanto Chemical Co., Inc.: for Electronics (EL grade)), the resultant xylene solution was placed on the film of CLEVIOS P, and spin-coated to form a film having a thickness of about 20 nm, and under a nitrogen atmosphere having an oxygen concentration and a moisture concentration of each 10 ppm or less (based on weight), the film was dried at 180°C for 60 minutes to obtain a thermally-treated film. Next, the polymer compound P-7, the electron transporting material ET-A and the phosphorescent compound A were dissolved at a concentration of 2.1 wt% (weight ratio: polymer compound P-7/electron transporting material ET-A/phosphorescent compound A = 42/28/30) in xylene (manufactured by Kanto Chemical Co., Inc.: for Electronics (EL grade)). The resultant xylene solution was placed on the thermally-treated film of the polymer compound P-3, and spin-coated to form a light emitting layer 4 having a thickness of about 80 nm. Then, under a nitrogen atmosphere having an oxygen concentration and a moisture concentration of each 10 ppm or less (based on weight), the film was dried at 130°C for 10 minutes. After pressure reduction to $1.0 \times 10^{-4}$ Pa or lower, barium was vapor-deposited with a thickness of about 5 nm on the film of the light emitting layer 4, then, aluminum was vapor-deposited with a thickness of about 60 nm on the barium layer, as a cathode. After vapor deposition, encapsulation was performed using a glass substrate, to fabricate an organic electroluminescent device 4.

Voltage was applied on the organic electroluminescent device 4, to observe electroluminescence (EL) of green light emission. The light emission efficiency at a luminance of 1000 cd/$m^2$ was 32.5 cd/A, and the voltage under this condition was 5.7 V. The current density at a voltage of 6.0 V was 4.0 mA/$cm^2$. The luminance half life under an initial luminance of 4000 cd/$m^2$ was 177.0 hours.

<Example 5> (Fabrication of organic electroluminescent device 5, not forming part of the invention)

On a glass substrate carrying thereon an ITO film having a thickness of 150 nm formed by a sputtering method, a suspension of poly(3,4)ethylenedioxythiophene/polystyrenesulfonic acid (CLEVIOS P) was placed, and spin-coated to form a film having a thickness of about 65 nm, and dried on a hot plate at 200°C for 10 minutes. Next, the polymer compound P-6 was dissolved at a concentration of 0.7 wt% in xylene (manufactured by Kanto Chemical Co., Inc.: for

Electronics (EL grade)), the resultant xylene solution was placed on the film of CLEVIOS P, and spin-coated to form a film having a thickness of about 20 nm, and under a nitrogen atmosphere having an oxygen concentration and a moisture concentration of each 10 ppm or less (based on weight), the film was dried at 180°C for 60 minutes to obtain a thermally-treated film. Next, the polymer compound P-7, the electron transporting material ET-A and the phosphorescent compound A were dissolved at a concentration of 2.1 wt% (weight ratio: polymer compound P-7/electron transporting material ET-A/phosphorescent compound A = 42/28/30) in xylene (manufactured by Kanto Chemical Co., Inc.: for Electronics (EL grade)). The resultant xylene solution was placed on the thermally-treated film of the polymer compound P-6, and spin-coated to form a light emitting layer 5 having a thickness of about 80 nm. Then, under a nitrogen atmosphere having an oxygen concentration and a moisture concentration of each 10 ppm or less (based on weight), the film was dried at 130°C for 10 minutes. After pressure reduction to $1.0 \times 10^{-4}$ Pa or lower, barium was vapor-deposited with a thickness of about 5 nm on the film of the light emitting layer 5, then, aluminum was vapor-deposited with a thickness of about 60 nm on the barium layer, as a cathode. After vapor deposition, encapsulation was performed using a glass substrate, to fabricate an organic electroluminescent device 5.

Voltage was applied on the organic electroluminescent device 5, to observe electroluminescence (EL) of green light emission. The light emission efficiency at a luminance of 1000 cd/$m^2$ was 22.0 cd/A, and the voltage under this condition was 6.2 V. The current density at a voltage of 6.0 V was 3.9 mA/$cm^2$. The luminance half life under an initial luminance of 4000 cd/$m^2$ was 147.0 hours.

<Comparative Example 3> (Fabrication of organic electroluminescent device C3)

On a glass substrate carrying thereon an ITO film having a thickness of 150 nm formed by a sputtering method, a suspension of CLEVIOS P was placed, and spin-coated to form a film having a thickness of about 65 nm, and dried on a hot plate at 200°C for 10 minutes. Next, the polymer compound P-1 was dissolved at a concentration of 0.7 wt% in xylene (manufactured by Kanto Chemical Co., Inc.: for Electronics (EL grade)), the resultant xylene solution was placed on the film of CLEVIOS P, and spin-coated to form a film having a thickness of about 20 nm, and under a nitrogen atmosphere having an oxygen concentration and a moisture concentration of each 10 ppm or less (based on weight), the film was dried at 180°C for 60 minutes to obtain a thermally-treated film. Next, the polymer compound P-7, the electron transporting material ET-A and the phosphorescent compound A were dissolved at a concentration of 2.1 wt% (weight ratio: polymer compound P-7/electron transporting material ET-A/phosphorescent compound A = 42/28/30) in xylene (manufactured by Kanto Chemical Co., Inc.: for Electronics (EL grade)). The resultant xylene solution was placed on the thermally-treated film of the polymer compound P-1, and spin-coated to form a light emitting layer C3 having a thickness of about 80 nm. Then, under a nitrogen atmosphere having an oxygen concentration and a moisture concentration of each 10 ppm or less (based on weight), the film was dried at 130°C for 10 minutes. After pressure reduction to $1.0 \times 10^{-4}$ Pa or lower, barium was vapor-deposited with a thickness of about 5 nm on the film of the light emitting layer C3, then, aluminum was vapor-deposited with a thickness of about 60 nm on the barium layer, as a cathode. After vapor deposition, encapsulation was performed using a glass substrate, to fabricate an organic electroluminescent device C3.

Voltage was applied on the organic electroluminescent device C3, to observe electroluminescence (EL) of green light emission. The light emission efficiency at a luminance of 1000 cd/$m^2$ was 34.5 cd/A, and the voltage under this condition was 5.9 V. The current density at a voltage of 6.0 V was 3.3 mA/$cm^2$. The luminance half life under an initial luminance of 4000 cd/$m^2$ was 48.7 hours.

<Comparative Example 4> (Fabrication of organic electroluminescent device C4)

On a glass substrate carrying thereon an ITO film having a thickness of 150 nm formed by a sputtering method, a suspension of CLEVIOS P was placed, and spin-coated to form a film having a thickness of about 65 nm, and dried on a hot plate at 200°C for 10 minutes. Next, the polymer compound P-2 was dissolved at a concentration of 0.7 wt% in xylene (manufactured by Kanto Chemical Co., Inc.: for Electronics (EL grade)), the resultant xylene solution was placed on the film of CLEVIOS P, and spin-coated to form a film having a thickness of about 20 nm, and under a nitrogen atmosphere having an oxygen concentration and a moisture concentration of each 10 ppm or less (based on weight), the film was dried at 180°C for 60 minutes to obtain a thermally-treated film. Next, the polymer compound P-7, the electron transporting material ET-A and the phosphorescent compound A were dissolved at a concentration of 2.1 wt% (weight ratio: polymer compound P-7/electron transporting material ET-A/phosphorescent compound A = 42/28/30) in xylene (manufactured by Kanto Chemical Co., Inc.: for Electronics (EL grade)). The resultant xylene solution was placed on the thermally-treated film of the polymer compound P-2, and spin-coated to form a light emitting layer C4 having a thickness of about 80 nm. Then, under a nitrogen atmosphere having an oxygen concentration and a moisture concentration of each 10 ppm or less (based on weight), the film was dried at 130°C for 10 minutes. After pressure reduction to $1.0 \times 10^{-4}$ Pa or lower, barium was vapor-deposited with a thickness of about 5 nm on the film of the light emitting

layer C4, then, aluminum was vapor-deposited with a thickness of about 60 nm on the barium layer, as a cathode. After vapor deposition, encapsulation was performed using a glass substrate, to fabricate an organic electroluminescent device C4.

Voltage was applied on the organic electroluminescent device C4, to observe electroluminescence (EL) of green light emission. The light emission efficiency at a luminance of 1000 cd/$m^2$ was 30.9 cd/A, and the voltage under this condition was 6.1 V. The current density at a voltage of 6.0 V was 2.9 mA/$cm^2$. The luminance half life under an initial luminance of 4000 cd/$m^2$ was 108.0 hours.

Table 3

| | hole transporting layer | light emitting layer | | $T1_t$-$T1_e$ (eV) | 4000 cd/$m^2$ luminance half life (hr) |
|---|---|---|---|---|---|
| | hole transporting compound | first light emitting layer material | second light emitting layer material | | |
| Example 1 | P-3 | phosphorescent compound A | P-4 | - 0.05 | 52.8 |
| Example 2 | P-5 | phosphorescent compound A | P-4 | - 0.05 | 54.0 |
| Example 3 | P-5 | phosphorescent compound A | P-7 | - 0.05 | 150.0 |
| Example 4 | P-3 | phosphorescent compound A | P-7 | - 0.05 | 177.0 |
| Example 5 | P-6 | phosphorescent compound A | P-7 | 0.10 | 147.0 |
| Comparative Example 1 | P-1 | phosphorescent compound A | P-4 | 0.16 | 27.8 |
| Comparative Example 2 | P-2 | phosphorescent compound A | P-4 | 0.15 | 39.6 |
| Comparative Example 3 | P-1 | phosphorescent compound A | P-7 | 0.16 | 48.7 |
| Comparative Example 4 | P-2 | phosphorescent compound A | P-7 | 0.15 | 108.0 |

Table 4

| | hole transporting layer | light emitting layer | | $IP_{eh}$-$IP_t$ (eV) | 1000 cd/$m^2$ light emission efficiency (cd/A) | 1000 cd/$m^2$ voltage (V) | 6.0 V current density (mA/$cm^2$) |
|---|---|---|---|---|---|---|---|
| | Hole transporting polymer compound | first light emitting layer material | second light emitting layer material | | | | |
| Example 1 | P-3 | phosphorescent compound A | P-4 | -0.05 | 25.9 | 5.8 | 4.7 |
| Example 2 | P-5 | phosphorescent compound A | P-4 | -0.05 | 24.3 | 5.9 | 4.3 |
| Example 3 | P-5 | phosphorescent compound A | P-7 | -0.04 | 27.3 | 5.9 | 4.0 |

(continued)

| | hole transporting layer | light emitting layer | | | | | |
|---|---|---|---|---|---|---|---|
| | Hole transporting polymer compound | first light emitting layer material | second light emitting layer material | $IP_{eh}$-$IP_t$ (eV) | 1000 cd/$m^2$ light emission efficiency (cd/A) | 1000 cd/$m^2$ voltage (V) | 6.0 V current density (mA/$cm^2$) |
| Example 4 | P-3 | phosphorescent compound A | P-7 | -0.05 | 32.5 | 5.7 | 4.0 |
| Example 5 | P-6 | phosphorescent compound A | P-7 | -0.05 | 22.0 | 6.2 | 3.9 |
| Comparative Example 1 | P-1 | phosphorescent compound A | P-4 | -0.22 | 27.5 | 6.6 | 1.7 |
| Comparative Example 2 | P-2 | phosphorescent compound A | P-4 | -0.21 | 30.8 | 6.4 | 2.2 |
| Comparative Example 3 | P-1 | phosphorescent compound A | P-7 | -0.22 | 34.5 | 5.9 | 3.3 |
| Comparative Example 4 | P-2 | phosphorescent compound A | P-7 | -0.21 | 30.9 | 6.1 | 2.9 |

Industrial Applicability

According to the invention, an organic electroluminescent device having a long luminance life can be provided.

## Claims

1. An organic electroluminescent device comprising
an anode,
a cathode,
a light emitting layer that is disposed between the anode and the cathode and contains a first light emitting layer material containing a phosphorescent compound and a second light emitting layer material containing a charge transporting polymer compound, and
a hole transporting layer that is disposed between the anode and the light emitting layer so as to be in contact with the light emitting layer and is composed of a hole transporting polymer compound,
**characterized in that** the lowest excitation triplet energy $T1_e$ (eV) of the first light emitting layer material, the lowest excitation triplet energy $T1_h$ (eV) of the second light emitting layer material and the lowest excitation triplet energy $T1_t$ (eV) of the hole transporting polymer compound satisfy the following formulae (A) and (B):

$$T1_e \leq T1_h \quad (A)$$

$$T1_t - T1_e \leq 0 \text{ eV} \quad (B).$$

2. The organic electroluminescent device according to claim 1, wherein, $T1_t$ and $T1_e$ further satisfy the following formula (B'):

$$T1_t - T1_e \geq -0.30 \text{ eV} \quad (B').$$

**3.** The organic electroluminescent device according to claim 1 or 2, wherein the minimum value $IP_{eh}$ (eV) of the ionization potential of said first light emitting layer material and the ionization potential of said second light emitting layer material, and the ionization potential $IP_t$ (eV) of said hole transporting polymer compound satisfy the following formula (C):

$$IP_{eh} - IP_t \geq -0.20 \text{ eV} \quad \text{(C)}.$$

**4.** The organic electroluminescent device according to any one of claims 1 to 3, wherein said hole transporting polymer compound is a polymer compound containing a repeating unit represented by the following formula (4):

$\{Ar^1\}$ (4)

in the formula (4), $Ar^1$ represents an arylene group, a divalent aromatic heterocyclic group, or a divalent group composed of two or more directly linked identical or different groups selected from the group consisting of the arylene group and the divalent aromatic heterocyclic group, wherein the group represented by $Ar^1$ may have an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group, a fluorine atom or a cyano group as a substituent;
and a repeating unit represented by the following formula (5) :

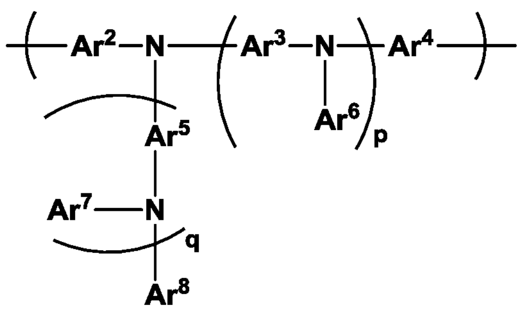

(5)

in the formula (5), $Ar^2$, $Ar^3$, $Ar^4$ and $Ar^5$ each independently represent an arylene group, a divalent aromatic heterocyclic group, or a divalent group composed of two or more directly linked identical or different groups selected from the group consisting of the arylene group and the divalent aromatic heterocyclic group; $Ar^6$, $Ar^7$ and $Ar^8$ each independently represent an aryl group or a monovalent aromatic heterocyclic group; p and q each independently represent 0 or 1, wherein the groups represented by $Ar^2$, $Ar^3$, $Ar^4$, $Ar^5$, $Ar^6$, $Ar^7$ and $Ar^8$ may have an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group, a fluorine atom or a cyano group as a substituent, and the groups represented by $Ar^5$, $Ar^6$, $Ar^7$ and $Ar^8$ may each be linked directly or via -O-, -S-, -C(=O)-, -C(=O)-O-, $-N(R^A)-$, $-C(=O)-N(R^A)-$ or $-C(R^A)_2-$ to the group represented by $Ar^2$, $Ar^3$, $Ar^4$, $Ar^5$, $Ar^6$, $Ar^7$ or $Ar^8$ linked to the nitrogen atom to which the groups are attached, thereby forming a 5 to 7-membered ring; $R^A$ represents an alkyl group, an aryl group, a monovalent aromatic heterocyclic group or an aralkyl group.

**5.** The organic electroluminescent device according to any one of claims 1 to 4, wherein said hole transporting polymer compound is a crosslinkable hole transporting polymer compound.

**6.** The organic electroluminescent device according to any one of claims 1 to 5, wherein said charge transporting polymer compound is a polymer compound containing at least one repeating unit selected from the group consisting of repeating units represented by the following formula (4):

$\{Ar^1\}$ (4)

in the formula (4), $Ar^1$ represents an arylene group, a divalent aromatic heterocyclic group, or a divalent group composed of two or more directly linked identical or different groups selected from the group consisting of the arylene group and the divalent aromatic heterocyclic group, wherein the group represented by $Ar^1$ may have an alkyl group, an aryl group, a monovalent aromatic heterocyclic group, an alkoxy group, an aryloxy group, an aralkyl group, an

arylalkoxy group, a substituted amino group, a substituted carbonyl group, a substituted carboxyl group, a fluorine atom or a cyano group as a substituent, and repeating units represented by the following formula (5) :

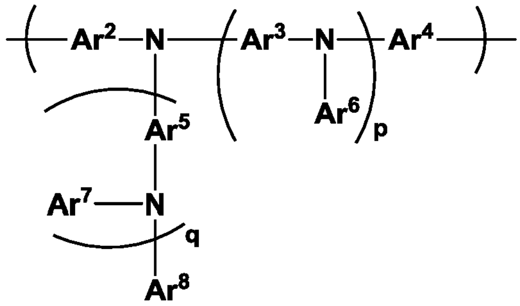

(5)

in the formula (5), $Ar^2$, $Ar^3$, $Ar^4$ and $Ar^5$ each independently represent an arylene group, a divalent aromatic heterocyclic group, or a divalent group composed of two or more directly linked identical or different groups selected from the group consisting of the arylene group and the divalent aromatic heterocyclic group; $Ar^6$, $Ar^7$ and $Ar^8$ each independently represent an aryl group or a monovalent aromatic heterocyclic group; p and q each independently represent 0 or 1, wherein the groups represented by $Ar^2$, $Ar^3$, $Ar^4$, $Ar^5$, $Ar^6$, $Ar^7$ and $Ar^8$ may have an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group, a fluorine atom or a cyano group as a substituent, and the groups represented by $Ar^5$, $Ar^6$, $Ar^7$ and $Ar^8$ may each be linked directly or via -O-, -S-, -C(=O)-, -C(=O)-O-, $-N(R^A)-$, $-C(=O)-N(R^A)-$ or $-C(R^A)_2-$ to the group represented by $Ar^2$, $Ar^3$, $Ar^4$, $Ar^5$, $Ar^6$, $Ar^7$ or $Ar^8$ linked to the nitrogen atom to which the groups are attached, thereby forming a 5 to 7-membered ring; $R^A$ represents an alkyl group, an aryl group, a monovalent aromatic heterocyclic group or an aralkyl group.

**7.** The organic electroluminescent device according to any one of claims 4 to 6, containing a repeating unit represented by the following formula (6) and/or a repeating unit represented by the following formula (7), as the repeating unit represented by said formula (4):

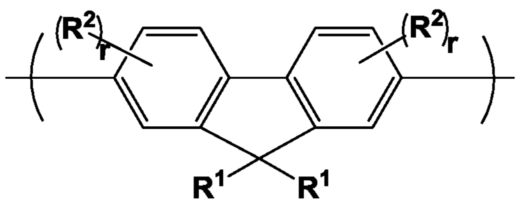

(6)

in the formula (6), each $R^1$ represents an alkyl group, an aryl group, a monovalent aromatic heterocyclic group or an aralkyl group; each $R^2$ represents an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group, a fluorine atom or a cyano group; each r represents an integer of 0 to 3, wherein two R' moieties may be the same or different, and two $R^1$ moieties may be linked to form a ring; when a plurality of $R^2$ moieties are present, these may be the same or different; two characters of r may be the same or different,

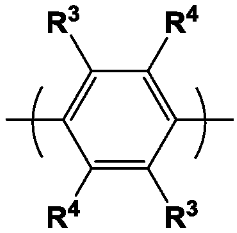

(7)

in the formula (7), each $R^3$ represents an alkyl group, an aryl group, a monovalent aromatic heterocyclic group, an alkoxy group, an aryloxy group, an aralkyl group, an arylalkoxy group, a substituted amino group, a substituted

carbonyl group, a substituted carboxyl group or a cyano group; each $R^4$ represents a hydrogen atom, an alkyl group, an aryl group, a monovalent aromatic heterocyclic group, an alkoxy group, an aryloxy group, an aralkyl group, an arylalkoxy group, a substituted amino group, a substituted carbonyl group, a substituted carboxyl group, a fluorine atom or a cyano group, wherein two $R^3$ moieties may be the same or different, and two $R^4$ moieties may be the same or different.

**8.** The organic electroluminescent device according to claim 7, wherein the repeating unit represented by said formula (4) is a repeating unit represented by said formula (6) .

**9.** The organic electroluminescent device according to claim 7, wherein the repeating unit represented by said formula (4) is a repeating unit represented by said formula (7) .

**10.** The organic electroluminescent device according to any one of claims 4 to 9, wherein at least one of p and q is 1 in said formula (5).

**11.** The organic electroluminescent device according to any one of claims 1 to 10, wherein said phosphorescent compound is an iridium complex.

**12.** The organic electroluminescent device according to any one of claims 1 to 11, having a hole injection layer between said anode and said hole transporting layer.

**Patentansprüche**

**1.** Eine organische Elektrolumineszenzvorrichtung, umfassend
eine Anode,
eine Kathode,
eine Licht emittierende Schicht, die zwischen der Anode und der Kathode angeordnet ist und ein erstes Licht emittierendes Schichtmaterial, das eine phosphoreszente Verbindung enthält, und ein zweites Licht emittierendes Schichtmaterial, das eine ladungstransportierende Polymerverbindung enthält, enthält, und
eine lochtransportierende Schicht, die so zwischen der Anode und der Licht emittierenden Schicht angeordnet ist, dass sie mit der Licht emittierenden Schicht in Kontakt steht und eine lochtransportierende Polymerverbindung aufweist,
**dadurch gekennzeichnet, dass** die niedrigste Anregungs-Triplettenergie $T1_e$ (eV) des ersten Licht emittierenden Schichtmaterials, die niedrigste Anregungs-Triplettenergie $T1_h$ (eV) des zweiten Licht emittierenden Schichtmaterials und die niedrigste Anregungs-Triplettenergie $T1_t$ (eV) der lochtransportierenden Polymerverbindung die folgenden Formeln (A) und (B) erfüllen:

$$T1_e \leq T1_h \qquad (A)$$

$$T1_t - T1_e \leq 0\ eV \qquad (B).$$

**2.** Die organische Elektrolumineszenzvorrichtung gemäß Anspruch 1, wobei $T1_t$ und $T1_e$ weiterhin die folgende Formel (B') erfüllen:

$$T1_t - T1_e \geq -0{,}30\ eV\ (B').$$

**3.** Die organische Elektrolumineszenzvorrichtung gemäß Anspruch 1 oder 2, wobei der Minimalwert $IP_{eh}$ (eV) des Ionisierungspotentials des ersten Licht emittierenden Schichtmaterials und das Ionisierungspotential des zweiten Licht emittierenden Schichtmaterials und das Ionisierungspotential $IP_t$ (eV) der lochtransportierenden Polymerverbindung die folgende Formel (C) erfüllen:

$$IP_{ch} - IP_t \geq -0{,}20\ eV \qquad (C).$$

**4.** Die organische Elektrolumineszenzvorrichtung gemäß einem der Ansprüche 1 bis 3, wobei die lochtransportierende Polymerverbindung eine Polymerverbindung ist, die eine durch die folgende Formel (4) dargestellte Wiederholungseinheit:

$$\{Ar^1\} \qquad (4)$$

wobei $Ar^1$ in der Formel (4) für eine Arylengruppe, eine zweiwertige aromatische heterocyclische Gruppe oder eine zweiwertige Gruppe, die zwei oder mehr direkt verknüpfte identische oder verschiedene Gruppen, ausgewählt aus der Gruppe bestehend aus der Arylengruppe und der zweiwertigen aromatischen heterocyclischen Gruppe, aufweist, steht, wobei die durch $Ar^1$ dargestellte Gruppe eine Alkylgruppe, eine Arylgruppe, eine einwertige aromatische heterocyclische Gruppe, eine Alkoxygruppe, eine Aryloxygruppe, eine Aralkylgruppe, eine Arylalkoxygruppe, eine substituierte Aminogruppe, eine substituierte Carbonylgruppe, eine substituierte Carboxylgruppe, ein Fluoratom oder eine Cyanogruppe als einen Substituent aufweisen kann;
und eine durch die folgende Formel (5) dargestellte Wiederholungeinheit enthält:

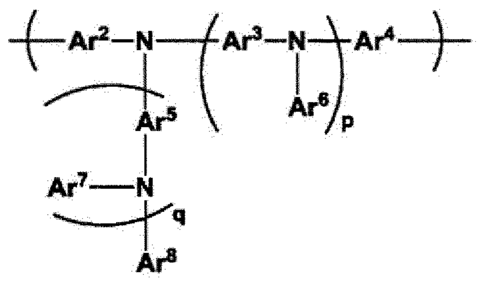

(5)

wobei in der Formel (5) $Ar^2$, $Ar^3$, $Ar^4$ und $Ar^5$ jeweils unabhängig für eine Arylengruppe, eine zweiwertige aromatische heterocyclische Gruppe oder eine zweiwertige Gruppe, die zwei oder mehr direkt verknüpfte identische oder verschiedene Gruppen, ausgewählt aus der Gruppe bestehend aus der Arylengruppe und der zweiwertigen aromatischen heterocyclischen Gruppe, aufweist, stehen; $Ar^6$ , $Ar^7$ und $Ar^8$ jeweils unabhängig für eine Arylgruppe oder eine einwertige aromatische heterocyclische Gruppe stehen; p und q jeweils unabhängig für 0 oder 1 stehen, wobei die durch $Ar^2$, $Ar^3$, $Ar^4$, $Ar^5$, $Ar^6$, $Ar^7$ und $Ar^8$ dargestellten Gruppen eine Alkylgruppe, eine Arylgruppe, eine einwertige aromatische heterocyclische Gruppe, eine Alkoxygruppe, eine Aryloxygruppe, eine Aralkylgruppe, eine Arylalkoxygruppe, eine substituierte Aminogruppe, eine substituierte Carbonylgruppe, eine substituierte Carboxylgruppe, ein Fluoratom oder eine Cyanogruppe als einen Substituent aufweisen können, und die durch $Ar^5$, $Ar^6$, $Ar^7$ und $Ar^8$ dargestellten Gruppen jeweils direkt oder über -O-, -S-, -C(=O)-, -C(=O)-O-, $-N(R^A)-$, $-C(=O)-N(R^A)-$ oder $-C(R^A)_2-$ mit der durch $Ar^2$, $Ar^3$, $Ar^4$, $Ar^5$, $Ar^6$, $Ar^7$ oder $Ar^8$ dargestellten Gruppe, die mit dem Stickstoffatom, an das die Gruppen gebunden sind, verknüpft ist, unter Bildung eines 5- bis 7-gliedrigen Rings verknüpft sein können; wobei $R^A$ für eine Alkylgruppe, eine Arylgruppe, eine einwertige aromatische heterocyclische Gruppe oder eine Aralkylgruppe steht.

**5.** Die organische Elektrolumineszenzvorrichtung gemäß einem der Ansprüche 1 bis 4, wobei die lochtransportierende Polymerverbindung eine vernetzbare lochtransportierende Polymerverbindung ist.

**6.** Die organische Elektrolumineszenzvorrichtung gemäß einem der Ansprüche 1 bis 5, wobei die ladungstransportierende Polymerverbindung eine Polymerverbindung ist, die mindestens eine Wiederholungseinheit, ausgewählt aus der Gruppe bestehend aus durch die folgende Formel (4) dargestellten Wiederholungseinheiten:

$$\{Ar^1\} \qquad (4)$$

wobei $Ar^1$ in der Formel (4) für eine Arylengruppe, eine zweiwertige aromatische heterocyclische Gruppe oder eine zweiwertige Gruppe, die zwei oder mehr direkt verknüpfte identische oder verschiedene Gruppen, ausgewählt aus der Gruppe bestehend aus der Arylengruppe und der zweiwertigen aromatischen heterocyclischen Gruppe, aufweist, steht, wobei die durch $Ar^1$ dargestellte Gruppe eine Alkylgruppe, eine Arylgruppe, eine einwertige aromatische heterocyclische Gruppe, eine Alkoxygruppe, eine Aryloxygruppe, eine Aralkylgruppe, eine Arylalkoxygruppe, eine substituierte Aminogruppe, eine substituierte Carbonylgruppe, eine substituierte Carboxylgruppe, ein Fluoratom oder eine Cyanogruppe als einen Substituent aufweisen kann,
und durch die folgende Formel (5) dargestellte Wiederholungseinheiten enthält:

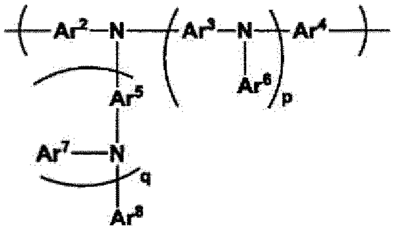

(5)

wobei in der Formel (5) $Ar^2$, $Ar^3$, $Ar^4$ und $Ar^5$ jeweils unabhängig für eine Arylengruppe, eine zweiwertige aromatische heterocyclische Gruppe oder eine zweiwertige Gruppe, die zwei oder mehr direkt verknüpfte identische oder verschiedene Gruppen, ausgewählt aus der Gruppe bestehend aus der Arylengruppe und der zweiwertigen aromatischen heterocyclischen Gruppe, aufweist, stehen; $Ar^6$, $Ar^7$ und $Ar^8$ jeweils unabhängig für eine Arylgruppe oder eine einwertige aromatische heterocyclische Gruppe stehen; p und q jeweils unabhängig für 0 oder 1 stehen, wobei die durch $Ar^2$, $Ar^3$, $Ar^4$, $Ar^5$, $Ar^6$, $Ar^7$ und $Ar^8$ dargestellten Gruppen eine Alkylgruppe, eine Arylgruppe, eine einwertige aromatische heterocyclische Gruppe, eine Alkoxygruppe, eine Aryloxygruppe, eine Aralkylgruppe, eine Arylalkoxygruppe, eine substituierte Aminogruppe, eine substituierte Carbonylgruppe, eine substituierte Carboxylgruppe, ein Fluoratom oder eine Cyanogruppe als einen Substituent aufweisen können, und die durch $Ar^5$, $Ar^6$, $Ar^7$ und $Ar^8$ dargestellten Gruppen jeweils direkt oder über -O-, -S-, -C(=O)-, -C(=O)-O-, -N($R^A$)-, -C(=O)-N($R^A$)- oder -C($R^A$)$_2$- mit der durch $Ar^2$, $Ar^3$, $Ar^4$, $Ar^5$, $Ar^6$, $Ar^7$ oder $Ar^8$ dargestellten Gruppe, die mit dem Stickstoffatom, an das die Gruppen gebunden sind, verknüpft ist, unter Bildung eines 5- bis 7-gliedrigen Rings verknüpft sein können; wobei $R^A$ für eine Alkylgruppe, eine Arylgruppe, eine einwertige aromatische heterocyclische Gruppe oder eine Aralkylgruppe steht.

**7.** Die organische Elektrolumineszenzvorrichtung gemäß einem der Ansprüche 4 bis 6, die eine durch die folgende Formel (6) dargestellte Wiederholungseinheit und/oder eine durch die folgende Formel (7) dargestellte Wiederholungseinheit, als die durch die Formel (4) dargestellte Wiederholungseinheit enthält:

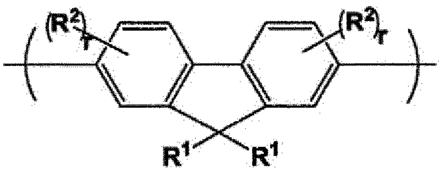

(6)

wobei in der Formel (6) jedes $R^1$ für eine Alkylgruppe, eine Arylgruppe, eine einwertige aromatische heterocyclische Gruppe oder eine Aralkylgruppe steht; jedes $R^2$ für eine Alkylgruppe, eine Arylgruppe, eine einwertige aromatische heterocyclische Gruppe, eine Alkoxygruppe, eine Aryloxygruppe, eine Aralkylgruppe, eine Arylalkoxygruppe, eine substituierte Aminogruppe, eine substituierte Carbonylgruppe, eine substituierte Carboxylgruppe, ein Fluoratom oder eine Cyanogruppe steht; jedes r für eine ganze Zahl von 0 bis 3 steht, wobei zwei R'-Einheiten gleich oder verschieden sein können, und zwei $R^1$-Einheiten verknüpft sein können, um einen Ring zu bilden; wenn mehrere $R^2$-Einheiten vorhanden sind, diese gleich oder verschieden sein können; zwei Zeichen von r gleich oder verschieden sein können,

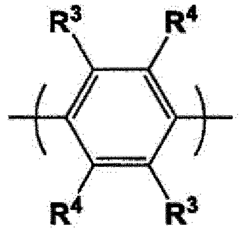

(7)

wobei in der Formel (7) jedes $R^3$ für eine Alkylgruppe, eine Arylgruppe, eine einwertige aromatische heterocyclische Gruppe, eine Alkoxygruppe, eine Aryloxygruppe, eine Aralkylgruppe, eine Arylalkoxygruppe, eine substituierte Aminogruppe, eine substituierte Carbonylgruppe, eine substituierte Carboxylgruppe oder eine Cyanogruppe steht; jedes $R^4$ für ein Wasserstoffatom, eine Alkylgruppe, eine Arylgruppe, eine einwertige aromatische heterocyclische Gruppe, eine Alkoxygruppe, eine Aryloxygruppe, eine Aralkylgruppe, eine Arylalkoxygruppe, eine substituierte Aminogruppe,

eine substituierte Carbonylgruppe, eine substituierte Carboxylgruppe, ein Fluoratom oder eine Cyanogruppe steht, wobei zwei $R^3$-Einheiten gleich oder verschieden sein können und zwei $R^4$-Einheiten gleich oder verschieden sein können.

**8.** Die organische Elektrolumineszenzvorrichtung gemäß Anspruch 7, wobei die durch die Formel (4) dargestellte Wiederholungseinheit eine durch die Formel (6) dargestellte Wiederholungseinheit ist.

**9.** Die organische Elektrolumineszenzvorrichtung gemäß Anspruch 7, wobei die durch die Formel (4) dargestellte Wiederholungseinheit eine durch die Formel (7) dargestellte Wiederholungseinheit ist.

**10.** Die organische Elektrolumineszenzvorrichtung gemäß einem der Ansprüche 4 bis 9, wobei mindestens eines von p und q in Formel (5) gleich 1 ist.

**11.** Die organische Elektrolumineszenzvorrichtung gemäß einem der Ansprüche 1 bis 10, wobei die phosphoreszente Verbindung ein Iridiumkomplex ist.

**12.** Die organische Elektrolumineszenzvorrichtung gemäß einem der Ansprüche 1 bis 11 mit einer Lochinjektionsschicht zwischen der Anode und der Lochtransportschicht.

**Revendications**

**1.** Dispositif électroluminescent organique comprenant
une anode,
une cathode,
une couche luminescente qui est disposée entre l'anode et la cathode et qui contient un premier matériau de couche luminescente contenant un composé phosphorescent et un deuxième matériau de couche luminescente contenant un composé polymère de transport de charges, et
une couche de transport de trous qui est disposée entre l'anode et la couche luminescente de façon à être en contact avec la couche luminescente et qui est composée d'un composé polymère de transport de trous,
**caractérisé en ce que** l'énergie de triplet d'excitation la plus faible $T1_e$ (eV) du premier matériau de couche luminescente, l'énergie de triplet d'excitation la plus faible $T1_h$ (eV) du deuxième matériau de couche luminescente et l'énergie de triplet d'excitation la plus faible $T1_t$ (eV) du composé polymère de transport de trous satisfont aux formules (A) et (B) suivante :

$$T1_e \leq T1_h \qquad (A)$$

$$T1_t - T1_e \leq 0 \text{ eV} \qquad (B).$$

**2.** Dispositif électroluminescent organique selon la revendication 1, dans lequel $T1_t$ et $T1_e$ satisfont en outre à la formule (B') suivante :

$$T1_t - T1_e \geq -0{,}30 \text{ eV} \qquad (B').$$

**3.** Dispositif électroluminescent organique selon la revendication 1 ou 2, dans lequel la valeur minimale $IP_{eh}$ (eV) du potentiel d'ionisation dudit premier matériau de couche luminescente et du potentiel d'ionisation dudit deuxième matériau de couche luminescente, et le potentiel d'ionisation $IP_t$ (eV) dudit composé polymère de transport de trous, satisfont à la formule (C) suivante :

$$IP_{eh} - IP_t \geq -0{,}20 \text{ eV} \qquad (C).$$

**4.** Dispositif électroluminescent organique selon l'une quelconque des revendications 1 à 3, dans lequel ledit composé polymère de transport de trous est un composé polymère contenant un motif répétitif représenté par la formule (4) suivante :

$\{Ar^1\}$ (4)

dans la formule (4), $Ar^1$ représente un groupe arylène, un groupe hétérocyclique aromatique divalent, ou un groupe divalent composé de deux ou plus de deux groupes identiques ou différents liés directement choisis dans l'ensemble constitué par un groupe arylène et un groupe hétérocyclique aromatique divalent, où le groupe représenté par $Ar^1$ peut porter en tant que substituant un groupe alkyle, un groupe aryle, un groupe hétérocyclique aromatique monovalent, un groupe alcoxy, un groupe aryloxy, un groupe aralkyle, un groupe arylalcoxy, un groupe amino substitué, un groupe carbonyle substitué, un groupe carboxyle substitué, un atome de fluor ou un groupe cyano ;
et un motif répétitif représenté par la formule (5) suivante :

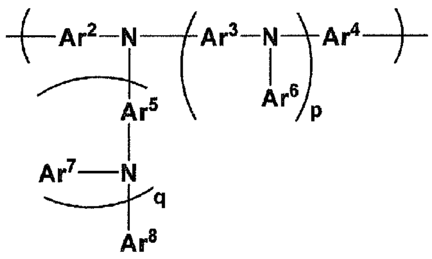

(5)

dans la formule (5), chacun de $Ar^2$, $Ar^3$, $Ar^4$ et $Ar^5$ représente indépendamment un groupe arylène, un groupe hétérocyclique aromatique divalent, ou un groupe divalent composé de deux ou plus de deux groupes identiques ou différents liés directement choisis dans l'ensemble constitué par un groupe arylène et un groupe hétérocyclique aromatique divalent ; chacun de $Ar^6$, $Ar^7$ et $Ar^8$ représente indépendamment un groupe aryle ou un groupe hétérocyclique aromatique monovalent ; chacun de p et q vaut indépendamment 0 ou 1, où les groupes représentés par $Ar^2$, $Ar^3$, $Ar^4$, $Ar^5$, $Ar^6$, $Ar^7$ et $Ar^8$ peuvent porter en tant que substituant un groupe alkyle, un groupe aryle, un groupe hétérocyclique aromatique monovalent, un groupe alcoxy, un groupe aryloxy, un groupe aralkyle, un groupe arylalcoxy, un groupe amino substitué, un groupe carbonyle substitué, un groupe carboxyle substitué, un atome de fluor ou un groupe cyano, et chacun des groupes représentés par $Ar^5$, $Ar^6$, $Ar^7$ et $Ar^8$ peut être lié directement ou via -O-, -S-, -C(=O)-, -C(=O)-O-, $-N(R^A)-$, $-C(=O)-N(R^A)-$ ou $-C(R^A)_2-$ au groupe représenté par $Ar^2$, $Ar^3$, $Ar^4$, $Ar^5$, $R^6$, $Ar^7$ ou $Ar^8$ lié à l'atome d'azote auquel les groupes sont rattachés, en formant ainsi un cycle à 5 à 7 chaînons ; $R^A$ représente un groupe alkyle, un groupe aryle, un groupe hétérocyclique aromatique monovalent ou un groupe aralkyle.

**5.** Dispositif électroluminescent organique selon l'une quelconque des revendications 1 à 4, dans lequel ledit composé polymère de transport de trous est un composé polymère de transport de trous réticulable.

**6.** Dispositif électroluminescent organique selon l'une quelconque des revendications 1 à 5, dans lequel ledit composé polymère de transport de charges est un composé polymère contenant au moins un motif répétitif choisi dans l'ensemble constitué par les motifs répétitifs représentés par la formule (4) suivante :

$\{Ar^1\}$ (4)

dans la formule (4), $Ar^1$ représente un groupe arylène, un groupe hétérocyclique aromatique divalent, ou un groupe divalent composé de deux ou plus de deux groupes identiques ou différents liés directement choisis dans l'ensemble constitué par un groupe arylène et un groupe hétérocyclique aromatique divalent, où le groupe représenté par $Ar^1$ peut porter en tant que substituant un groupe alkyle, un groupe aryle, un groupe hétérocyclique aromatique monovalent, un groupe alcoxy, un groupe aryloxy, un groupe aralkyle, un groupe arylalcoxy, un groupe amino substitué, un groupe carbonyle substitué, un groupe carboxyle substitué, un atome de fluor ou un groupe cyano ;
et des motifs répétitifs représentés par la formule (5) suivante :

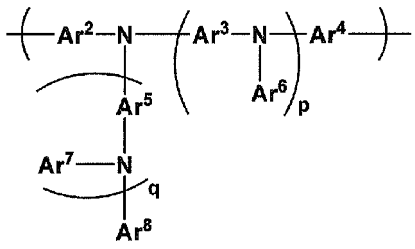

(5)

dans la formule (5), chacun de $Ar^2$, $Ar^3$, $Ar^4$ et $Ar^5$ représente indépendamment un groupe arylène, un groupe hétérocyclique aromatique divalent, ou un groupe divalent composé de deux ou plus de deux groupes identiques ou différents liés directement choisis dans l'ensemble constitué par un groupe arylène et un groupe hétérocyclique aromatique divalent ; chacun de $Ar^6$, $Ar^7$ et $Ar^8$ représente indépendamment un groupe aryle ou un groupe hétérocyclique aromatique monovalent ; chacun de p et q vaut indépendamment 0 ou 1, où les groupes représentés par $Ar^2$, $Ar^3$, $Ar^4$, $Ar^5$, $Ar^6$, $Ar^7$ et $Ar^8$ peuvent porter en tant que substituant un groupe alkyle, un groupe aryle, un groupe hétérocyclique aromatique monovalent, un groupe alcoxy, un groupe aryloxy, un groupe aralkyle, un groupe arylalcoxy, un groupe amino substitué, un groupe carbonyle substitué, un groupe carboxyle substitué, un atome de fluor ou un groupe cyano, et chacun des groupes représentés par $Ar^5$, $Ar^6$, $Ar^7$ et $Ar^8$ peut être lié directement ou via -O-, -S-, -C(=O)-, -C(=O)-O-, -N($R^A$)-, -C(=O)-N($R^A$)- ou -C($R^A$)$_2$- au groupe représenté par $Ar^2$, $Ar^3$, $Ar^4$, $Ar^5$, $R^6$, $Ar^7$ ou $Ar^8$ lié à l'atome d'azote auquel les groupes sont rattachés, en formant ainsi un cycle à 5 à 7 chaînons ; $R^A$ représente un groupe alkyle, un groupe aryle, un groupe hétérocyclique aromatique monovalent ou un groupe aralkyle.

**7.** Dispositif électroluminescent organique selon l'une quelconque des revendications 4 à 6, contenant un motif répétitif représenté par la formule (6) suivante et/ou un motif répétitif représenté par la formule (7) suivante, en tant que motif répétitif représenté par ladite formule (4) :

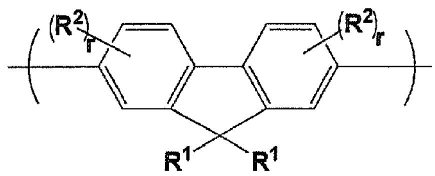

(6)

dans la formule (6), chaque $R^1$ représente un groupe alkyle, un groupe aryle, un groupe hétérocyclique aromatique monovalent ou un groupe aralkyle ; chaque $R^2$ représente un groupe alkyle, un groupe aryle, un groupe hétérocyclique aromatique monovalent, un groupe alcoxy, un groupe aryloxy, un groupe aralkyle, un groupe arylalcoxy, un groupe amino substitué, un groupe carbonyle substitué, un groupe carboxyle substitué, un atome de fluor ou un groupe cyano ; chaque r représente un entier de 0 à 3, où deux fragments R' peuvent être identiques ou différents, et deux fragments $R^1$ peuvent être liés pour former un cycle ; quand plusieurs fragments $R^2$ sont présents, ceux-ci peuvent être identiques ou différents ; deux itérations de r peuvent être identiques ou différentes,

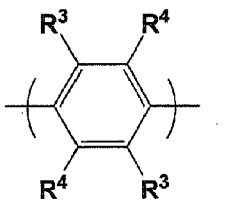

(7)

dans la formule (7), chaque $R^3$ représente un groupe alkyle, un groupe aryle, un groupe hétérocyclique aromatique monovalent, un groupe alcoxy, un groupe aryloxy, un groupe aralkyle, un groupe arylalcoxy, un groupe amino substitué, un groupe carbonyle substitué, un groupe carboxyle substitué ou un groupe cyano ; chaque $R^4$ représente un atome d'hydrogène, un groupe alkyle, un groupe aryle, un groupe hétérocyclique aromatique monovalent, un groupe alcoxy, un groupe aryloxy, un groupe aralkyle, un groupe arylalcoxy, un groupe amino substitué, un groupe carbonyle substitué, un groupe carboxyle substitué, un atome de fluor ou un groupe cyano, où deux fragments $R^3$ peuvent être identiques ou différents, et deux fragments $R^4$ peuvent être identiques ou différents.

8. Dispositif électroluminescent organique selon la revendication 7, dans lequel le motif répétitif représenté par ladite formule (4) est un motif répétitif représenté par ladite formule (6).

9. Dispositif électroluminescent organique selon la revendication 7, dans lequel le motif répétitif représenté par ladite formule (4) est un motif répétitif représenté par ladite formule (7).

10. Dispositif électroluminescent organique selon l'une quelconque des revendications 4 à 9, dans lequel au moins l'un de p et q vaut 1 dans ladite formule (5).

11. Dispositif électroluminescent organique selon l'une quelconque des revendications 1 à 10, dans lequel ledit composé phosphorescent est un complexe d'iridium.

12. Dispositif électroluminescent organique selon l'une quelconque des revendications 1 à 11, ayant une couche d'injection de trous entre ladite anode et ladite couche de transport de trous.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008179617 A
- JP 10092582 A
- WO 200549548 A
- WO 2006070619 A1
- JP 2006128636 A
- JP 2004139819 A
- WO 2002066552 A
- WO 2004020504 A
- WO 2004020448 A


### Non-patent literature cited in the description


- *Appl. Phys. Lett.,* 1999, vol. 75 (1), 4
- *Jpn. J. Appl. Phys.,* 1995, vol. 34, 1883
- *Appl. Phys. Lett.,* 2001, vol. 78 (11), 1622
- *Nature,* 1998, vol. 395, 151
- *Proc. SPIE-Int. Soc. Opt. Eng.,* 2001, vol. 4105
- *Organic Light-Emitting Materials and Devices IV,* vol. 119
- *J. Am. Chem. Soc.,* 2001, vol. 123, 4304
- *Appl. Phys. Lett.,* 1997, vol. 71 (18), 2596
- *Syn. Met.,* 1998, vol. 97 (2), 113
- *Syn. Met.,* 1999, vol. 99 (2), 127
- *Adv. Mater.,* 1999, vol. 11 (10), 852
- *Inorg. Chem.,* 2003, vol. 42, 8609
- *Inorg. Chem.,* 2004, vol. 43, 6513
- *Inorg. Chem.,* 2007, vol. 46, 11082
- *Journal of the SID 11/1,* 2003, vol. 161